(19)

**Europäisches Patentamt**
**European Patent Office**
**Office européen des brevets**

(11)  **EP 2 976 433 B1**

(12)  # EUROPEAN PATENT SPECIFICATION

(45) Date of publication and mention
of the grant of the patent:
**31.01.2018 Bulletin 2018/05**

(51) Int Cl.:
***C12Q 1/68*** *(2018.01)*      ***G06F 19/00*** *(2018.01)*

(21) Application number: **14712601.5**

(86) International application number:
**PCT/EP2014/000761**

(22) Date of filing: **20.03.2014**

(87) International publication number:
**WO 2014/146793 (25.09.2014 Gazette 2014/39)**

(54) **METHOD FOR THE DETERMINATION OF BIOLOGICAL AGE IN HUMAN BEINGS**

VERFAHREN ZUR ERMITTLUNG DES BIOLOGISCHEN ALTERS VON MENSCHEN

PROCÉDÉ POUR LA DÉTERMINATION DE L'ÂGE BIOLOGIQUE CHEZ LES ÊTRES HUMAINS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.03.2013 EP 13001450**

(43) Date of publication of application:
**27.01.2016 Bulletin 2016/04**

(73) Proprietors:
• **Universität Konstanz
78464 Konstanz (DE)**
• **BioTeSys GmbH
73728 Esslingen (DE)**
• **Fundación Centro Nacional de Investigaciones
Oncológicas Carlos III
28029 Madrid (ES)**
• **Erasmus Universitair Medisch Centrum
Rotterdam
3015 CE Rotterdam (NL)**
• **Université de Namur
5000 Namur (BE)**
• **Universität Innsbruck
6020 Innsbruck (AT)**
• **Istituto Nazionale di Riposo e Cura per
Anziani (INCRA)
60121 Ancona (IT)**
• **Nestec S.A.
1800 Vevey (CH)**
• **National Hellenic Research Foundation
11635 Athens (GR)**
• **INSTYTUT BIOLOGII DOSWIADCZALNEJ IM. M.
NECKIEGO
PAN (NENCKI INSTITUTE)
02093 Warszawa (PL)**

• **Institutul National de Gerontologie si Geriatrie
Ana Aslan
011241 Bucharest (RO)**
• **RIJKSINSTITUUT VOOR VOLKSGEZONDHEID
EN
MILIEU
3721 MA Bilthoven (NL)**
• **Aarhus Universitet
8000 Aarhus C (DK)**
• **Aston University
Birmingham B4 7ET (GB)**
• **Vlaams Instituut voor Biotechnologie (VIB), vzw
9052 Zwijnaarde (BE)**
• **Universität Hohenheim
70593 Stuttgart (DE)**
• **ALMA MATER STUDIORUM-UNIVERSITA DI
BOLOGNA
40126 Bologna (IT)**
• **Martin-Luther-Universität Halle-Wittenberg
06120 Halle (DE)**
• **Università degli Studi di Roma "La Sapienza"
00161 Rome (IT)**
• **UNIVERSITE PIERRE ET MARIE CURIE - PARIS VI
75252 Paris (FR)**
• **TAMPEREEN YLIOPISTO
33014 Tampere (FI)**
• **Cranfield University
Cranfield, Bedfordshire MK43 0AL (GB)**

(72) Inventors:
• **BÜRKLE, Alexander
78479 Reichenau (DE)**
• **BERTHOLD, Michael
78465 Konstanz (DE)**
• **JUNK, Michael
78465 Konstanz (DE)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- **MORENO-VILLANUEVA, Maria**
  78465 Konstanz (DE)
- **BERNHARDT, Jürgen**
  73728 Esslingen (DE)
- **BLASCO, María**
  28029 Madrid (ES)
- **GALLARDO, Mercedes**
  28029 Madrid (ES)
- **HOEIJMAKERS, Jan H. J.**
  3015 CE Rotterdam (NL)
- **TOUSSAINT, Olivier**
  5000 Namur (BE)
- **GRUBECK-LOEBENSTEIN, Beatrix**
  6020 Innsbruck (AT)
- **MOCCHEGIANI, Eugenio**
  60121 Ancona (IT)
- **MALAVOLTA, Marco**
  60121 Ancona (IT)
- **COLLINO, Sebastiano**
  1000 Lausanne 26 (CH)
- **MOCO, Sofia**
  1000 Lausanne 26 (CH)
- **GONOS, Efstathios S.**
  11635 Athens (GR)
- **SIKORA, Ewa**
  02093 Waszawa (PL)
- **GRADINARU, Daniela**
  011241 Bucharest (RO)
- **DOLLÉ, Martijn**
  3721 MA Bilthoven (NL)
- **KRISTENSEN, Peter**
  8000 Aarhus C (DK)
- **GRIFFITHS, Helen**
  Birmingham B4 7ET (GB)
- **LIBERT, Claude**
  9052 Zwijnaarde (BE)
- **GRUNE, Tilman**
  10823 Berlin (DE)
- **BREUSING, Nicolle**
  70569 Stuttgart (DE)
- **FRANCESCHI, Claudio**
  40126 Bologna (IT)
- **SIMM, Andreas**
  06120 Halle (DE)
- **CAIAFA, Paola**
  00161 Rome (IT)
- **FRIGUET, Bertrand**
  75252 Paris (FR)
- **HERVONEN, Antti**
  33014 Tampere (FI)
- **ASPINALL, Richard**
  Cranfield, Bedfordshire MK 43 0AL (GB)

(74) Representative: **Müller-Boré & Partner**
**Patentanwälte PartG mbB**
**Friedenheimer Brücke 21**
**80639 München (DE)**

(56) References cited:
WO-A1-2012/162139　WO-A2-02/10445

- D. G. HERNANDEZ ET AL: "Distinct DNA methylation changes highly correlated with chronological age in the human brain", HUMAN MOLECULAR GENETICS, vol. 20, no. 6, 7 January 2011 (2011-01-07), pages 1164-1172, XP055069768, ISSN: 0964-6906, DOI: 10.1093/hmg/ddq561
- PAOLO GARAGNANI ET AL: "Methylation of ELOVL2 gene as a new epigenetic marker of age", AGING CELL, vol. 11, no. 6, 14 October 2012 (2012-10-14), pages 1132-1134, XP055069767, ISSN: 1474-9718, DOI: 10.1111/acel.12005
- MADRIGANO JAIME ET AL: "Aging and epigenetics Longitudinal changes in gene-specific DNA methylation", EPIGENETICS, LANDES BIOSCIENCE, US, vol. 7, no. 1, 1 January 2012 (2012-01-01), pages 63-70, XP008157957, ISSN: 1559-2294, DOI: 10.4161/EPI.7.1.18750
- BOCKLANDT S ET AL: "Epigenetic Predictor of Age", PLOS ONE, PUBLIC LIBRARY OF SCIENCE, US, vol. 6, no. 6, 22 June 2011 (2011-06-22), pages E14821-1, XP002687317, ISSN: 1932-6203, DOI: 10.1371/JOURNAL.PONE.0014821
- KOCH CARMEN M ET AL: "Epigenetic-aging-signature to determine age in different tissues", AGING, NEW YORK, NY, US, vol. 3, no. 10, 1 October 2011 (2011-10-01), pages 1018-1027, XP002687316, ISSN: 0160-2721
- J. P. DE MAGALHAES ET AL: "Meta-analysis of age-related gene expression profiles identifies common signatures of aging", BIOINFORMATICS, vol. 25, no. 7, 2 February 2009 (2009-02-02), pages 875-881, XP055069769, ISSN: 1367-4803, DOI: 10.1093/bioinformatics/btp073
- VALERIE VANHOOREN ET AL: "N-Glycan profiling in the study of human aging", BIOGERONTOLOGY, KLUWER ACADEMIC PUBLISHERS, DO, vol. 9, no. 5, 23 April 2008 (2008-04-23), pages 351-356, XP019599816, ISSN: 1573-6768

**Description**

The present invention relates to methods for the determination of the biological age of a human being which is defined in the appendent claims. This method is based on the findings of a multinational study screening for biomarkers of aging in humans.

Aging has been defined as the time-dependent decline of functional capacity and stress resistance, associated with increased risk of morbidity and mortality. Aging is a process that affects most if not all tissues and organs of the body. Moreover, cross-talk can occur between multiple physiological systems, e.g. cognitive or metabolic systems, which may influence the aging of the immune system. The mechanisms underlying the aging process are only beginning to be unraveled at the molecular level, yet there is clear evidence that the rate of aging differs significantly between members of the same animal species, including humans. In other words, the "biological age" may differ from the chronological age. The prior art describes different methods for determining the age of a human being, Hernandez et al., Hum. Mol. Genetics, 20,1164-1172, 2011; Garagnani et al., Aging Cell, 11, 1132-1134, 2012; Madrigano et al., Epigenetics, 7, 63-70, 2012; Bocklandt et al., Plos One, 6, e14821, 2011; Koch et al., Aging, 3, 1018-1027, 2001; WO0210445 A2; WO2012162139 A1; Magalhaes et al., Bioinformatics, 25, 875-881, 2009 and Vanhooren et al., Biogerontology, 9, 351-356, 2008. One important reason for such heterogeneity is individual genetic make-up. In several biological model systems, which are being extensively used in experimental gerontology, mutants have been isolated with either longer or shorter life span than the wild-type. In human beings, several rare hereditary, single-gene diseases are known to cause progeroid syndromes that are characterized by the early onset of some of the typical changes found in old individuals and premature death from diseases typical of old age. On the other hand, it is known that exceptional longevity in humans and the phenotype of "healthy aging" or "successful aging" have a hereditary, most likely polygenic component. This observation is the basis for the search for gene polymorphisms that are associated with this phenotype. The EC-funded "Genetics of Healthy Aging (GeHA)" study for example was a large-scale European effort focused on the systematic identification of the genetic components of human aging.

It is estimated, however, that genetic variation can account of maximally one third of the variability of the lifetime of human beings. Therefore other factors have to play a role. The influence of environmental factors, including nutrition, seems to be very important. This has been demonstrated experimentally in a wide variety of model systems, from *Saccharomyces cerevisiae* to rodents, and is very likely to hold true for humans as well.

But even under the most highly controlled experimental conditions, *i.e.* using model organisms and studying genetically identical individuals kept under identical environmental conditions, a significant degree of heterogeneity in lifetime can be observed, which has led to the conclusion that some element of chance (or some hitherto unknown and uncontrollable factor) will also play a role.

Although aging *per se* is a physiological phenomenon, it is also a very important risk factor for a wide variety of chronic and debilitating diseases. The rate of aging in humans is not uniform, due to genetic heterogeneity and the influence of environmental factors. Age-related changes in body function or composition that could serve as a measure of "biological" age and predict the onset of age-related diseases and/or residual lifetime are termed "biomarkers of aging". This term has been coined in analogy to biomarkers of specific chronic diseases, such as HIV infection, or biomarkers of exposure to toxins. Biomarkers of aging could be used to determine the rate of the aging process even in healthy human beings, and the identification of a relatively high biological age in a given person would indicate the necessity to initiate diagnostic procedures and early interventions to prevent or postpone the outbreak of overt age-related disease. Therefore, the availability of valid biomarkers of aging could represent an enormous step forward for Preventive Medicine.

The classical quantitative assessment of "aging" relies on the analysis of mortality curves of populations. In other words, individuals have to be followed up until the end of their lives in order to determine their "biological age" at any time point during their life. Therefore, at the level of a living individual, a reliable assessment of the state of aging, *i.e.* the state of the above-mentioned functional decline and a prediction of the risk of the onset of morbidity and the residual individual life expectancy are not possible with this method.

A strategy to solve this problem is the identification of age-related changes in body function or composition that could serve as a measure of "biological" age and predict the future onset of age-related diseases and/or residual lifetime more accurately than chronological age.

The American Federation for Aging Research has proposed the following criteria for a biomarker of aging. First, it must predict the rate of aging. In other words, it would tell exactly where a person is in their total life span. Further, it must be a better predictor of life span than chronological age. Second, it must monitor a basic process that underlies the aging process, not the effects of disease. Third, it must be able to be tested repeatedly without harming the person, for example by a blood test or an imaging technique. Fourth, it must be something that works in humans and in laboratory animals, such as mice. This is so that it can be tested in lab animals before being validated in humans. However, this last criterion is debatable because some biomarkers of human aging might exist that are of little relevance in model systems as some parameters/functions might be critical and/or limiting for the aging process only in complex organisms and not in simpler models. On the other hand, the primary identification and validation of biomarkers in model systems of aging can, of course, provide very promising candidates for targeted evaluation in humans.

Biomarkers of human aging are urgently needed for a variety of reasons. These include the identification of individuals at high risk of developing age-associated disease or disability. This would then prompt targeted follow-up examinations and, if available, prophylactic intervention or early-stage treatment of age-related disease. Furthermore, the availability of powerful biomarkers would allow the assessment of the efficacy of forthcoming pharmacological and other interventions (including optimization of micronutrient intake and other dietary components or physical activity) currently being developed and aimed to lower the risk of age-associated disease even in individuals without accelerated aging.

In view of the rapidly increasing average life expectancy of human beings worldwide, the prevalence of age-related diseases is likely to increase as well. This necessitates effective new strategies for prevention and early diagnosis of such conditions.

In order to determine biological age, previous studies have either relied on conventional clinical chemistry parameters measured in blood or its components or other body fluids as potential biomarkers of human aging, such as e.g. markers of kidney function, or on individual molecular parameters measured in blood or its components, such as e.g. telomere length. Occasionally, such measurements also comprised small groups of parameters and some studies also comprised physiological parameters, such as e.g. lung function, although these require full cooperation and motivation of the person to be tested.

However, conventional clinical chemistry parameters on their own do not reflect the specific changes occurring during aging at the molecular level. Therefore, analyses exclusively based on such parameters are not very powerful. Further, many individual candidate biomarkers of aging have been proposed, but in all cases their variability in cross-sectional studies is considerable, and therefore no single measurement has so far proven to yield a useful biomarker of aging on its own, probably due to the multi-causal and multi-systemic nature of aging. In addition, any assessment of biological age that requires full cooperation and motivation of the person to be tested is prone to errors and labor-intensive, which restricts their practical use at a wider scale.

Accordingly, the technical problem underlying the present invention is to provide a method for the determination of the biological age of a human being using a valid set of biomarkers of aging in humans that is based on biochemical and molecular analyses of blood and its components and/or urine and/or cellular samples. This set of biomarkers should be applicable to humans in the middle age range (e.g. 30 to 80 years) and should serve as a valuable diagnostic tool for preventive medicine by enabling identification of healthy persons whose aging process is accelerated and who thus are likely to be affected by typical age-related diseases at relatively young chronological age.

The solution to the above technical problem is achieved by the embodiments characterized in the claims.

In particular, in a first aspect, the present invention relates to a method for the determination of the biological age of a human being, comprising the steps of:

(a) providing one or more biological samples, selected from the group consisting of whole blood, serum, plasma, and urine, from the human being,
(b1) in case the human being is female, determining at least the following biomarkers (i) to (x) in the respective biological samples, wherein the necessary biological samples are provided in step (a):

(i) cumulative level of cytosine methylation at gene positions ELOVL_2_CpG_11, ELOVL_2_CpG_12, ELOVL_2_CpG_13, and ELOVL_2_CpG_14 in blood cells;
(ii) cumulative level of cytosine methylation at gene positions ELOVL_2_CpG_15, ELOVL_2_CpG_16, and ELOVL_2_CpG_17 in blood cells;
(iii) cumulative level of cytosine methylation at gene positions FHL_2_CpG_11, and FHL_2_CpG_12 in blood cells;
(iv) cumulative level of cytosine methylation at gene positions FHL_2_CpG_13, FHL_2_CpG_14, and FHL_2_CpG_15 in blood cells;
(v) cumulative level of cytosine methylation at gene positions FHL_2_CpG_16 and FHL_2_CpG_17 in blood cells;
(vi) concentration of dehydroepiandrosterone sulfate in plasma;
(vii) concentration of ferritin in serum;
(viii) decadic logarithm of the ratio between the concentration of NGA2F glycan in serum and the concentration of NA2F glycan in serum;
(ix) concentration of alpha-tocopherol in plasma;
(x) S_p6_n_glycan in serum

(b2) in case the human being is male, determining at least the following biomarkers (i) to (x) in the respective biological samples, wherein the necessary biological samples are provided in step (a):

(i) cumulative level of cytosine methylation at gene positions ELOVL_2_CpG_11, ELOVL_2_CpG_12, ELOVL_2_CpG_13, and ELOVL_2_CpG_14 in blood cells;
(ii) cumulative level of cytosine methylation at gene positions ELOVL_2_CpG_15, ELOVL_2_CpG_16, and ELOVL_2_CpG_17 in blood cells;
(iii) FHL_2_CpG_11, FHL_2_CpG_12
(iv) cumulative level of cytosine methylation at gene positions FHL_2_CpG_13, FHL_2_CpG_14, and FHL_2_CpG_15 in blood cells;
(v) level of cytosine methylation at gene position FHL_2_CpG_16, FHL_2_CpG_17;
(vi) concentration of dehydroepiandrosterone sulfate in plasma;
(vii) concentration of alpha-2-macroglubulin in serum;
(viii) concentration of lycopene in plasma;
(ix) prostate_specific_antigen in serum;
(x) S_p6_n_glycan in serum and

(c) determining the biological age of the human being based on the biomarkers determined in step (b1)/(b2).

As used herein the formulation "providing one or more biological samples" means the provision of samples which have already been taken from the human being the biological age of which is determined by the claimed method. The act of taking the samples from the human being the biological age of which is determined by the claimed method is not comprised in the formulation "providing one or more biological samples". While it is clear to a person skilled in the art that a sample has to be taken from the human being the biological age of which is determined by the claimed method at some point, this does not necessarily mean that taking the sample is included in the method according to the present invention. In a preferred embodiment of the present invention, the method according to the present invention does not encompass the act of taking the one or more biological samples from the human being the biological age of which is determined by the claimed method. In another preferred embodiment of the present invention the formulation "providing one or more biological samples" means "providing one or more biological samples, wherein the provision of the one or more biological samples does not include any interaction with the body of a human or animal being". In another preferred embodiment of the present invention the present invention relates to a method for the determination of the biological age of a human being as defined herein, wherein the step of taking the one or more biological samples from the human being to be tested has been carried out prior to carrying out the method according to the present invention.

In the context of the present invention, the terms "chronological age" and "biological age" or "bioage" are used and discriminated from each other. The term "chronological age" relates to the age of a human being as calculated as the amount of time since the person's day of birth. By way of example, a person that is born on April 27, 1975 has on February 22, 2013 a chronological age of 37 years, 9 months, and 26 days, or 37.816 years. The term "biological age" relates to a human being's bodily age, *i.e.* an artificial value that denotes how far the body has aged in terms of age-related changes in body function or composition. Again by way of example, the above person having a chronological age of 37.816 years might have (i) a lower biological age, e.g. 30 years, indicating that said person's body shows less age-related changes in body function or composition as compared to the average population, (ii) a higher biological age, e.g. 45 years, indicating that that said person's body shows more age-related changes in body function or composition as compared to the average population, or (iii) a biological age that is more or less identical to the person's chronological age, indicating that this person's body shows age-related changes in body function or composition that correspond largely to those of the average population.

The biomarkers determined in the methods of the present invention, as well as methods for determining the biological age of a human being based on the determined biomarkers will be extensively discussed hereinafter. In this context, the small roman numerals assigned to the biomarkers herein and in the claims are always to be seen in the context of whether the biomarkers are determined for females or males, *i.e.,* by way of example, biomarkers (i) to (x) used for females differ from biomarkers (i) to (x) used for males; further, the same biomarker may have one numeral when used for females, and another when used for males. Sometimes hereinafter, biomarkers used for females or males are referred to as female or male biomarkers. Table 1 below provides the respective numerals for all biomarkers used in the present invention.

In a preferred embodiment of the method of the present invention, in case the human being is female, in step (b1) one or more of the following biomarkers (xi) to (xl) are determined in addition to the biomarkers (i) to (x), and the necessary biological samples are provided in step (a):

(xi) cumulative level of cytosine methylation at gene positions ELOVL_2_CpG_9 in blood cells;
(xii) cumulative level of cytosine methylation at gene positions FHL_2_CpG_19 and FHL_2_CpG_20 in blood cells;

(xiii) cumulative level of cytosine methylation at gene positions FHL_2_CpG_9 and FHL_2_CpG_10 in blood cells;

(xiv) cumulative level of cytosine methylation at gene positions ELOVL_2_CpG_18, ELOVL_2_CpG_19, ELOVL_2_CpG_20 and ELOVL_2_CpG_21 in blood cells;

(xv) cumulative level of cytosine methylation at gene positions ELOVL_2_CpG_22, ELOVL_2_CpG_23 and ELOVL_2_CpG_24 in blood cells;

(xvi) cumulative level of cytosine methylation at gene positions ELOVL_2_CpG_10 in blood cells;

(xvii) concentration of s_p2_n_glycan in serum;

(xviii) concentration of s_p1_n_glycan in serum;

(xix) cumulative level of cytosine methylation at gene positions FHL_2_CpG_18 in blood cells;

(xx) concentration of fibrinogen in plasma;

(xxi) cumulative level of cytosine methylation at gene positions ELOVL_2_CpG_2 and ELOVL_2_CpG_3 in blood cells;

(xxii) cumulative level of cytosine methylation at gene positions ELOVL_2_CpG_5 in blood cells;

(xxiii) cumulative level of cytosine methylation at gene positions ELOVL_2_CpG_6 and ELOVL_2_CpG_7 in blood cells;

(xxiv) concentration of urea in plasma;

(xxv) cumulative level of cytosine methylation at gene positions ELOVL_2_CpG_8 in blood cells;

(xxvi) concentration of glucose in serum;

(xxvii) accumulated concentration of threonine and lactate in urine;

(xxviii) concentration of triglycerides in serum;

(xxix) concentration of VLDL2 triglycerides in serum;

(xxx) concentration of uric acid in whole blood

(xxxi) concentration of VLDL2 cholesterol in serum;

(xxxii) cumulative level of cytosine methylation at gene positions ELOVL_2_CpG_28 and ELOVL_2_CpG_29 in blood cells;

(xxxiii) concentration of immunoglobulin M (IgM) in serum

(xxxiv) concentration of uric acid in plasma;

(xxxv) concentration of VLDL cholesterol in serum;

(xxxvi) cumulative level of cytosine methylation at gene positions FHL_2_CpG_5 in blood cells;

(xxxvii) cumulative level of cytosine methylation at gene positions ELOVL_2_CpG_27 in blood cells;

(xxxviii) concentration of 8-isoprstane in urine;

(xxxix) concentration of creatinine in urine

(xl) cumulative level of cytosine methylation at gene positions ELOVL_2_CpG_34, ELOVL_2_CpG_35 and ELOVL_2_CpG_36 in blood cells;

In a further preferred embodiment of the method of the present invention, in case the human being is male, in step (b1) one or more of the following biomarkers (xi) to (xl) are determined in addition to the biomarkers (i) to (x), and the necessary biological samples are provided in step (a):

(xi) cumulative level of cytosine methylation at gene positions ELOVL_2_CpG_9 in blood cells;

(xii) cumulative level of cytosine methylation at gene positions FHL_2_CpG_19, and FHL_2_CpG_20 in blood cells;

(xiii) cumulative level of cytosine methylation at gene positions FHL_2_CpG_9, and FHL_2_CpG_10 in blood cells;

(xiv) cumulative level of cytosine methylation at gene positions ELOVL_2_CpG_10 in blood cells;

(xv) cumulative level of cytosine methylation at gene positions ELOVL_2_CpG_18, ELOVL_2_CpG_19, ELOVL_2_CpG_20 and ELOVL_2_CpG_21 in blood cells;

(xvi) cumulative level of cytosine methylation at gene positions ELOVL_2_CpG_22, ELOVL_2_CpG_23 and ELOVL_2_CpG_24 in blood cells;

(xvii) cumulative level of cytosine methylation at gene positions ELOVL_2_CpG_6 and ELOVL_2_CpG_7 in blood cells;

(xviii) cumulative level of cytosine methylation at gene positions FHL_2_CpG_18 in blood cells;

(xix) cumulative level of cytosine methylation at gene positions ELOVL_2_CpG_8 in blood cells;

(xx) cumulative level of cytosine methylation at gene positions ELOVL_2_CpG_2 and ELOVL_2_CpG_3 in blood cells;

(xxi) cumulative level of cytosine methylation at gene positions ELOVL_2_CpG_5 in blood cells;

(xxii) cumulative level of cytosine methylation at gene positions ELOVL_2_CpG_28 and ELOVL_2_CpG_29 in blood cells;

(xxiii) cumulative level of cytosine methylation at gene positions ELOVL_2_CpG_27 in blood cells;

(xxiv) concentration of Albumin in plasma;

(xxv) concentration of fibrinogen in plasma;
(xxvi) concentration of glucose in serum;
(xxvii) concentration of creatinine in urine;
(xxviii) concentration of s_p2_n_glycan in serum;
(xxix) concentration of 8-isoprstane in urine;
(xxx) Red blood cells (RBC) in whole blood;
(xxxi) cumulative level of cytosine methylation at gene positions FHL_2_CpG_5 in blood cells;
(xxxii) ratio of the concentration of Cu in plasma to the concentration of Zn in plasma;
(xxxiii) cumulative level of cytosine methylation at gene positions ELOVL_2_CpG_34, ELOVL_2_CpG_35 and ELOVL_2_CpG_36 in blood cells;
(xxxiv) cumulative level of cytosine methylation at gene positions ELOVL_2_CpG_33 in blood cells;
(xxxv) concentration of s_p1_n_glycan in serum;
(xxxvi) concentration of urea in plasma;
(xxxvii) concentration of VLDL cholesterol in serum;
(xxxviii) concentration of immunoglobulin M (IgM) in serum;
(xxxix) concentration of VLDL2 cholesterol in serum;
(xl) concentration of uric acid in whole blood

The present invention has been realized based on the findings of a multinational population study (sometimes referred to as "the study underlying the present invention" hereinafter) on approximately 3,300 subjects from eight European countries which aims to identify a set of biomarkers of aging which, as a combination of parameters with appropriate weighting, can measure biological age better than any marker in isolation. Two groups of subjects were recruited, *i.e.* a large randomly recruited age-stratified group of individuals from the general population (labeled "RASIG"; ratio male : female = 1:1), covering the chronological age range of 35 to 74 years, and, for reasons of comparison and validation, a group of subjects born from a long-living parent and belonging to a family with long living sibling(s) already recruited in the framework of the EU Integrated Project GeHA (Genetics of Healthy Aging). For genetic reasons, such individuals ("GeHA offspring" or short "GO" hereinafter) are expected to age at a slower rate.

Approximately 300 different clinical chemistry, genetic, cellular or molecular biomarkers were tested, including classical ones for which a correlation with age has been known, new ones that are based on recent findings, and parameters that are based on recent research on mechanistic aspects of aging, conducted by study participants. All analyses have been done on coded samples. Analyses resulted in a large amount of data containing the values for the determined biomarkers for all study subjects (sometimes referred to as "the data provided in the study underlying the present invention" hereinafter).

Bioinformatics was used in order to extract a robust set of biomarkers of human aging from the large amounts of data obtained. This was done by using a graphical workbench for the entire analysis process, including data access in databases, data transformation, initial investigation, powerful predictive analytics, visualization and reporting. Further, the "leaps" package which is a component of the free statistics software "R" was used for statistical analysis and calculations. This package can perform an exhaustive search for the best subsets of the variables in x for predicting y in linear regression, using an efficient branch-and-bound algorithm. Furthermore, the package "stats" which is also a component of "R", was used for the fitting of linear models. This package can be used to carry out regression, single stratum analysis of variance and analysis of covariance.

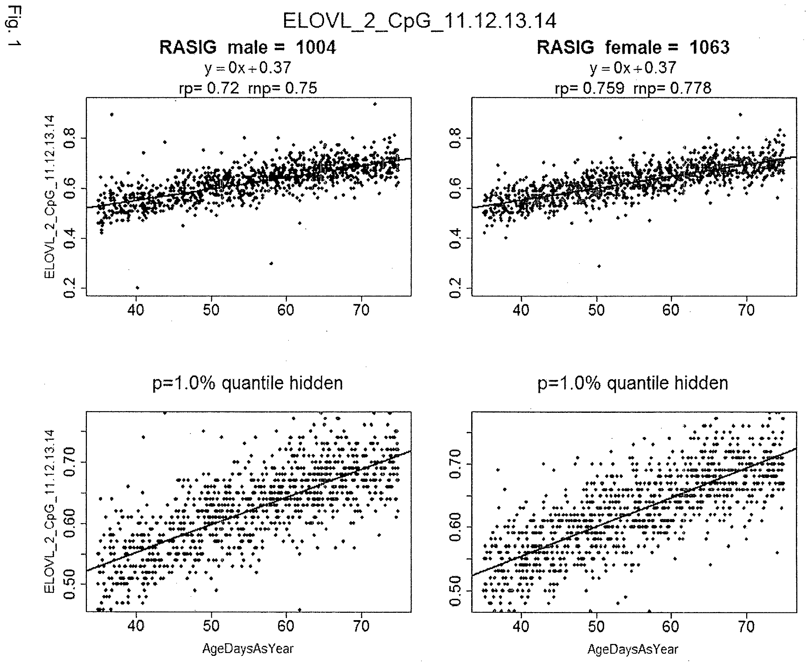

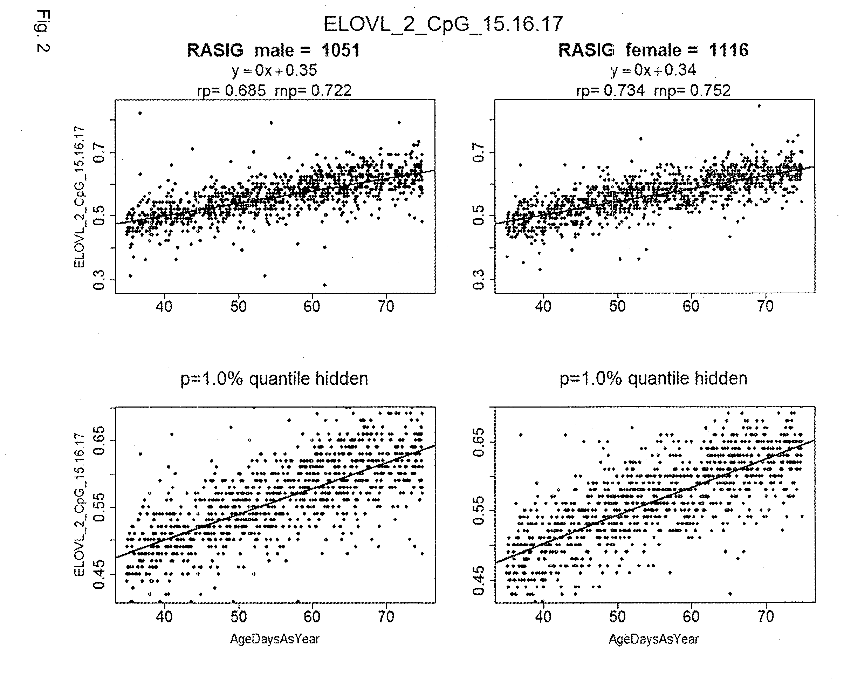

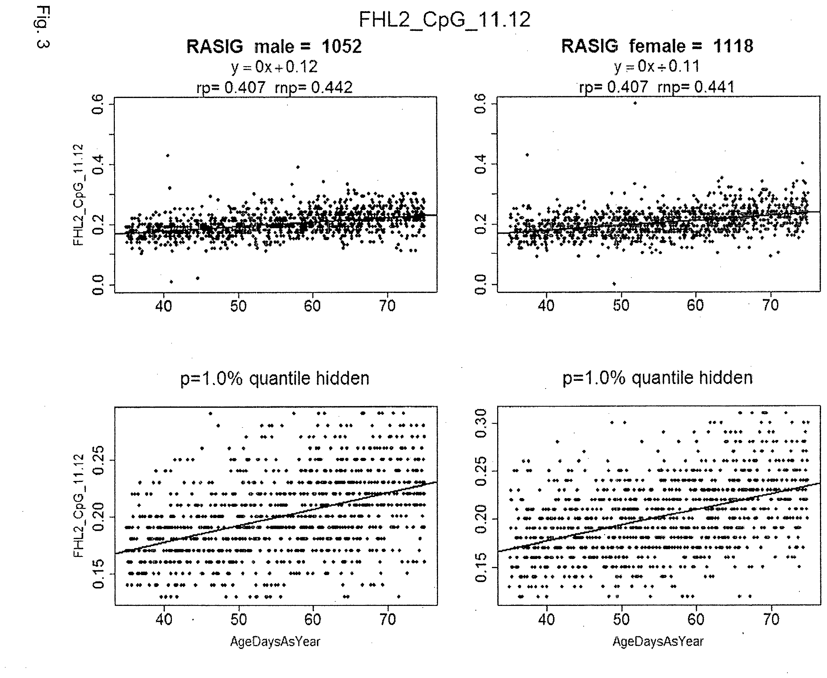

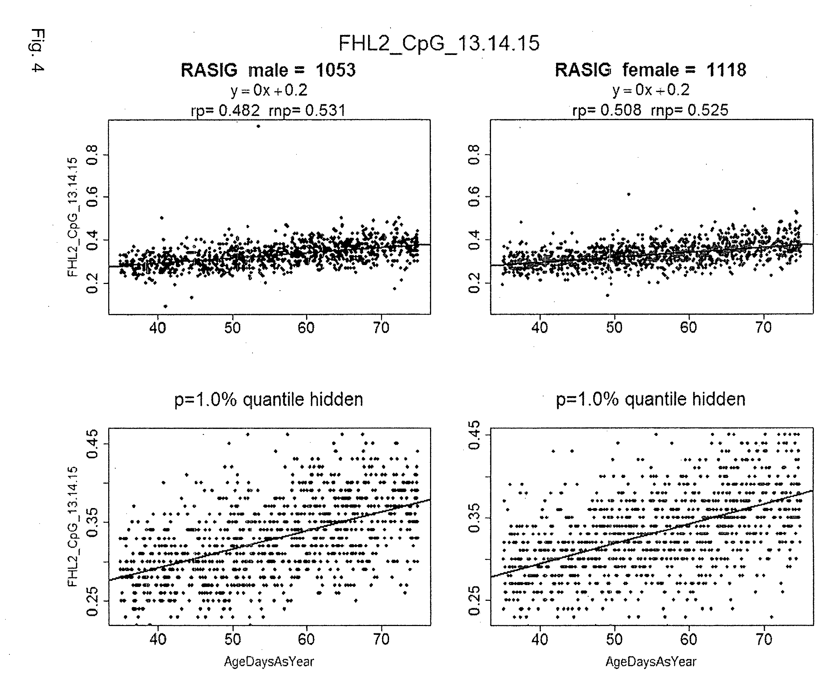

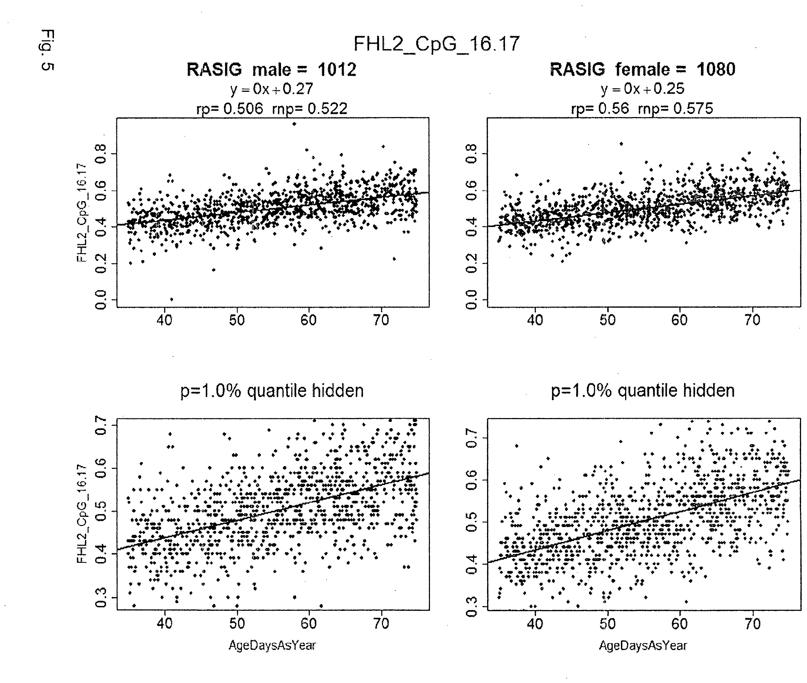

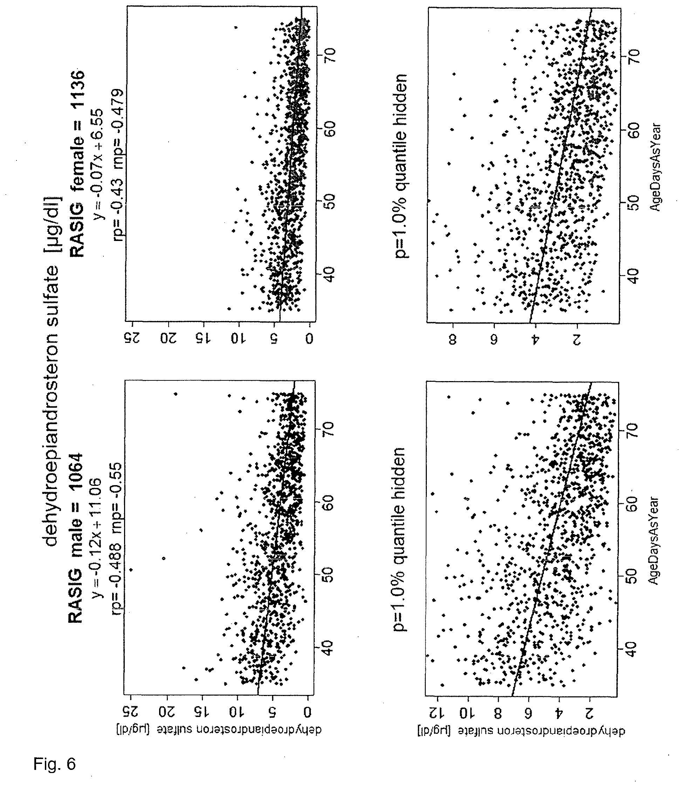

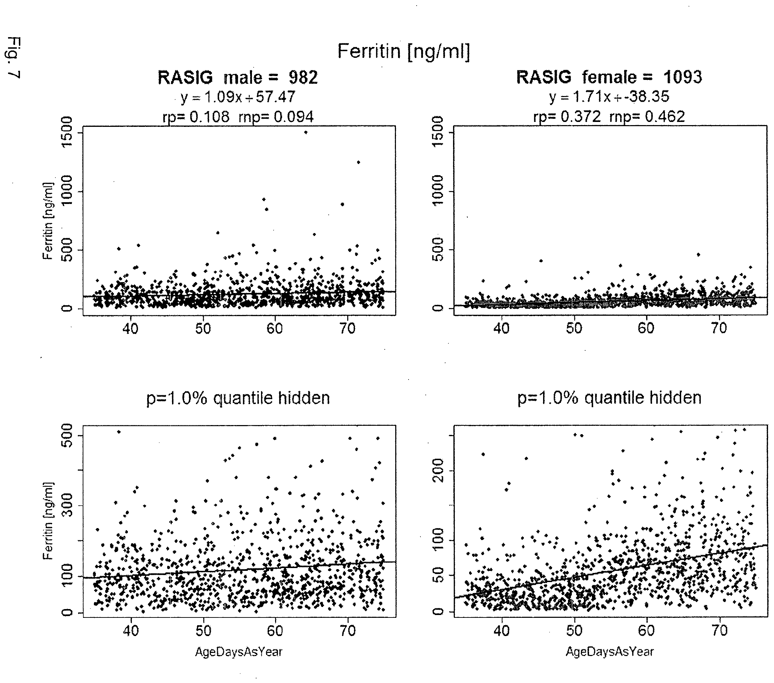

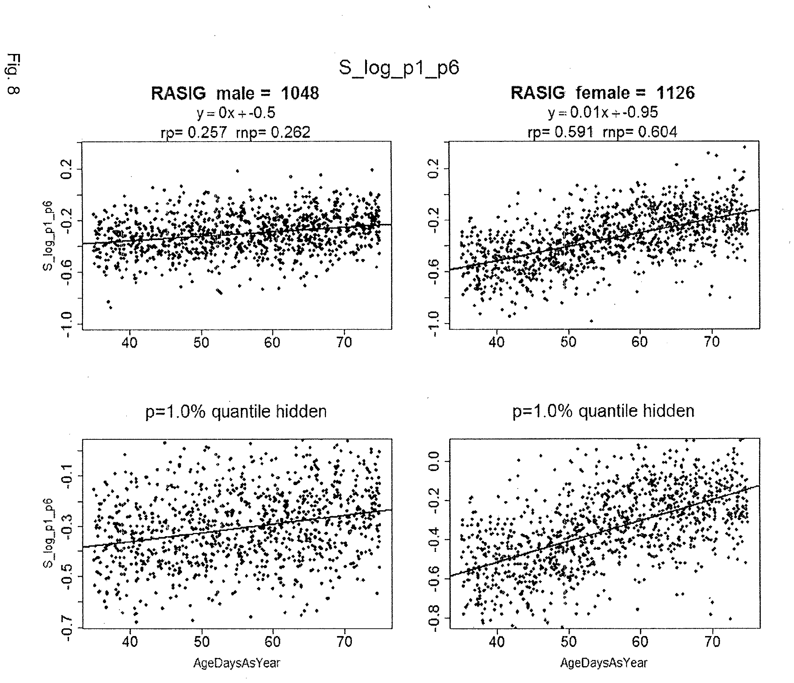

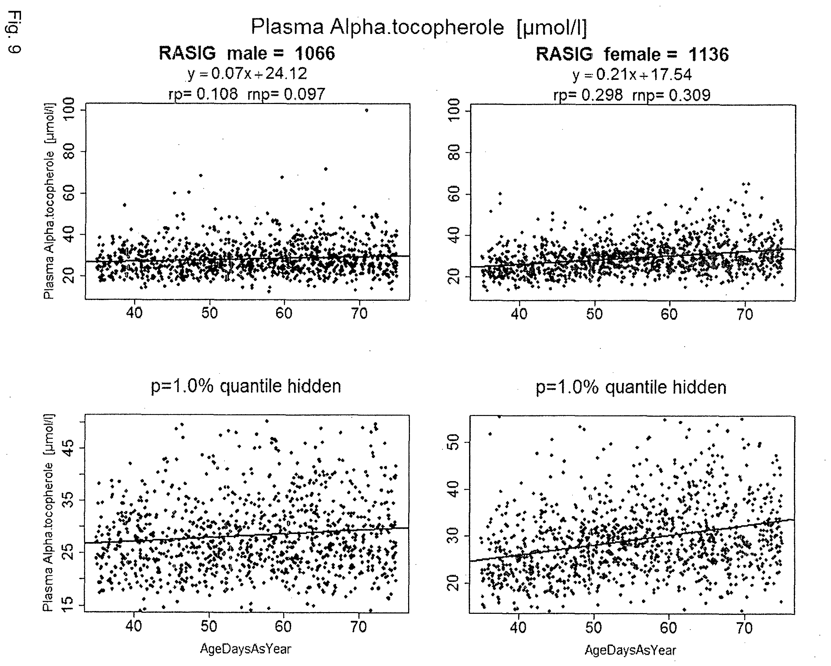

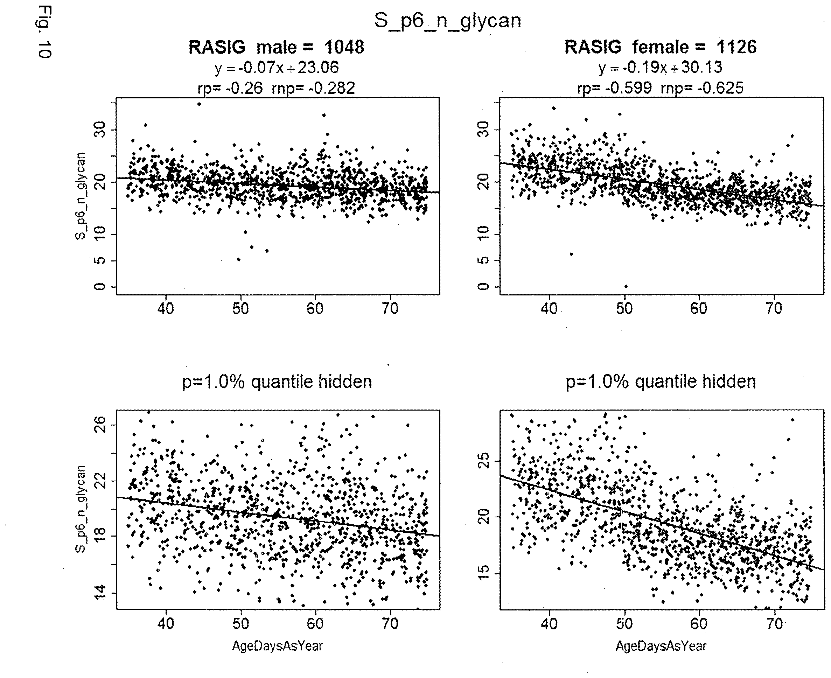

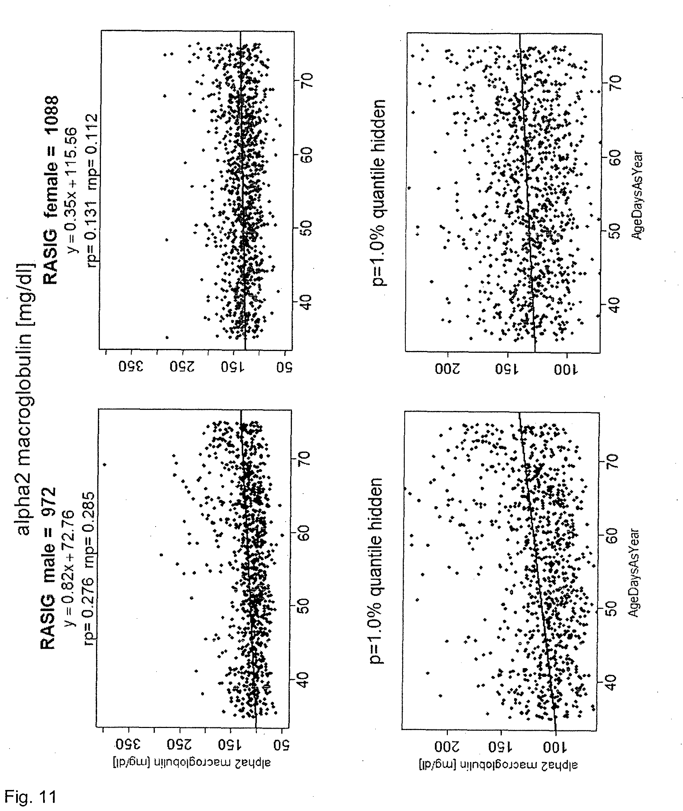

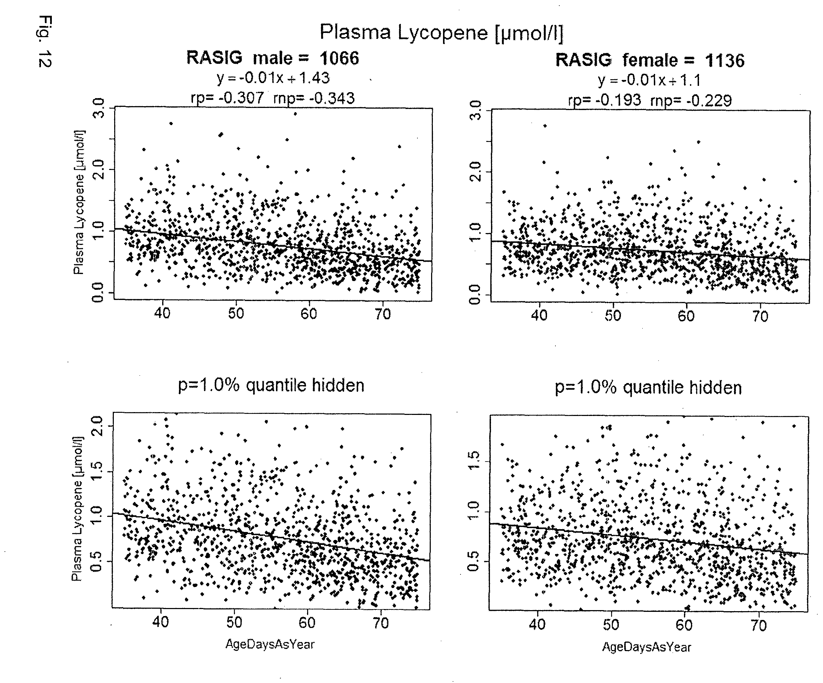

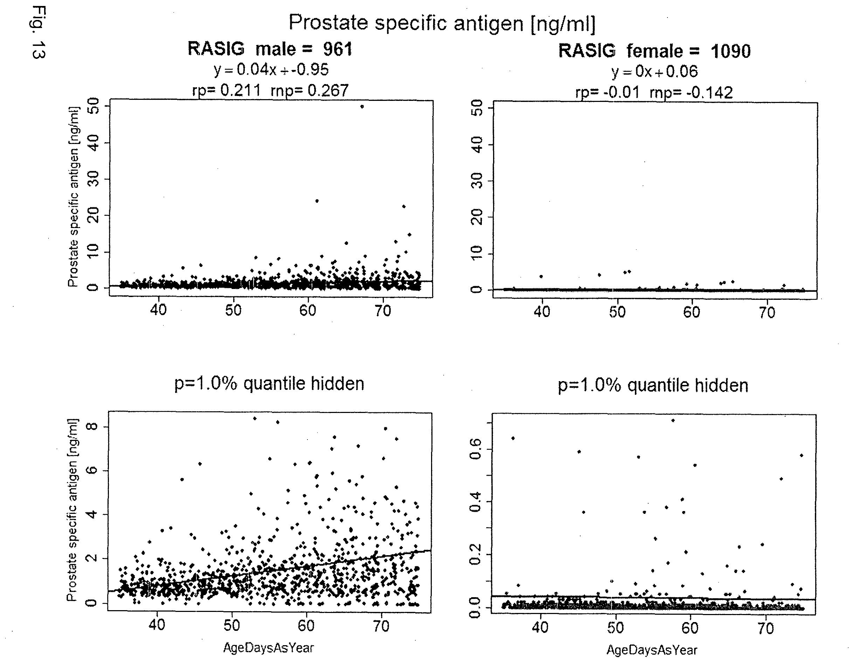

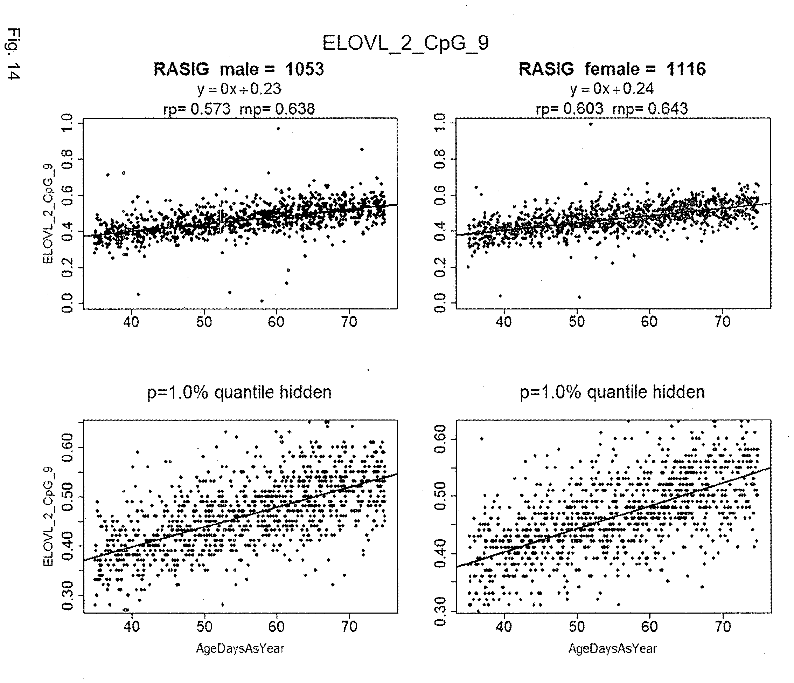

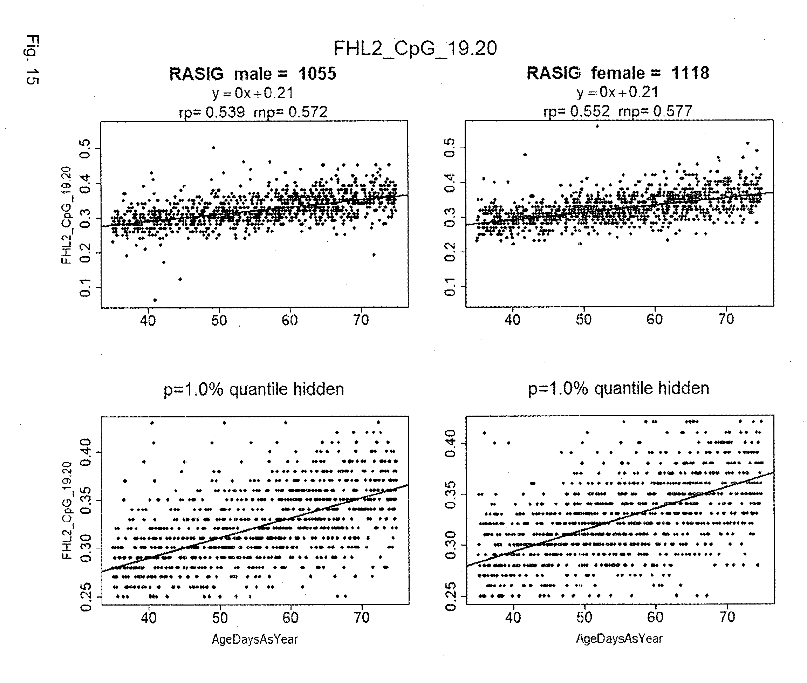

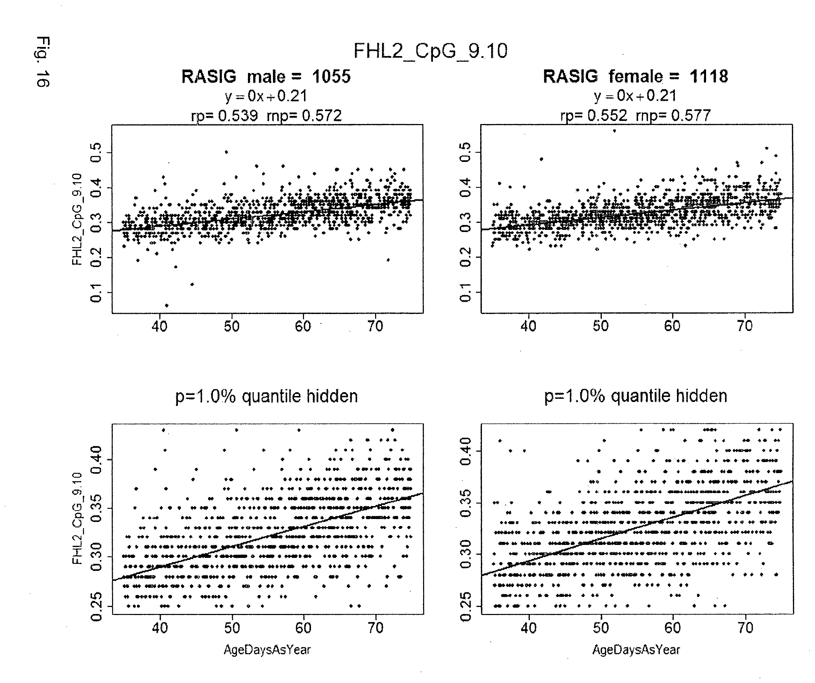

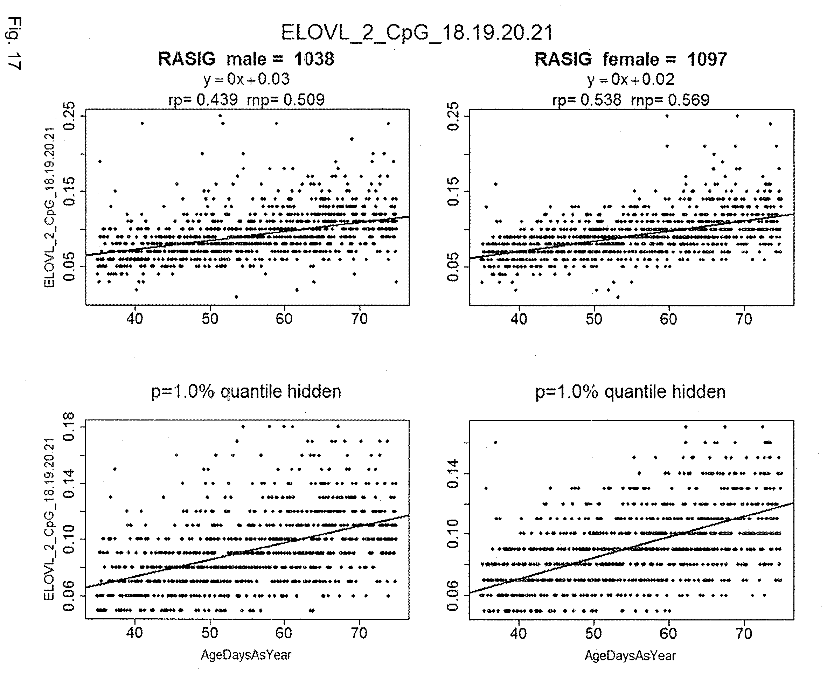

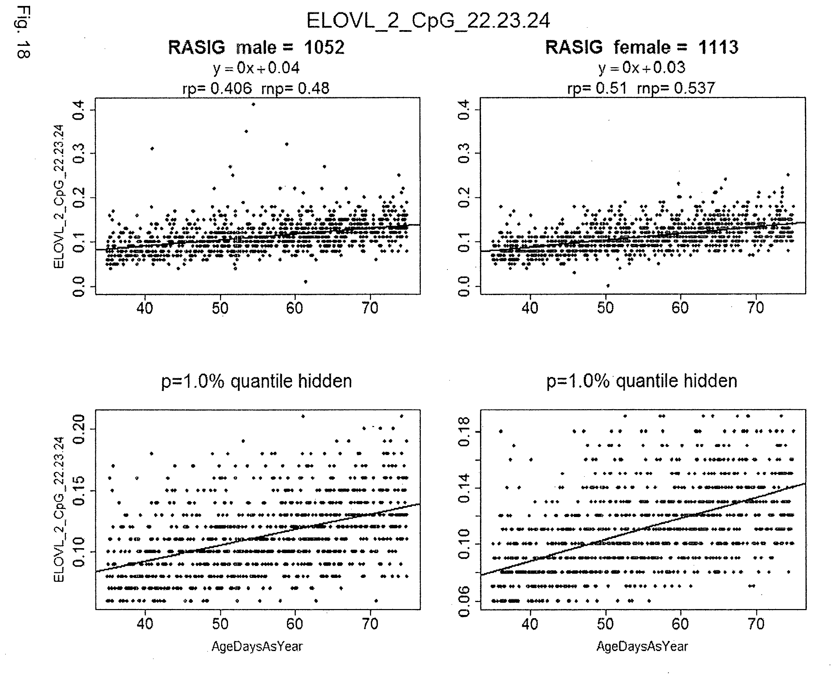

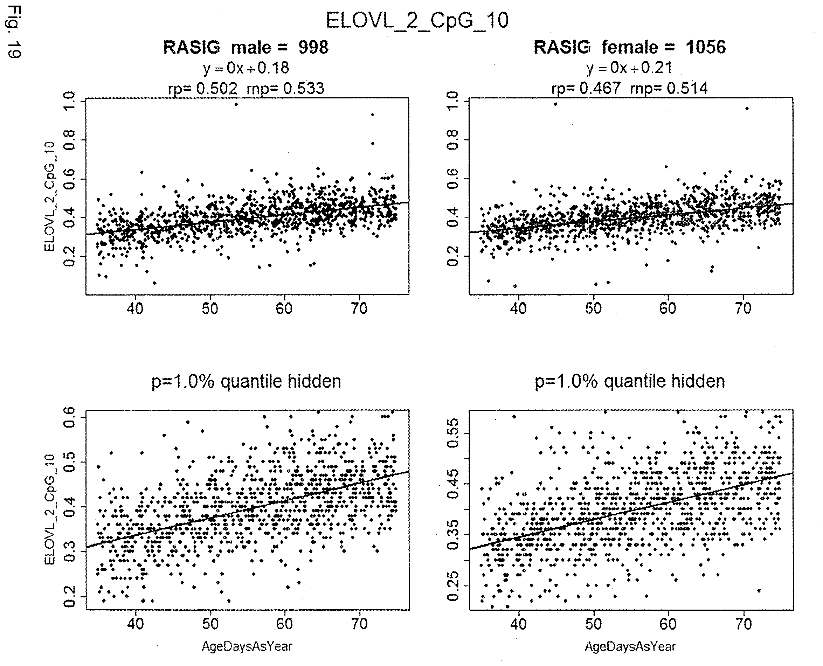

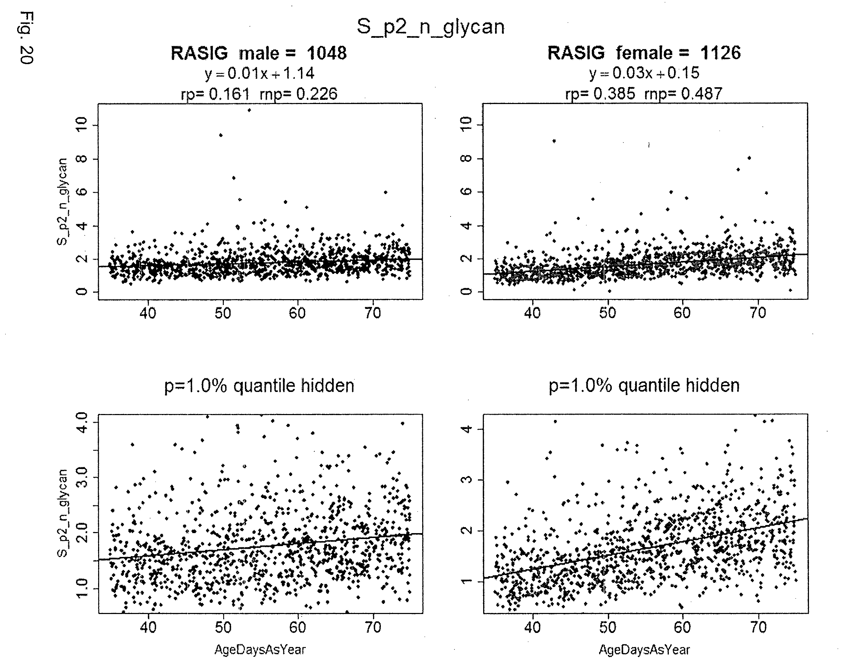

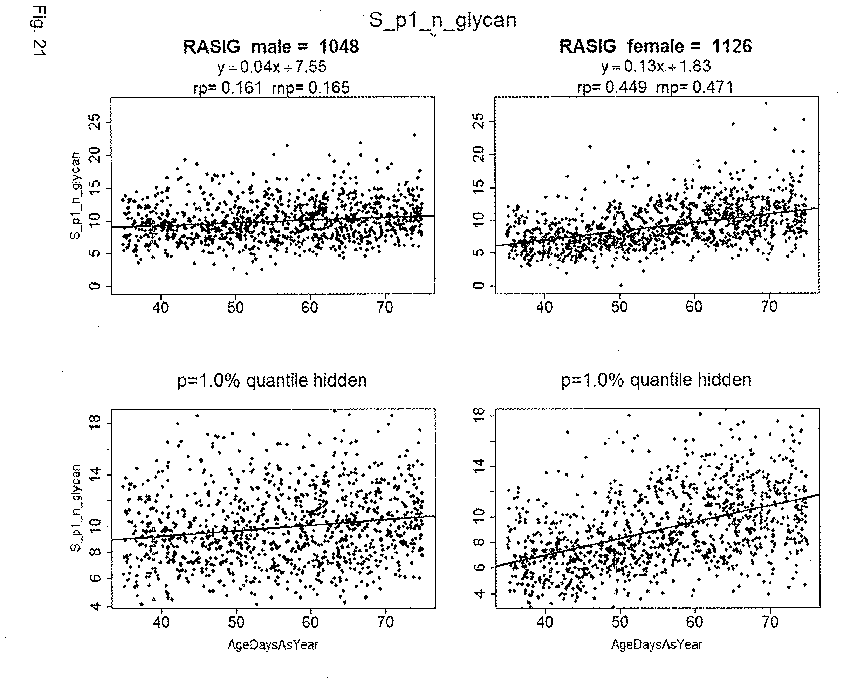

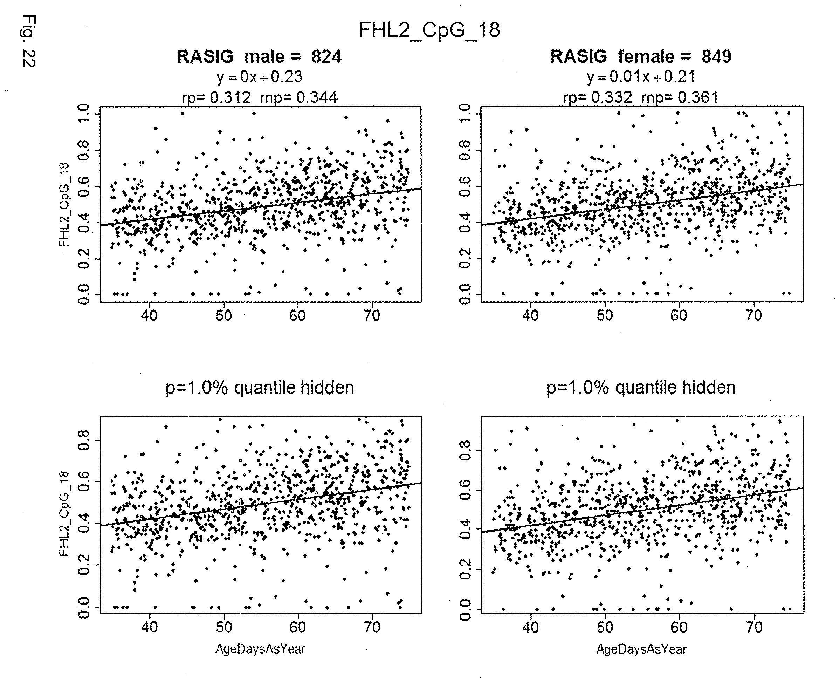

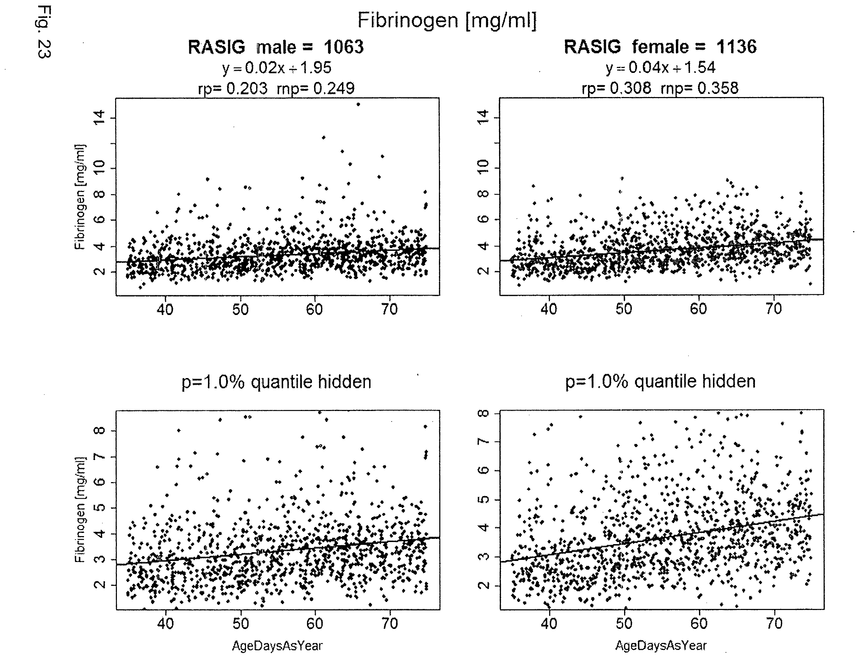

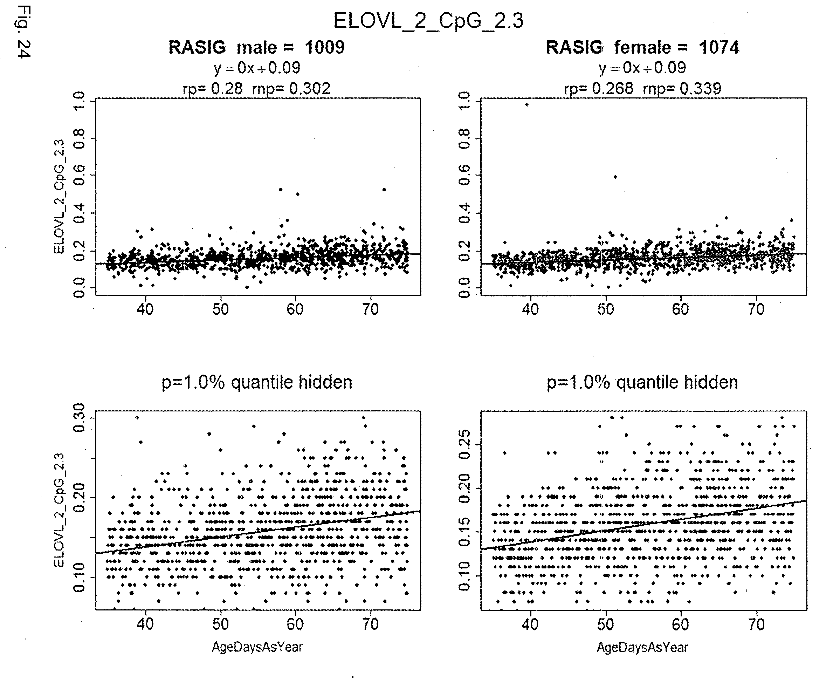

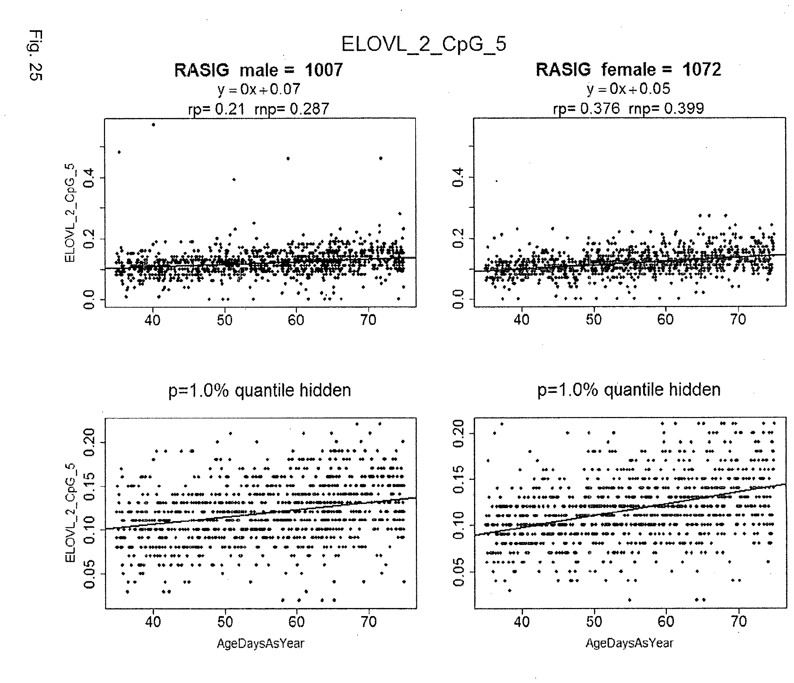

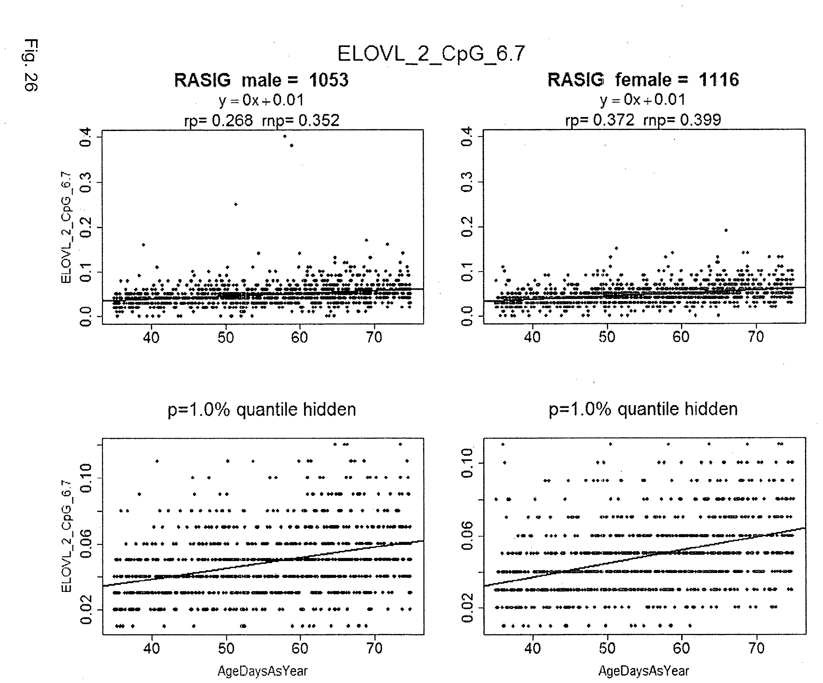

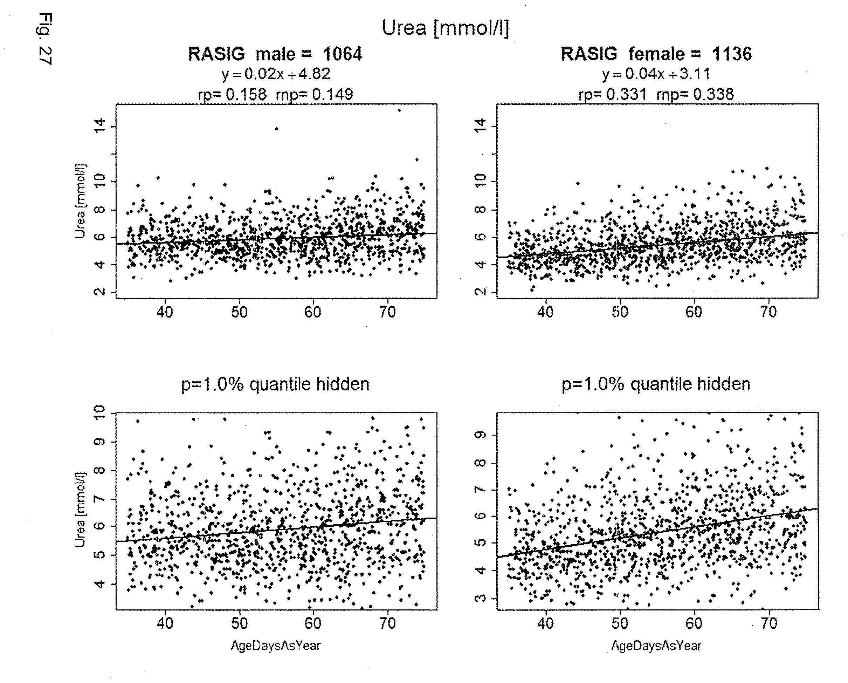

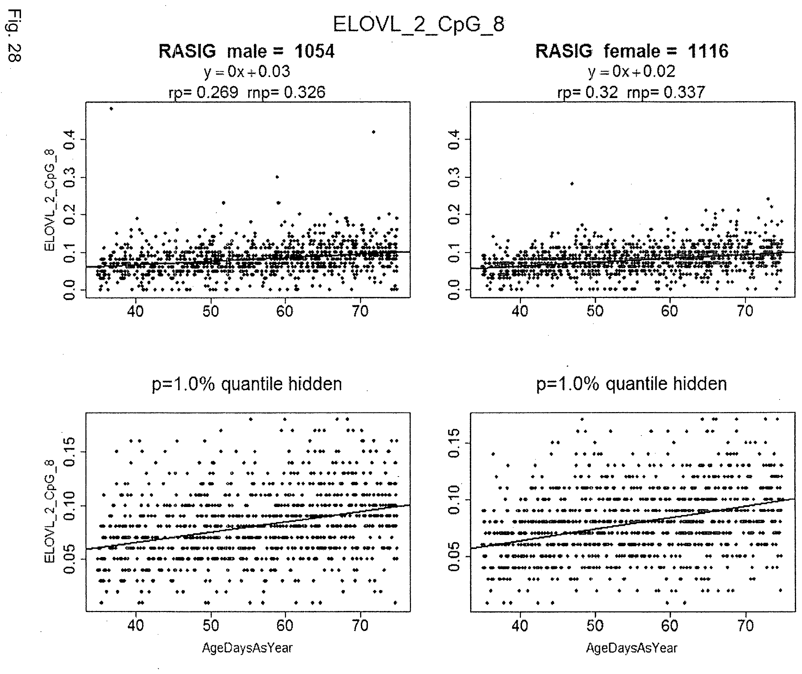

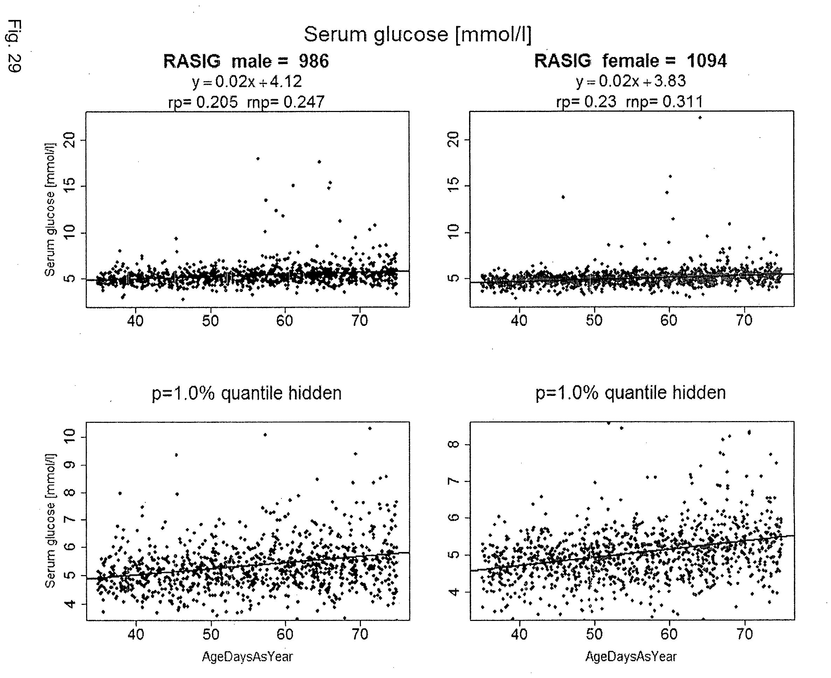

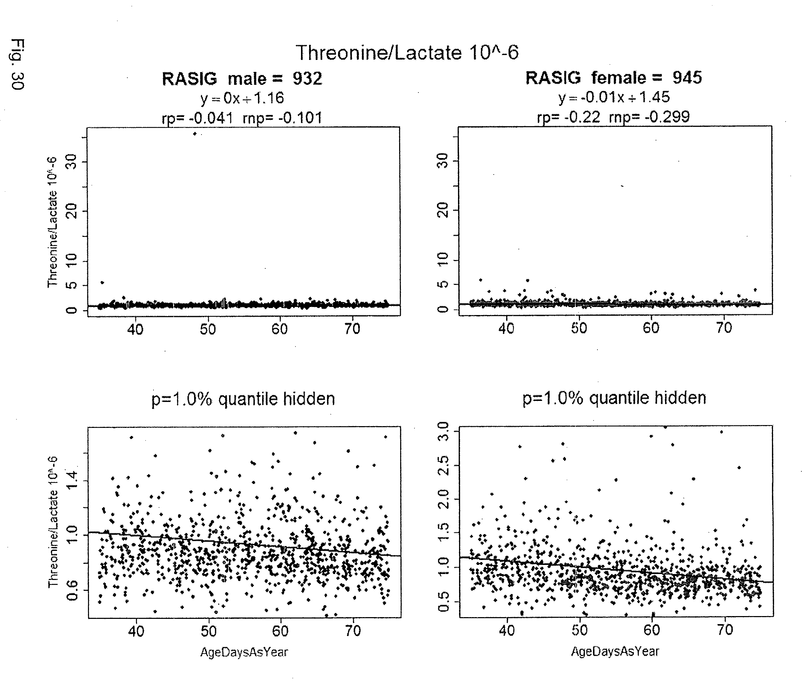

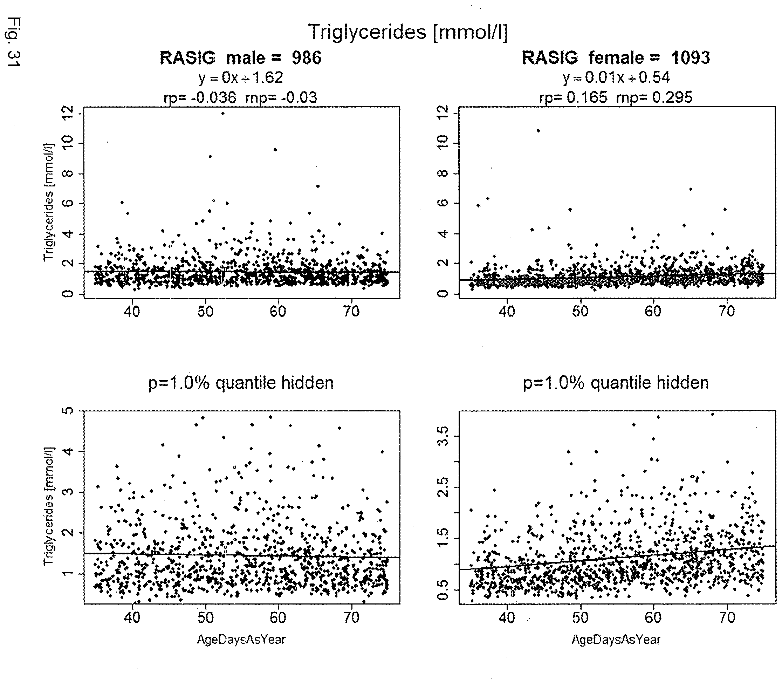

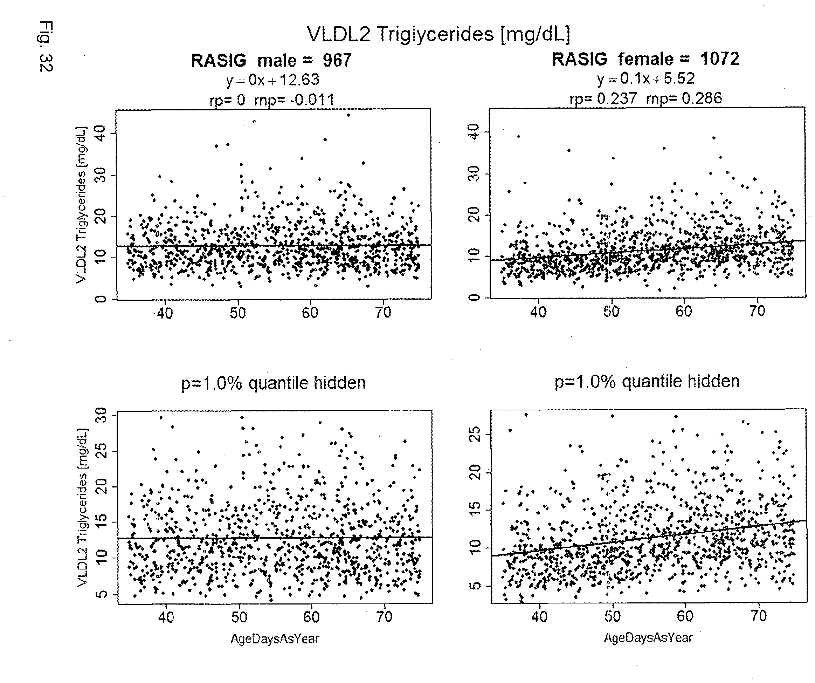

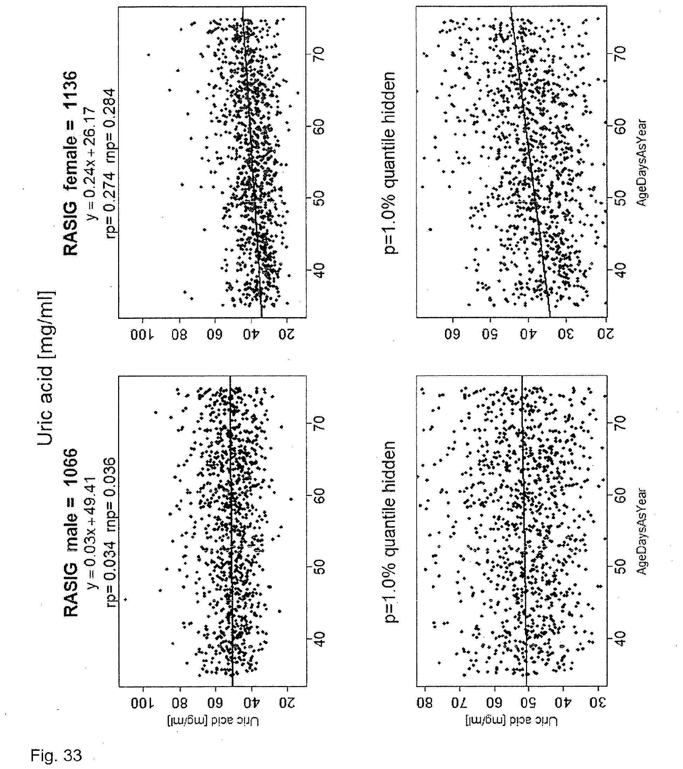

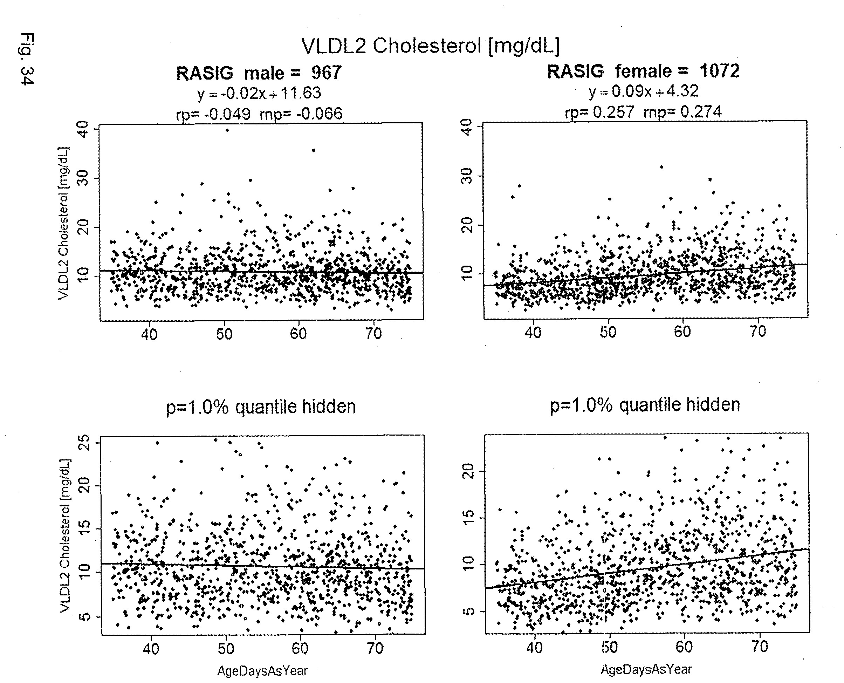

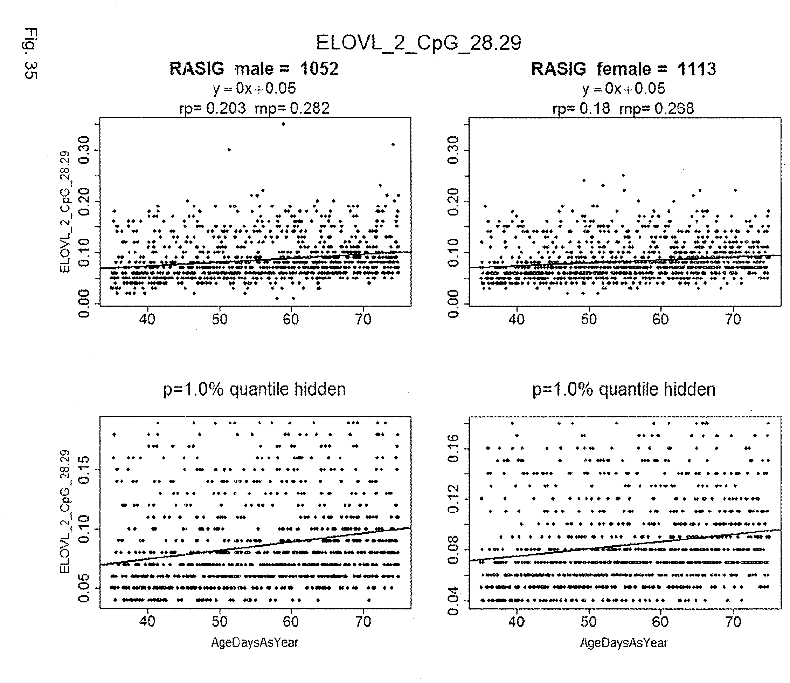

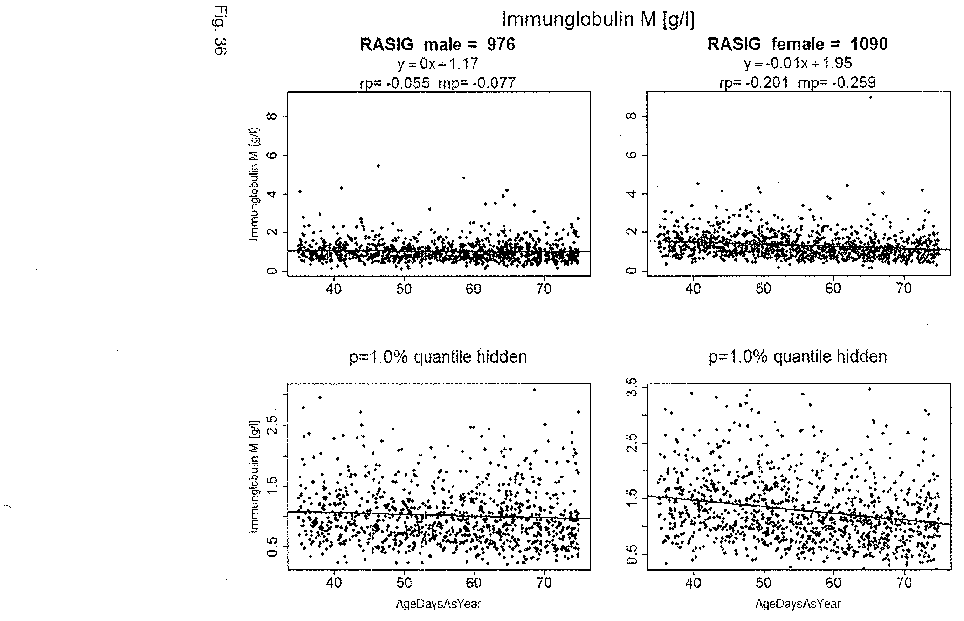

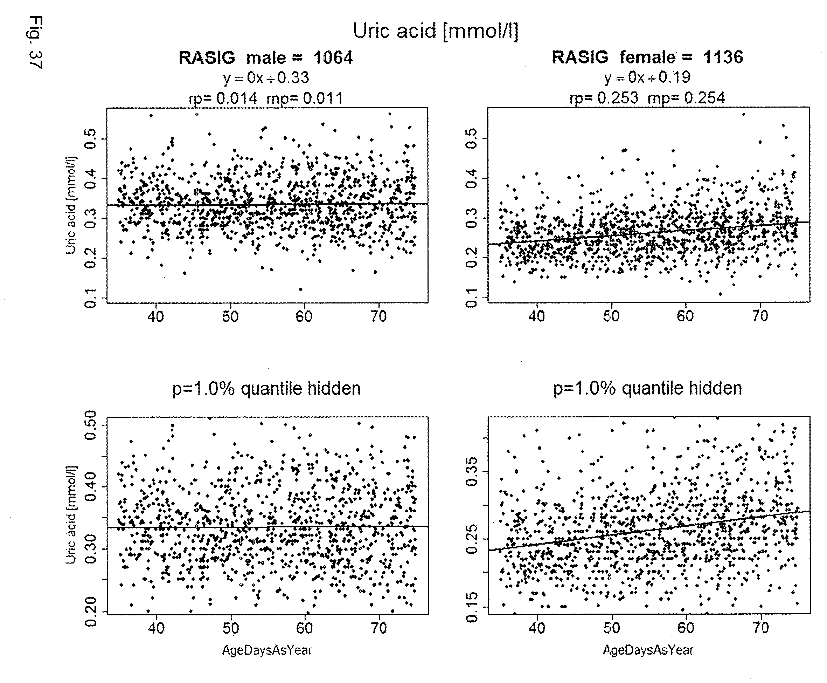

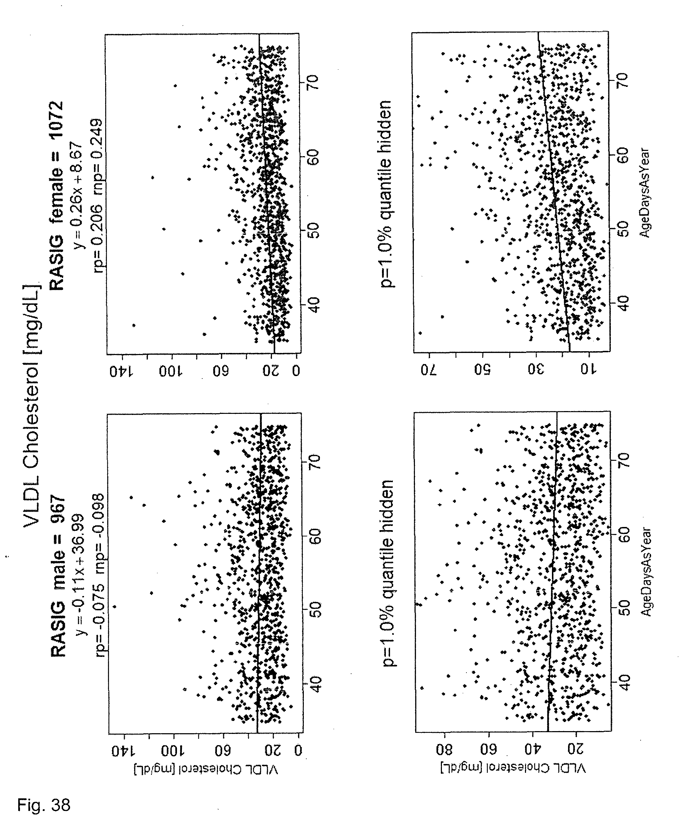

More specifically, in the study underlying the present invention, a correlation between each determined biomarker and age has been determined, resulting in (i) a parametric coefficient of correlation (rp), (ii) a non-parametric coefficient of correlation (rnp), (iii) the slope of the linear regression curve (a), and (iv) the intercept of the linear regression curve (c). This was done separately for female and male subjects, since sex differences in the quality of biomarkers were expected. The non-parametric coefficient of correlation (rnp) for the biomarkers used in the present invention is shown in Table 2, below, and the respective graphs are shown in Figures 1 to 38. Then, the 30 biomarkers showing the best correlation with age were chosen, independent of whether the correlation was positive or negative. Subsequently, a regression subset analysis for the chosen parameters was performed, resulting in a subset of the ten best biomarkers as far as the capacity to predict the chronological age of the subjects was concerned. Next, a Z-score scaling was applied to these ten parameters and a multiple linear regression model was build, resulting in a weighting factor for each scaled biomarker (weighting factors $b_1$ to $b_{10}$), a correlation coefficient $r^2$ for the multiple linear regression, and an intercept c for the multiple linear regression. Based on these data, the biological age of any individual subject can be calculated according to the formula

$$B = \sum_{i=1}^{10} b_i z_i + c$$

wherein B is the apparent biological age, $z_1$ to $z_{10}$ are the scaled values of $x_1$ to $x_{10}$ which are the determined values of the respective biomarkers. The scaling is defined according to the rule

$$z_i = \frac{x_i - \overline{x}_i}{\sigma_i}$$

where $\overline{x}_i$ is the arithmetic average and $\sigma_i$ the standard deviation of biomarker i in the RASIG population used in the study. In order to alleviate the usage of the biological age values, a strictly monotone transformation is applied to the apparent biological age which ensures that the average biological age approximately equals the chronological age in randomly selected even-aged groups of individuals. The transformation is given by the formula

$$Bioage = \frac{B - mean(a)}{r^2} + mean(a)$$

wherein Bioage is the actual, corrected biological age and mean(a) is the mean age of the whole test population.

However, according to the embodiments described above, one or more additional biomarkers can be used for the calculation of the biological age of a human being, *i.e.* for females one or more of the above biomarkers (xi) to (xl), and for males one or more of the above biomarkers (xi) to (xl). In case any of these additional biomarkers are used, in step (c) determining the biological age of the human being based on the biomarkers determined in step (b1)/(b2) comprises the steps:

(c1) establishing a multiple linear regression model for the determined, scaled biomarkers based on the data provided in the study underlying the present invention;
(c2) determining from the multiple linear regression model established in step (c1)

(i) the correlation coefficient $r^2$,
(ii) the weighting factors $b_i$ for each determined biomarker, wherein i is 1 through n, and n is the number of biomarkers determined, and
(iii) the intercept c;

(c3) calculating an apparent biological age B according to formulas (1a) and (1 b)

$$z_i = \frac{x_i - \overline{x}_i}{\sigma_i} \qquad \text{(1a)}$$

$$B = \sum_{i=1}^{n} b_i z_i + c \quad \text{(1b)}$$

wherein

$b_i$　　are the weighting factors determined in step (c2),
$x_i$　　are the values of the respective biomarkers as determined in step (b1)/(b2), wherein i is 1 to n and
$\overline{x}_i$　　are the arithmetic averages of the respective biomarker values as determined in step (c1) and
$\sigma_i$　　are the standard deviations of the respective biomarker values as determined in step (c1) and
$z_i$　　are the Z-scaled values of the respective biomarker values as determined in step (c1), wherein i is 1 to n and
c　　is the intercept determined in step (c2); and

(c4) calculating the corrected biological age (Bioage) according to formula (2)

$$Bioage = \frac{B - mean(a)}{r^2} + mean(a) \qquad (2)$$

wherein

B is the apparent biological age determined in step (c3), and

mean(a) is the mean chronological age of the statistical population used in the study underlying the present invention.

In this embodiment, establishing a multiple linear regression model for the determined biomarkers based on the data provided in the study underlying the present invention, and thus determining the parameters $r^2$, $b_1$ to $b_n$, and c, is not particularly limited and can be done with statistical methods known in the art. As an example, the package "stats" which is a component of the free software "R" can be used in this respect.

In another aspect of the present invention, any number n of the above biomarkers (i) to (xl) for females or of the above biomarkers (i) to (xl) for males can be used for the determination of the biological age of a human being, provided that n is at least 3, at least 4, at least 5, at least 6, at least 7, at least 8, at least 9, or at least 10. In these embodiments, step (c) comprises the steps (c1) to (c4) as described above. In this context, it is emphasized that the above parameter $r^2$ as determined from the multiple linear regression model established in step (c1) is a measure for the correlation of the biological age determined with a particular subset of biomarkers with the chronological age. Accordingly, the quality of any given subset of biomarkers, *i.e.* the ability of said subset to predict the biological age, can be determined. Thus, respective high quality subset of biomarkers can be identified and chosen for further use.

In this context, the above female and male biomarkers (i) to (x) have been identified in the study underlying the present invention to result in a particularly good correlation with chronological age and, thus, in a particularly good determination of the biological age. This is based on a definition of biological age as the expected chronological age given only biological information.

Therefore, in a particularly preferred embodiment of the present invention, the human being is female, the biomarkers (i) to (x) are determined in step (b1), and determining the biological age of the human being based on said biomarkers in step (c) comprises the steps of:

(c1) calculating an apparent biological age B according to formulas (3a) and (3b)

$$z_i = \frac{x_i - \bar{x}_i}{\sigma_i} \qquad (3a)$$

$$B = \sum_{i=1}^{n} b_i z_i + c \qquad (3b)$$

wherein

$x_1$ is the determined value of biomarker (i), preferably expressed as a percentage between 0 and 1,
$x_2$ is the determined value of biomarker (ii), preferably expressed as a percentage between 0 and 1,
$x_3$ is the determined value of biomarker (iii), preferably expressed as a percentage between 0 and 1,
$x_4$ is the determined value of biomarker (iv), preferably expressed as a percentage between 0 and 1,
$x_5$ is the determined value of biomarker (v), preferably expressed as a percentage between 0 and 1,
$x_6$ is the determined value of biomarker (vi), preferably expressed in $\mu$g per dl,
$x_7$ is the determined value of biomarker (vii), preferably expressed in ng per ml,
$x_8$ is the determined value of biomarker (viii),
$x_9$ is the determined value of biomarker (ix), preferably expressed in $\mu$mole per l,
$x_{10}$ is the determined value of biomarker (x); and

(c2) calculating the corrected biological age (Bioage) according to formula (2)

$$Bioage = \frac{B - mean(a)}{r^2} + mean(a) \qquad\qquad (2)$$

wherein

B is the apparent biological age determined in step (c1).

The scaling parameters to determine the Z-score have the following values:

$\overline{x}_1$ is 0.6273743,
$\overline{x}_2$ is 0.5658207,
$\overline{x}_3$ is 0.2035695,
$\overline{x}_4$ is 0.3319397,
$\overline{x}_5$ is 0.5037121,
$\overline{x}_6$ is 2.8052048,
$\overline{x}_7$ is 56.38858,
$\overline{x}_8$ is -0.351995,
$\overline{x}_9$ is 29.271944,
$\overline{x}_{10}$ is 19.363444,

and

$\sigma_1$ is 0.069951,
$\sigma_2$ is 0.063629,
$\sigma_3$ is 0.045639,
$\sigma_4$ is 0.053611,
$\sigma_5$ is 0.092218,
$\sigma_6$ is 1.78002,
$\sigma_7$ is 51.4718,
$\sigma_8$ is 0.205710,
$\sigma_9$ is 7.9826,
$\sigma_{10}$ is 3.65247.

Further, by way of a multiple linear regression model that has been established for the above biomarkers (i) to (x), the above parameters $b_1$ to $b_{10}$ have been determined to have the following values:

$b_1$ is 2.988,
$b_2$ is 2.900,
$b_3$ is-1.129,
$b_4$ is 1.580, $b_5$ is 1.295,
$b_6$ is -1.425,
$b_7$ is 1.379,
$b_8$ is 1.615,
$b_9$ is 0.818,
$b_{10}$ is-1.182,
c is 55.526, and
$r^2$ is 0.742.

Further, mean(a), *i.e.* the mean age of the test population examined in the study underlying the present invention, is 55.751 years.
Accordingly, in a preferred embodiment, the parameters $b_1$ to $b_{10}$, c, $r^2$, and mean(a) have the above values.
However, a person skilled in the art will understand that a change of the test population, will subject the above parameters to variation. Therefore, it will be understood that the above parameters may vary within a certain range, and as a consequence, also the final bioage value is subject to variation. A bootstrap analysis (with 10000 samples) reveals that the bioage data of female RASIG subjects collected in the study leads to a typical standard deviation of 0.85 years in bioage. The same bootstrap analysis yields ranges for the weight parameters $b_1$ to $b_{10}$ which give rise to a maximal variation of +/- 2 years in bioage for at least 95% of the subjects participating in the regression analysis.. Accordingly, the respective values in this range are intended to be encompassed in the present invention.

According to a particular embodiment,

$b_1$ is a number between 1.83 and 4.14,
$b_2$ is a number between 1.81 and 4.11,
$b_3$ is a number between -2.14 and -0.11,
$b_4$ is a number between 0.54 and 2.62,
$b_5$ is a number between 0.42 and 2.14,
$b_6$ is a number between -2.17 and -0.69,
$b_7$ is a number between 0.78 and 2.10,
$b_8$ is a number between 0.70 and 2.57,
$b_9$ is a number between 0.15 and 1.47,
$b_{10}$ is a number between -2.14 and -0.23,
c is a number between 54.73 and 56.28,
mean(a) is a number between 54.35 and 57.09, and
$r^2$ is a number between 0.68 and 0.79.

In a corresponding particularly preferred embodiment, the human being is male, the biomarkers (i) to (x) are determined in step (b2), and determining the biological age of the human being based on said biomarkers in step (c) comprises the steps of:

(c1) calculating an apparent biological age B according to formulas(3a) and (3b)

$$z_i = \frac{x_i - \overline{x}_i}{\sigma_i} \qquad (3a)$$

$$B = \sum_{i=1}^{n} b_i z_i + c \qquad (3b)$$

wherein

$x_1$ is the determined value of biomarker (i), preferably expressed as a percentage between 0 and 1,
$x_2$ is the determined value of biomarker (ii), preferably expressed as a percentage between 0 and 1,
$x_3$ is the determined value of biomarker (iii), preferably expressed as a percentage between 0 and 1,
$x_4$ is the determined value of biomarker (iv), preferably expressed as a percentage between 0 and 1,
$x_5$ is the determined value of biomarker (v), preferably expressed as a percentage between 0 and 1,
$x_6$ is the determined value of biomarker (vi), preferably expressed in $\mu$g per dl,
$x_7$ is the determined value of biomarker (vii), preferably expressed in mg per dl,
$x_8$ is the determined value of biomarker (viii), preferably expressed in $\mu$mole per I,
$x_9$ is the determined value of biomarker (ix), preferably expressed in ng per ml,
$x_{10}$ is the determined value of biomarker (x), and

(c2) calculating the corrected biological age (Bioage) according to the formula (2)

$$Bioage = \frac{B - mean(a)}{r^2} + mean(a) \qquad (2)$$

wherein

B is the apparent biological age determined in step (c1).

The scaling parameters to determine the Z-score have the following values:

$$\overline{x}_1 \quad \text{is } 0.6255050,$$
$$\overline{x}_2 \quad \text{is } 0.561652,$$
$$\overline{x}_3 \quad \text{is } 0.2002358,$$
$$\overline{x}_4 \quad \text{is } 0.3296136,$$
$$\overline{x}_5 \quad \text{is } 0.5024118,$$
$$\overline{x}_6 \quad \text{is } 4.478134,$$
$$\overline{x}_7 \quad \text{is } 118.8759,$$
$$\overline{x}_8 \quad \text{is } 0.7709553,$$
$$\overline{x}_9 \quad \text{is } 1.502275,$$
$$\overline{x}_{10} \quad \text{is } 19.36541,$$

and

$$\sigma_1 \quad \text{is } 0.071819,$$
$$\sigma_2 \quad \text{is } 0.064699,$$
$$\sigma_3 \quad \text{is } 0.040895,$$
$$\sigma_4 \quad \text{is } 0.055907,$$
$$\sigma_5 \quad \text{is } 0.094050,$$
$$\sigma_6 \quad \text{is } 2.75790,$$
$$\sigma_7 \quad \text{is } 34.0785,$$
$$\sigma_8 \quad \text{is } 0.436302,$$
$$\sigma_9 \quad \text{is } 2.38521,$$
$$\sigma_{10} \quad \text{is } 2.94662.$$

Further, by way of a multiple linear regression model that has been established for the above biomarkers (i) to (x), the above parameters $b_1$ to $b_{10}$ have been determined to have the following values:

$$b_1 \quad \text{is } 3.488,$$
$$b_2 \quad \text{is } 2.678,$$
$$b_3 \quad \text{is } -1.522,$$
$$b_4 \quad \text{is } 2.470,$$
$$b_5 \quad \text{is } 1.143,$$
$$b_6 \quad \text{is } -2.715,$$
$$b_7 \quad \text{is } 1.020,$$
$$b_8 \quad \text{is } -1.220,$$
$$b_9 \quad \text{is } 2.032, \quad b_{10} \quad \text{is } -1.225,$$
$$c \quad \text{is } 55.906, \text{ and}$$
$$r^2 \quad \text{is } 0.707.$$

Further, mean(a), *i.e.* the mean age of the test population examined in the study underlying the present invention, is 55.829.

Accordingly, in a preferred embodiment, the parameters $b_1$ to $b_{10}$, c, $r^2$, and mean(a) have the above values.

However, as has been stated above, a person skilled in the art will understand that an increase of the test population, will subject the above parameters to variation. Therefore, it will be understood that the above parameters may vary within a certain range, and as a consequence, also the final bioage value is subject to variation. A bootstrap analysis (with 10000 samples) reveals that the bioage data of male RASIG subjects collected in the study leads to a typical standard deviation of 1.04 years in bioage. The same bootstrap analysis yields ranges for the weight parameters

$b_1$ to $b_{10}$ which give rise to a maximal variation of +/- 2 years in bioage for at least 95% of the subjects participating in the regression analysis. Accordingly, the respective values in this range are intended to be encompassed in the present invention.

Thus, according to a particular embodiment,

| | |
|---|---|
| $b_1$ | is a number between 2.38 and 4.52, |
| $b_2$ | is a number between 1.54 and 3.69, |
| $b_3$ | is a number between -2.56 and -0.64, $b_4$ is a number between 1.38 and 3.70, |
| $b_5$ | is a number between 0.21 and 2.05, |
| $b_6$ | is a number between -3.45 and -2.05, |
| $b_7$ | is a number between 0.30 and 1.79, |
| $b_8$ | is a number between -1.85 and -0.57, |
| $b_9$ | is a number between 0.98 and 3.24, |
| $b_{10}$ | is a number between -1.86 and -0.62, |
| c | is a number between 55.21 and 56.65,; and |
| mean(a) | is a number between 54.54 and 57.27, and |
| $r^2$ | is a number between 0.64 and 0.77. |

The test population used in the study underlying the present invention had chronological ages between 30 and 80 years. Accordingly, the human being for which the biological age should be determined with a method of the present invention has preferably a chronological age between 30 and 80 years,.

In further aspects, the present invention relates to the use of the above biomarkers for the determination of the biological age of a human being.

In particular, the present invention relates to the use of at least the following biomarkers (i) to (x) for the determination of the biological age of a female human being:

(i) cumulative level of cytosine methylation at gene positions ELOVL_2_CpG_11, ELOVL_2_CpG_12, ELOVL_2_CpG_13, and ELOVL_2_CpG_14 in blood cells;
(ii) cumulative level of cytosine methylation at gene positions ELOVL_2_CpG_15, ELOVL_2_CpG_16, and ELOVL_2_CpG_17 in blood cells;
(iii) cumulative level of cytosine methylation at gene positions FHL_2_CpG_11, and FHL_2_CpG_12 in blood cells;
(iv) cumulative level of cytosine methylation at gene positions FHL_2_CpG_13, FHL_2_CpG_14, and FHL_2_CpG_15 in blood cells;
(v) cumulative level of cytosine methylation at gene positions FHL_2_CpG_16 and FHL_2_CpG_17 in blood cells;
(vi) concentration of dehydroepiandrosterone sulfate in plasma;
(vii) concentration of ferritin in serum;
(viii) decadic logarithm of the ratio between the concentration of NGA2F glycan in serum and the concentration of NA2F glycan in serum;
(ix) concentration of alpha-tocopherol in plasma;
(x) S_p6_n_glycan in serum.

In a preferred embodiment of this aspect, one or more of the following biomarkers (xi) to (xl) are used in addition to the biomarkers (i) to (x):

(xi) cumulative level of cytosine methylation at gene positions ELOVL_2_CpG_9 in blood cells;
(xii) cumulative level of cytosine methylation at gene positions FHL_2_CpG_19 and FHL_2_CpG_20 in blood cells;
(xiii) cumulative level of cytosine methylation at gene positions FHL_2_CpG_9 and FHL_2_CpG_10 in blood cells;
(xiv) cumulative level of cytosine methylation at gene positions ELOVL_2_CpG_18, ELOVL_2_CpG_19, ELOVL_2_CpG_20 and ELOVL_2_CpG_21 in blood cells;
(xv) cumulative level of cytosine methylation at gene positions ELOVL_2_CpG_22, ELOVL_2_CpG_23 and ELOVL_2_CpG_24 in blood cells;
(xvi) cumulative level of cytosine methylation at gene positions ELOVL_2_CpG_10 in blood cells;
(xvii) concentration of s_p2_n_glycan in serum;
(xviii) concentration of s_p1_n_glycan in serum;
(xix) cumulative level of cytosine methylation at gene positions FHL_2_CpG_18 in blood cells;
(xx) concentration of fibrinogen in plasma;

(xxi) cumulative level of cytosine methylation at gene positions ELOVL_2_CpG_2 and ELOVL_2_CpG_3 in blood cells;

(xxii) cumulative level of cytosine methylation at gene positions ELOVL_2_CpG_5 in blood cells;

(xxiii) cumulative level of cytosine methylation at gene positions ELOVL_2_CpG_6 and ELOVL_2_CpG_7 in blood cells;

(xxiv) concentration of urea in plasma;

(xxv) cumulative level of cytosine methylation at gene positions ELOVL_2_CpG_8 in blood cells;

(xxvi) concentration of glucose in serum;

(xxvii) accumulated concentration of threonine and lactate in urine;

(xxviii) concentration of triglycerides in serum;

(xxix) concentration of VLDL2 triglycerides in serum;

(xxx) concentration of uric acid in serum;

(xxxi) concentration of VLDL2 cholesterol in serum;

(xxxii) cumulative level of cytosine methylation at gene positions ELOVL_2_CpG_28 and ELOVL_2_CpG_29 in blood cells;

(xxxiii) concentration of immunoglobulin M (IgM) in serum;

(xxxiv) concentration of uric acid whole blood;

(xxxv) concentration of VLDL cholesterol in serum;

(xxxvi) cumulative level of cytosine methylation at gene positions FHL_2_CpG_5 in blood cells;

(xxxvii) cumulative level of cytosine methylation at gene positions ELOVL_2_CpG_27 in blood cells;

(xxxviii) concentration of 8-isoprstane in urine;

(xxxix) concentration of creatinine in urine;

(xl) cumulative level of cytosine methylation at gene positions ELOVL_2_CpG_34, ELOVL_2_CpG_35 and ELOVL_2_CpG_36 in blood cells.

However, in another preferred embodiment of this aspect, only the above biomarkers (i) to (x) are used.

In another aspect, the present invention relates to the use of at least the following biomarkers (i) to (x) for the determination of the biological age of a male human being:

(i) cumulative level of cytosine methylation at gene positions ELOVL_2_CpG_11, ELOVL_2_CpG_12, ELOVL_2_CpG_13, and ELOVL_2_CpG_14 in blood cells;

(ii) cumulative level of cytosine methylation at gene positions ELOVL_2_CpG_15, ELOVL_2_CpG_16, and ELOVL_2_CpG_17 in blood cells;

(iii) FHL_2_CpG_11, FHL_2_CpG_12

(iv) cumulative level of cytosine methylation at gene positions FHL_2_CpG_13, FHL_2_CpG_14, and FHL_2_CpG_15 in blood cells;

(v) level of cytosine methylation at gene position FHL_2_CpG_16, FHL_2_CpG_17;

(vi) concentration of dehydroepiandrosterone sulfate in plasma;

(vii) concentration of alpha-2-macroglubulin in serum;

(viii) concentration of lycopene in plasma;

(ix) prostate_specific_antigen in serum;

(x) s_p6_n_glycan in serum.

In a preferred embodiment of this aspect, one or more of the following biomarkers (xi) to (xl) are used in addition to the biomarkers (i) to (x):

(xi) cumulative level of cytosine methylation at gene positions ELOVL_2_CpG_9 in blood cells;

(xii) cumulative level of cytosine methylation at gene positions FHL_2_CpG_19, and FHL_2_CpG_20 in blood cells;

(xiii) cumulative level of cytosine methylation at gene positions FHL_2_CpG_9, and FHL_2_CpG_10 in blood cells;

(xiv) cumulative level of cytosine methylation at gene positions ELOVL_2_CpG_10 in blood cells;

(xv) cumulative level of cytosine methylation at gene positions ELOVL_2_CpG_18, ELOVL_2_CpG_19, ELOVL_2_CpG_20 and ELOVL_2_CpG_21 in blood cells;

(xvi) cumulative level of cytosine methylation at gene positions ELOVL_2_CpG_22, ELOVL_2_CpG_23 and ELOVL_2_CpG_24 in blood cells;

(xvii) cumulative level of cytosine methylation at gene positions ELOVL_2_CpG_6 and ELOVL_2_CpG_7 in blood cells;

(xviii) cumulative level of cytosine methylation at gene positions FHL_2_CpG_18 in blood cells;

(xix) cumulative level of cytosine methylation at gene positions ELOVL_2_CpG_8 in blood cells;

(xx) cumulative level of cytosine methylation at gene positions ELOVL_2_CpG_2 and ELOVL_2_CpG_3 in blood cells;

(xxi) cumulative level of cytosine methylation at gene positions ELOVL_2_CpG_5 in blood cells;

(xxii) cumulative level of cytosine methylation at gene positions ELOVL_2_CpG_28 and ELOVL_2_CpG_29 in blood cells;

(xxiii) cumulative level of cytosine methylation at gene positions ELOVL_2_CpG_27 in blood cells;

(xxiv) concentration of Albumin in plasma;

(xxv) concentration of fibrinogen in plasma;

(xxvi) concentration of glucose in serum;

(xxvii) concentration of creatinine in urine;

(xxviii) concentration of s_p2_n_glycan in serum;

(xxix) concentration of 8-isoprstane in urine;

(xxx) Red blood cells (RBC) in whole blood;

(xxxi) cumulative level of cytosine methylation at gene positions FHL_2_CpG_5 in blood cells;

(xxxii) ratio of the concentration of Cu in plasma to the concentration of Zn in plasma;

(xxxiii) cumulative level of cytosine methylation at gene positions ELOVL_2_CpG_34, ELOVL_2_CpG_35 and ELOVL_2_CpG_36 in blood cells;

(xxxiv) cumulative level of cytosine methylation at gene positions ELOVL_2_CpG_33 in blood cells;

(xxxv) concentration of s_p1_n_glycan in serum;

(xxxvi) concentration of urea in plasma;

(xxxvii) concentration of VLDL cholesterol in serum;

(xxxviii) concentration of immunoglobulin M (IgM) in serum;

(xxxix) concentration of VLDL2 cholesterol in serum;

(xl) concentration of uric acid in whole blood.

However, in another preferred embodiment of this aspect, only the above biomarkers (i) to (x) are used.

Finally, in another preferred embodiment, the present invention relates to the use of any number n of the above biomarkers (i) to (xl) for females or of the above biomarkers (i) to (xl) for males for the determination of the biological age of a human being, provided that n is at least 3, at least 4, at least 5, at least 6, at least 7, at least 8, at least 9, or at least 10.

The purpose of the present invention is to provide a valid set of biomarkers of aging in humans that is based on biochemical and molecular analyses of blood and its component or urine, enabling the determination of the biological age of the individual. This can serve as a valuable diagnostic tool for Preventive Medicine by enabling identification of healthy persons whose aging process is accelerated and who are thus likely to be affected by typical age-related diseases at relatively young chronological age. Accordingly, a diagnostic tool is provided for use in all fields wherein an individual's biological age is a valuable information, such as e.g. individual health care, health care systems, insurances and others.

In the following, the biomarkers used in the present invention are described. Table 1 below provides an overview over said biomarkers, assigning a running number (#) to each biomarker, giving the short name for each biomarker as used herein, providing a reference to the numbering in small roman numerals as used in the claims and the above description for female and male biomarkers (#(f) and #(m)), respectively, and providing information as to where in the Figures data concerning the correlation with chronological age of the respective biomarkers is shown.

Further, Table 2 below provides detailed information concerning the correlation of the respective biomarkers with chronological age, in particular the size of the test population (count), and the non-parametric correlation coefficient (rnp).

Table 1: Overview of the biomarkers used in the present invention.

| # | short name | #(f) | #(m) | Fig. |
|---|---|---|---|---|
| 1 | ELOVL_2_CpG_11, 12, 13, 14 | (i) | (i) | 1 |
| 2 | ELOVL_2_CpG_15, 16, 17 | (ii) | (ii) | 2 |
| 3 | FHL_2_CpG_11, 12 | (iii) | (iii) | 3 |
| 4 | FHL_2_CpG_13, 14, 15 | (iv) | (iv) | 4 |
| 5 | FHL_2_CpG_16, 17 | (v) | (v) | 5 |
| 6 | dehydroepiandrosterone sulfate | (vi) | (vi) | 6 |
| 7 | Ferritin | (vii) | - | 7 |

(continued)

| # | short name | #(f) | #(m) | Fig. |
|---|---|---|---|---|
| 8 | S_log_p1_p6 | (viii) | - | 8 |
| 9 | plasma alpha.tocopherole | (ix) | - | 9 |
| 10 | S_p6_n_glycan | (x) | (x) | 10 |
| 11 | alpha-2-macroglubulin | - | (vii) | 11 |
| 12 | lycopene | - | (viii) | 12 |
| 13 | prostate specific antigen | - | (ix) | 13 |
| 14 | ELOVL_2_CpG_9 | (xi) | (xi) | 14 |
| 15 | FHL_2_CpG_19, 20 | (xii) | (xii) | 15 |
| 16 | FHL_2_CpG_9, 10 | (xiii) | (xiii) | 16 |
| 17 | ELOVL_2_CpG_18, 19, 20, 21 | (xiv) | (xv) | 17 |
| 18 | ELOVL_2_CpG_22, 23, 24 | (xv) | (xvi) | 18 |
| 19 | ELOVL_2_CpG_10 | (xvi) | (xiv) | 19 |
| 20 | S_p2_n_glycan | (xvii) | (xxviii) | 20 |
| 21 | S_p1_n_glycan | (xviii) | (xxxv) | 21 |
| 22 | FHL_2_CpG_18 | (xix) | (xviii) | 22 |
| 23 | fibrinogen | (xx) | (xxv) | 23 |
| 24 | ELOVL_2_CpG_2, 3 | (xxi) | (xx) | 24 |
| 25 | ELOVL_2_CpG_5 | (xxii) | (xxi) | 25 |
| 26 | ELOVL_2_CpG_6, 7 | (xxiii) | (xvii) | 26 |
| 27 | urea | (xxiv) | (xxxvi) | 27 |
| 28 | ELOVL_2_CpG_8 | (xxv) | (xix) | 28 |
| 29 | serum glucose | (xxvi) | (xxvi) | 29 |
| 30 | threonine/lactate | (xxvii) | - | 30 |
| 31 | triglyceride | (xxviii) | - | 31 |
| 32 | VLDL2 triglyceride | (xxix) | - | 32 |
| 33 | uric acid | (xxx) | (xl) | 33 |
| 34 | VLDL2 cholesterol | (xxxi) | (xxxix) | 34 |
| 35 | ELOVL_2_CpG_28, 29 | (xxxii) | (xxii) | 35 |
| 36 | Immunglobuline M | (xxxiii) | (xxxviii) | 36 |
| 37 | plasma uric acid | (xxxiv) | - | 37 |
| 38 | VLDL cholesterol | (xxxv) | (xxxvii) | 38 |
| 39 | FHL_2_CpG_5 | (xxxvi) | (xxxi) | 39 |
| 40 | ELOVL_2_CpG_27 | (xxxvii) | (xxiii) | 40 |
| 41 | urinary 8-isoprostane | (xxxviii) | (xxix) | 41 |
| 42 | creatinine urine | (xxxix) | (xxvii) | 42 |
| 43 | ELOVL_2_CpG_34, 35, 36 | (xl) | (xxxiii) | 43 |
| 44 | ELOVL_2_CpG_33 | - | (xxxiv) | 44 |
| 45 | Cu/Zn | - | (xxxii) | 45 |

(continued)

| # | short name | #(f) | #(m) | Fig. |
|---|---|---|---|---|
| 46 | RBC | - | (xxx) | 46 |
| 47 | albumin | - | (xxiv) | 47 |

#: running number
short name: short name of the biomarker
#(f): numbering as used in the claims for female biomarkers
#(m): numbering as used in the claims for male biomarkers
Fig.: biomarker correlation data shown in Figure

Table 2: Correlation data with chronological age

| # | short name | count female | rnp female | count male | rnp male |
|---|---|---|---|---|---|
| 1 | ELOVL_2_CpG_11, 12, 13, 14 | 1063 | 0.778 | 1004 | 0.75 |
| 2 | ELOVL_2_CpG_15, 16, 17 | 1116 | 0.752 | 1051 | 0.722 |
| 3 | FHL_2_CpG_11, 12 | 1118 | 0.441 | 1052 | 0.442 |
| 4 | FHL_2_CpG_13, 14, 15 | 1118 | 0.525 | 1053 | 0.531 |
| 5 | FHL_2_CpG_16, 17 | 1080 | 0.575 | 1012 | 0.522 |
| 6 | dehydroepiandrosterone sulfate | 1136 | 0.479 | 1064 | 0.55 |
| 7 | Ferritin | 1093 | 0.462 | 982 | 0.094 |
| 8 | S_log_p1_p6 | 1126 | 0.604 | 1048 | 0.262 |
| 9 | plasma alpha.tocopherole | 1136 | 0.309 | 1066 | 0.097 |
| 10 | S_p6_n_glycan | 1126 | 0.625 | 1048 | 0.282 |
| 11 | alpha-2-macroglubulin | 1088 | 0.122 | 972 | 0.285 |
| 12 | lycopene | 1126 | 0.229 | 1066 | 0.343 |
| 13 | prostate specific antigen | 1090 | 0.142 | 961 | 0.267 |
| 14 | ELOVL_2_CpG_9 | 1116 | 0.643 | 1053 | 0.638 |
| 15 | FHL_2_CpG_19, 20 | 1118 | 0.577 | 1055 | 0.572 |
| 16 | FHL_2_CpG_9, 10 | 1118 | 0.577 | 1055 | 0.572 |
| 17 | ELOVL_2_CpG_18, 19, 20, 21 | 1097 | 0.569 | 1038 | 0.509 |
| 18 | ELOVL_2_CpG_22, 23, 24 | 1113 | 0.537 | 1052 | 0.48 |
| 19 | ELOVL_2_CpG_10 | 1056 | 0.514 | 998 | 0.533 |
| 20 | S_p2_n_glycan | 1126 | 0.487 | 1048 | 0.226 |
| 21 | S_p1_n_glycan | 1126 | 0.471 | 1048 | 0.165 |
| 22 | FHL_2_CpG_18 | 849 | 0.361 | 424 | 0.344 |
| 23 | fibrinogen | 1136 | 0.358 | 1063 | 0.249 |
| 24 | ELOVL_2_CpG_2, 3 | 1074 | 0.339 | 1009 | 0.302 |
| 25 | ELOVL_2_CpG_5 | 1072 | 0.399 | 1007 | 0.287 |
| 26 | ELOVL_2_CpG_6, 7 | 1116 | 0.399 | 1053 | 0.352 |
| 27 | urea | 1136 | 0.338 | 1064 | 0.149 |
| 28 | ELOVL_2_CpG_8 | 1116 | 0.337 | 1054 | 0.326 |
| 29 | serum glucose | 1094 | 0.311 | 986 | 0.247 |

(continued)

| # | short name | count female | rnp female | count male | rnp male |
|---|---|---|---|---|---|
| 30 | threonine/lactate | 945 | 0.299 | 932 | 0.101 |
| 31 | triglyceride | 1093 | 0.295 | 986 | 0.03 |
| 32 | VLDL2 triglyceride | 1072 | 0.286 | 967 | 0.011 |
| 33 | uric acid | 1136 | 0.284 | 1066 | 0.036 |
| 34 | VLDL2 cholesterol | 1072 | 0.274 | 967 | 0.066 |
| 35 | ELOVL_2_CpG_28, 29 | 1113 | 0.268 | 1052 | 0.282 |
| 36 | Immunglobuline M | 1090 | 0.259 | 976 | 0.077 |
| 37 | plasma uric acid | 1136 | 0.254 | 1064 | 0.011 |
| 38 | VLDL cholesterol | 1072 | 0.249 | 967 | 0.098 |
| 39 | FHL_2_CpG_5 | 1116 | 0.228 | 1052 | 0.2 |
| 40 | ELOVL_2_CpG_27 | 1011 | 0.227 | 959 | 0.257 |
| 41 | urinary 8-isoprostane | 1135 | 0.226 | 1064 | 0.221 |
| 42 | creatinine urine | 945 | 0.217 | 932 | 0.238 |
| 43 | ELOVL_2_CpG_34, 35, 36 | 1115 | 0.202 | 1046 | 0.19 |
| 44 | ELOVL_2_CpG_33 | 1114 | 0.168 | 1050 | 0.186 |
| 45 | Cu/Zn | 1135 | 0.117 | 1065 | 0.199 |
| 46 | RBC | 1174 | 0.009 | 1103 | 0.218 |
| 47 | albumin | 1136 | 0.072 | 1064 | 0.252 |

#: running number
short name: short name of the biomarker
count: number of subjects
rnp: non-parametric coefficient of correlation

The following provides a detailed description of the biomarkers.

1. ELOVL 2 CpG 11, 12, 13, 14

This biomarker is defined as the cumulative level of cytosine methylation at gene positions ELOVL_2_CpG_11, ELOVL_2_CpG_12, ELOVL_2_CpG_13, and ELOVL_2_CpG_14. ELOVL_2 is a gene belonging to the ELOVL (Elongation of very long chain fatty acids) gene family whose gene products control the synthesis of very-long-chain fatty acids. The ELOVL_2 gene has a number of CpG motifs in which the cytosine can be methylated or unmethylated. For this biomarker, the methylation of the CpG motifs ELOVL_2_CpG_11, ELOVL_2_CpG_12, ELOVL_2_CpG_13, and ELOVL_2_CpG_14 is determined in blood cells in a cumulative manner over all measured cells. This marker is given as a percentage between 0 and 1, *i.e.* when e.g. none of the above CpG motifs are methylated, the value is 0, when e.g. 50% of the above CpG motifs are methylated, the value is 0.5, and when e.g. all of the above CpG motifs are methylated, the value is 1. Methods for the determination of the level of cytosine methylation at certain gene positions are not particularly limited and are known in the art.

2. ELOVL 2 CpG 15, 16, 17

In an analogous manner to the above biomarker ELVOL_2_CpG_11, 12, 13, 14, this biomarker is defined as the cumulative level of cytosine methylation at gene positions ELOVL_2_CpG_15, ELOVL_2_CpG_16, and ELOVL_2_CpG_17.

### 3. FHL 2 CpG 11, 12

This biomarker is defined as the cumulative level of cytosine methylation at gene positions FHL_2_CpG_11 and FHL_2_CpG_12. FHL_2 is the gene encoding the "Four and a half LIM domains protein 2" (FHL2) which contains highly conserved double zinc finger motifs called the LIM domain. FHL2 has been shown to interact with a number of intracellular protein factors. Cumulative cytosine methylation is determined in an analogous manner as described for the above biomarker ELOVL_2_CpG_11, 12, 13, 14.

### 4. FHL 2 CpG 13, 14, 15

In an analogous manner to the above biomarker FHL_2_CpG_11, 12, this biomarker is defined as the cumulative level of cytosine methylation at gene positions FHL_2_CpG_13, FHL_2_CpG_14, and FHL_2_CpG_15.

### 5. FHL 2 CpG 16, 17

In an analogous manner to the above biomarker FHL_2_CpG_11, 12, this biomarker is defined as the cumulative level of cytosine methylation at gene positions FHL_2_CpG_16, and FHL_2_CpG_17.

### 6. Dehydroepiandrosterone sulfate

This biomarker is defined as the concentration of dehydroepiandrosterone sulfate in plasma. Dehydroepiandrosterone is an important endogenous steroid hormone and the most abundant steroid in humans. It is produced in the adrenal glands, the gonads and the brain and functions as a metabolic intermediate in the biosynthesis of the androgen and estrogen sex steroids. Dehydroepiandrosterone sulfate is produced exclusively in the adrenal glands via the sulfotransferases SULT1A1 and SULT1E1. Preferably, the value of this biomarker is given in $\mu$g per dL. Methods for the determination of the concentration of dehydroepiandrosterone sulfate in plasma are not particularly limited and are known in the art.

### 7. Ferritin

This biomarker is defined as the concentration of ferritin in serum. Ferritin is an ubiquitous intracellular protein that stores and releases iron in a controlled fashion. Preferably, the value of this biomarker is given in ng per mL. Methods for the determination of the concentration of ferritin in serum are not particularly limited and are known in the art.

### 8. S log p1 p6

This biomarker is defined as the decadic logarithm of the ratio between the concentration of NGA2F glycan in serum and the concentration of NA2F glycan in serum. NGA2F glycan is an agalactosylated core-$\alpha$-1,6-fucosylated biantennary oligosaccharide, whereas NA2F glycan is a bigalactosylated core-$\alpha$-1,6-fucosylated biantennary oligosaccharide. Methods for the determination of the concentration of NGA2F glycan in serum and the concentration of NA2F glycan in serum are not particularly limited and are known in the art.

### 9. Plasma alpha.tocopherole

This biomarker is defined as the concentration of alpha-tocopherole in plasma. Alpha-tocopherole is a form of vitamin E. Preferably, the value of this biomarker is given in $\mu$mole per L. Methods for the determination of the concentration of alpha-tocopherole in plasma are not particularly limited and are known in the art.

### 10. S log p6 n glycan

Methods for the determination of the concentration of glycans in serum are not particularly limited and are known in the art.

### 11. Alpha2 macroglobulin

This biomarker is defined as the concentration of alpha-2-macroglobulin in serum. Alpha-2-macroglobulin is the largest major non-immunoglobulin protein in plasma. It acts as an antiprotease and inhibitor of both fibrinolysis and coagulation. Preferably, the value of this biomarker is given in mg per dL. Methods for the determination of the concentration of alpha-2-macroglobulin in serum are not particularly limited and are known in the art.

### 12. Plasma lycopene

This biomarker is defined as the concentration of lycopene in plasma. Lycopene is a carotene and carotinoid pigment and a phytochemical found in tomatoes. It is not an essential nutrient for humans, has antioxidant activity and is transported in the blood by various lipoproteins. Lycopene accumulates in the liver, adrenal glands and testes. It is a potential agent for the prevention of some types of cancer. Preferably, the value of this biomarker is given in $\mu$mole per L. Methods for the determination of the concentration of lycopene in plasma are not particularly limited and are known in the art.

### 13. Prostate specific antigen

This biomarker is defined as the concentration of prostate specific antigen (PSA) in serum. PSA a member of the kallikrein-related peptidase family and is secreted by the epithelial cells of the prostate gland. PSA is produced for the ejaculate, where it liquefies semen in the seminal coagulum and allows sperm to swim freely. It is also believed to be instrumental in dissolving cervical mucus, allowing the entry of sperm into the uterus. PSA is present in small quantities in the serum of men with healthy prostates, but is often elevated in the presence of prostate cancer or other prostate disorders. Preferably, the value of this biomarker is given in ng per mL. Methods for the determination of the concentration of prostate specific antigen (PSA) in serum are not particularly limited and are known in the art.

### 14. ELOVL 2 CpG 9

In an analogous manner to the above biomarker, this biomarker is defined as the cumulative level of cytosine methylation at gene positions ELOVL_2_CpG_9.

### 15. FHL 2 CpG 19 and FHL 2 CpG 20

In an analogous manner to the above biomarker, this biomarker is defined as the cumulative level of cytosine methylation at gene positions FHL_2_CpG_19 and FHL 2 CpG 20

### 16. FHL 2 CpG 9 and FHL 2 CpG 10

In an analogous manner to the above biomarker, this biomarker is defined as the cumulative level of cytosine methylation at gene positions FHL_2_CpG_9 and FHL 2 CpG 10

### 17. ELOVL 2 CpG 18, 19, 20, 21

In an analogous manner to the above biomarker ELVOL_2_CpG_18, 19, 20, 21, this biomarker is defined as the cumulative level of cytosine methylation at gene positions ELOVL_2_CpG_18, ELOVL_2_CpG_19, ELOVL_2_CpG_20 and ELOVL_2_CpG_21.

### 18. ELOVL 2 CpG 22, 23, 24

In an analogous manner to the above biomarker ELVOL_2_CpG_11, 12, 13, 14, this biomarker is defined as the cumulative level of cytosine methylation at gene positions ELOVL_2_CpG_22, ELOVL_2_CpG_23, and ELOVL_2_CpG_24.

### 19. ELOVL 2 CpG 10

In an analogous manner to the above biomarker, this biomarker is defined as the cumulative level of cytosine methylation at gene positions ELOVL_2_CpG_10.

### 20. S p2 n glycan

This biomarker is defined as the concentration of NGA2FB glycan in serum. NGA2FB glycan is an agalactos-ylated core-$\alpha$-1,6-fucosylated bisecting biantennary oligosaccharide. Preferably, the value of this biomarker is given as a percentage of all glycan signals. Methods for the determination of the concentration of NGA2FB glycan in serum are not particularly limited and are known in the art.

### 21. S log p1 n glycan

Methods for the determination of the concentration of glycans in serum are not particularly limited and are known in the art.

### 22. FHL 2 CpG 18

In an analogous manner to the above biomarker FHL_2_CpG_11, 12, this biomarker is defined as the cumulative level of cytosine methylation at gene position FHL_2_CpG_18.

### 23. Fibrinogen

This biomarker is defined as the concentration of fibrinogen in plasma. Fibrinogen is a soluble plasma glycoprotein that is synthesized in the liver and is converted by thrombin to fibrin during blood coagulation. Fibrin plays a key role in the inflammatory response and development of rheumatoid arthritis. Preferably, the value of this biomarker is given in mg per mL. Methods for the determination of the concentration of fibrinogen in plasma are not particularly limited and are known in the art.

### 24. ELOVL 2 CpG 2, 3

In an analogous manner to the above biomarker this biomarker is defined as the cumulative level of cytosine methylation at gene positions ELOVL_2_CpG_2 and ELOVL_2_CpG_3.

### 25. ELOVL 2 CpG 5

In an analogous manner to the above biomarker this biomarker is defined as the cumulative level of cytosine methylation at gene positions ELOVL_2_CpG_5.

### 26. ELOVL 2 CpG 6, 7

In an analogous manner to the above biomarker this biomarker is defined as the cumulative level of cytosine methylation at gene positions ELOVL_2_CpG_6 and ELOVL_2_CpG_7.

### 27. Urea

This biomarker is defined as the concentration of urea in plasma. Preferably, the value of this biomarker is given in mmole per L. Methods for the determination of the concentration of urea in plasma are not particularly limited and are known in the art.

### 28. ELOVL 2 CpG 8

In an analogous manner to the above biomarker this biomarker is defined as the cumulative level of cytosine methylation at gene positions ELOVL_2_CpG_8.

### 29. Serum glucose

This biomarker is defined as the concentration of glucose in serum. Preferably, the value of this biomarker is given in mmole per L. Methods for the determination of the concentration of glucose in serum are not particularly limited and are known in the art.

### 30. Threonine/Lactate

This biomarker is defined as the accumulated, *i.e.* added, concentration of threonine and lactate in urine. Methods for the determination of the concentrations of threonine and lactate in urine are not particularly limited and are known in the art.

### 31. Triglycerides

This biomarker is defined as the concentration of triglycerides in serum. Preferably, the value of this biomarker is given in mmole per L. Methods for the determination of the concentration of triglycerides in serum are not particularly limited and are known in the art.

### 32. VDL2 triglyceride

VLDL stands for "very low density lipoprotein", which is one of the five major classes of lipoproteins (chylomicrons, VLDL, low-density lipoprotein, intermediate-density lipoprotein, high-density lipoprotein) that enable transport of fats and cholesterol within the water-based blood plasma. VLDL is assembled in the liver from triglycerides, cholesterol, and apolipoproteins. VLDL is of medical relevance as it is thought to be an important risk factor for atherosclerosis. VLDL particles have a diameter of 30-80 nm and they can further be separated into different subclasses. VLDL2 is one of the subclasses of VLDL particles. "VLDL2 triglyceride" indicates the amounts of triglycerides transported by this subclass. Methods for the determination of VLDL2 triglyceride in plasma are not particularly limited and are known in the art.

### 33. Uric acid

Uric acid is a heterocyclic compound of carbon, nitrogen, oxygen, and hydrogen with the formula C5H4N4O3. It forms ions and salts known as urates and acid urates such as ammonium acid urate. Uric acid is a product of the metabolic breakdown of purine nucleotides. Methods for the determination of the concentration of albumin in plasma are not particularly limited and are known in the art.

### 34. VLDL2 cholesterol

VLDL stands for "very low density lipoprotein", which is one of the five major classes of lipoproteins (chylomicrons, VLDL, low-density lipoprotein, intermediate-density lipoprotein, high-density lipoprotein) that enable transport of fats and cholesterol within the water-based blood plasma. VLDL is assembled in the liver from triglycerides, cholesterol, and apolipoproteins. VLDL is of medical relevance as it is thought to be an important risk factor for atherosclerosis. VLDL particles have a diameter of 30-80 nm and they can further be separated into different subclasses. VLDL2 is one of the subclasses of VLDL particles. "VLDL2 cholesterol" indicates the amounts cholesterol transported by this subclass.
Methods for the determination of VLDL2 cholesterol in plasma are not particularly limited and are known in the art.

### 35. ELOVL 2 CpG 28, 29

In an analogous manner to the above biomarker this biomarker is defined as the cumulative level of cytosine methylation at gene positions ELOVL_2_CpG_28 and ELOVL_2_CpG_29.

### 36. Immunoglobulin M

This biomarker is defined as the concentration of immunoglobulin M (IgM) in serum. Preferably, the value of this biomarker is given in g per L. Methods for the determination of the concentration of immunoglobulin M (IgM) in serum are not particularly limited and are known in the art.

### 37. Plasma uric acid

Uric acid is a heterocyclic compound of carbon, nitrogen, oxygen, and hydrogen with the formula $C_5H_4N_4O_3$. It forms ions and salts known as urates and acid urates such as ammonium acid urate. Uric acid is a product of the metabolic breakdown of purine nucleotides. Methods for the determination of the concentration of albumin in plasma are not particularly limited and are known in the art.

### 38. VLDL cholesterol

VLDL stands for "very low density lipoprotein", which is one of the five major classes of lipoproteins (chylomicrons, VLDL, low-density lipoprotein, intermediate-density lipoprotein, high-density lipoprotein) that enable transport of fats and cholesterol within the water-based blood plasma. VLDL is assembled in the liver from triglycerides, cholesterol, and apolipoproteins. VLDL is of medical relevance as it is thought to be an important risk factor for atherosclerosis. VLDL particles have a diameter of 30-80 nm and they can further be separated into different subclasses. VLDL is one of the subclasses of VLDL particles. "VLDL cholesterol" indicates the amounts of cholesterol transported by this subclass. Methods for the determination of VLDL cholesterol in plasma are not particularly limited and are known in the art.

### 39. FHL 2 CpG 5

In an analogous manner to the above biomarker, this biomarker is defined as the cumulative level of cytosine methylation at gene position FHL_2_CpG_5.

### 40. ELOVL 2 CpG 27

In an analogous manner to the above biomarker this biomarker is defined as the cumulative level of cytosine methylation at gene positions ELOVL_2_CpG_27.

### 41. 8-isoprostane

The isoprostanes are prostaglandin-like compounds formed in vivo from the free radical-catalyzed peroxidation of essential fatty acids (primarily arachidonic acid) without the direct action of cyclooxygenase (COX) enzymes. Methods for the determination of the concentration of albumin in plasma are not particularly limited and are known in the art.

### 42. Creatinine.urine

This biomarker is defined as the concentration of creatinine in urine. Methods for the determination of the concentration of creatinine in urine are not particularly limited and are known in the art.

### 43. ELOVL 2 CpG 34, 35, 36

In an analogous manner to the above biomarker this biomarker is defined as the cumulative level of cytosine methylation at gene positions ELOVL_2_CpG_34, ELOVL_2_CpG_35 and ELOVL_2_CpG_36.

### 44. ELOVL 2 CpG 33

In an analogous manner to the above biomarker this biomarker is defined as the cumulative level of cytosine methylation at gene positions ELOVL_2_CpG_33.

### 45. Plasma Cu/Zn

This biomarker is defined as the ratio of the concentration of Cu in plasma to the concentration of Zn in plasma. Methods for the determination of the concentration of Cu in plasma and the concentration of Zn in plasma are not particularly limited and are known in the art.

### 46. Red blood cells

Red blood cells, or erythrocytes, are the most common type of blood cell and the vertebrate organism's principal means of delivering oxygen ($O_2$) to the body tissues via the blood flow through the circulatory system. Methods for the determination of the concentration of albumin in plasma are not particularly limited and are known in the art.

### 47. Albumin

This biomarker is defined as the concentration of albumin in plasma. Albumin is the main protein of human plasma. It binds water, cations, fatty acids, hormones, bilirubin, thyroxine (T4) and drugs (including barbiturates). Its main function is to regulate the colloidal osmotic pressure of blood. Preferably, the values of this biomarker is given in g per L. Methods for the determination of the concentration of albumin in plasma are not particularly limited and are known in the art.

The present invention provides methods for the determination of the biological age for women or men, respectively, which is based on a set of at least ten different molecular and clinical chemistry biomarkers that have been assessed in blood, blood components or urine. Selection of these parameters and their weighting was done in such as to way as to maximize the correlation coefficient between the biological age and the chronological age in the population.

The methods of the present invention advantageously reflect biological mechanisms known to play important roles in the aging of cells or tissues, including epigenetic changes, changes in N-glycan profiles, hormonal changes and changes in metabolism. Said methods are based on a set of at least ten different biomarkers, which obviously reflects the aging process better than any single marker on its own. Further, the biomarkers used in the present invention are solely based on analyses on blood or urine and therefore do not require physical examination of the subjects nor their cooperation nor motivation to give maximum performance in physiological assessment (e.g. lung function). This also reduces labor costs.

The figures show:

Figures 1 to 47:
These figures show the correlation of the respective biomarkers given in the top line of each figure with chronological age. Data for males is shown in the left panel, data for females in the right panel. In the graphs shown in the lower row, the 1% quantile is hidden, *i.e.* the highest and lowest 1% of datapoints with respect to the y-axis are excluded. The x-axis shows chronological age in years. The y-axis shows the respective biomarker values. The number of test subjects, the function of the linear regression, and the parametric (rp) and non-parametric (rnp) correlation coefficients are shown.
Figure 48:
Correlation between chronological age and biological age as determined according to the present invention using the female and male biomarkers (i) to (x), respectively, showing results from RASIG population (circles). count: number of test subjects; cor: correlation coefficient.

The present invention will be further illustrated in the following examples without being limited thereto.

**Examples**

Example 1:

Determination of the biological age of females

For ten exemplary female test subjects, the above biomarkers (i) to (x) (*i.e.* the biomarkers ELOVL_2_CpG_11, 12, 13, 14; ELOVL_2_CpG_15, 16, 17; FHL_2_CpG_11, 12; FHL_2_CpG_13, 14, 15; FHL_2_CpG_16, 17; dehydroepiandrosterone sulfate; ferritin; S_log_p1_p6; plasma alpha.tocopherole; and S_p6_n_glycan) were determined. Based on the respective data, the biological age of the test subjects was determined according to the method of the present invention. Table 3 below shows test subject ID, and the chronological age (A) and biological age (B) of the test subject.

Table 3

| ID | A | B |
|----|-------|--------|
| 95 | 61.105 | 58.673 |
| 97 | 67.032 | 81.144 |
| 77 | 67.687 | 74.381 |
| 104 | 48.461 | 45.433 |
| 105 | 70.408 | 60.339 |
| 350 | 38.75 | 34.214 |
| 109 | 67.618 | 60.97 |
| 107 | 48.067 | 45.566 |
| 108 | 48.675 | 42.889 |
| 106 | 65.277 | 79.036 |

Example 2:

Determination of the biological age of males

For ten exemplary male test subjects, the above biomarkers (i) to (x) (*i.e.* the biomarkers ELOVL_2_CpG_11, 12, 13, 14; ELOVL_2_CpG_15, 16, 17; FHL_2_CpG_11, 12; FHL_2_CpG_13, 14, 15; FHL_2_CpG_16, 17; dehydroepiandrosterone sulfate; alpha2 macroglobulin; plasma lycopene; prostate specific antigen; and S_p6_n_glycan) were determined. Based on the respective data, the biological age of the test subjects was determined according to the method of the present invention. Table 4 below shows test subject ID, and the chronological age (A) and biological age (B) of the test subject.

Table 4

| ID | A | B |
|---|---|---|
| 364 | 37.181 | 37.842 |
| 373 | 37.737 | 44.747 |
| 2225 | 46.391 | 36.699 |
| 2070 | 46.446 | 45.871 |
| 1462 | 46.372 | 60.232 |
| 1370 | 58.671 | 50.486 |
| 600 | 58.506 | 59.573 |
| 1294 | 58.191 | 64.418 |
| 590 | 74.928 | 75.546 |
| 2942 | 74.608 | 67.489 |

Example 3:

Detailed determination of the biological age

For a female individual, the above biomarkers (i) to (x) (*i.e.* the biomarkers ELOVL_2_CpG_11, 12, 13, 14; ELOVL_2_CpG_15, 16, 17; FHL_2_CpG_11, 12; FHL_2_CpG_13, 14, 15; FHL_2_CpG_16, 17; dehydroepiandrosterone sulfate; ferritin; S_log_p1_p6; plasma alpha.tocopherole; and S_p6_n_glycan) were determined, wherein the respective values are given in Table 5 below, together with the corresponding scaled values.

Table 5

| # | Biomarker | Value | Scaled |
|---|---|---|---|
| i | ELOVL_2_CpG_11, 12, 13, 14 | 0.63 | 0.03754 |
| ii | ELOVL_2_CpG_15, 16, 17 | 0.56 | -0.09148 |
| iii | FHL_2_CpG_11, 12 | 0.16 | -0.9547 |
| iv | FHL_2_CpG_13, 14, 15 | 0.29 | -0.7823 |
| v | FHL_2_CpG_16, 17 | 0.43 | -0.7993 |
| vi | dehydroepiandrosterone sulfate [$\mu$g/dL] | 1.08 | -0.9692 |
| vii | ferritin [ng/mL] | 146.1 | 1.7429 |
| viii | S_log_p1_p6 | -0.44479 | -0.4511 |
| ix | plasma alpha.tocopherole [$\mu$mol/L] | 35.33 | 0.7589 |
| x | S_p6_n_glycan | 19.1703 | -0.05289 |

According to the formula of the present invention (cf. pages 13 and 14, *supra*), the biological age of the individual was calculated to be 58.67 years. The chronological age of the individual at that time was 61.11 years.

**Claims**

1. A method for the determination of the biological age of a human being, comprising the steps of:

(a) providing biological samples, selected from the group consisting of whole blood, serum, plasma, and urine, from the human being, wherein the provision of the biological samples does not include any interaction with the human body,

(b1) in case the human being is female, determining at least the following biomarkers (i) to (x) in the respective biological samples, wherein the necessary biological samples are provided in step (a):

(i) cumulative level of cytosine methylation at gene positions ELOVL_2_CpG_11, ELOVL_2_CpG_12, ELOVL_2_CpG_13, and ELOVL_2_CpG_14 in blood cells;
(ii) cumulative level of cytosine methylation at gene positions ELOVL_2_CpG_15, ELOVL_2_CpG_16, and ELOVL_2_CpG_17 in blood cells;
(iii) cumulative level of cytosine methylation at gene positions FHL_2_CpG_11, and FHL_2_CpG_12 in blood cells;
(iv) cumulative level of cytosine methylation at gene positions FHL_2_CpG_13, FHL_2_CpG_14, and FHL_2_CpG_15 in blood cells;
(v) cumulative level of cytosine methylation at gene positions FHL_2_CpG_16 and FHL_2_CpG_17 in blood cells;
(vi) concentration of dehydroepiandrosterone sulfate in plasma;
(vii) concentration of ferritin in serum;
(viii) decadic logarithm of the ratio between the concentration of NGA2F glycan in serum and the concentration of NA2F glycan in serum;
(ix) concentration of alpha-tocopherol in plasma;
(x) concentration of S_p6_n_glycan in serum; or

(b2) in case the human being is male, determining at least the following biomarkers (i) to (x) in the respective biological samples, wherein the necessary biological samples are provided in step (a):

(i) cumulative level of cytosine methylation at gene positions ELOVL_2_CpG_11, ELOVL_2_CpG_12, ELOVL_2_CpG_13, and ELOVL_2_CpG_14 in blood cells;
(ii) cumulative level of cytosine methylation at gene positions ELOVL_2_CpG_15, ELOVL_2_CpG_16, and ELOVL_2_CpG_17 in blood cells;
(iii) cumulative level of cytosine methylation at gene positions FHL_2_CpG_13, FHL_2_CpG_14, and FHL_2_CpG_15 in blood cells;
(iv) cumulative level of cytosine methylation at gene position FHL_2_CpG_18;
(v) concentration of creatinine in urine;
(vi) ratio of the number of $CD3^+CD4^+$ cells in whole blood to the number of $CD8^+$ cells in whole blood;
(vii) concentration of dehydroepiandrosterone sulfate in plasma;
(viii) concentration of alpha-2-macroglubulin in serum;
(ix) concentration of lycopene in plasma;
(x) concentration of S_p6_n_glycan in serum; and

(c) determining the biological age of the human being based on the biomarkers determined in step (b1) or (b2) by

(c1) establishing a multiple linear regression model for the determined biomarkers based on the data provided in the study underlying the present invention;
(c2) determining from the multiple linear regression model established in step (c1)

(i) the correlation coefficient $r^2$,
(ii) the weighting factors $b_i$ for each determined biomarker, wherein i is 1 through n, and n is the number of biomarkers determined, and
(iii) the intercept c;

(c3) calculating an apparent biological age B according to formula (1)

$$B = \sum_i^n b_i x_i + c \qquad (1)$$

wherein

$b_i$ are the weighting factors determined in step (c2),
$x_i$ are the values of the respective biomarkers as determined in step (b1) or (b2),

wherein i is 1 to n and

c is the intercept determined in step (c2); and

(c4) calculating the corrected biological age (Bioage) according to formula (2)

$$Bioage = \frac{B - mean(a)}{r^2} + mean(a) \qquad (2)$$

wherein

B is the apparent biological age determined in step (c3), and
mean(a) is the mean chronological age of the statistical population used in the study underlying the present invention.

2. The method according to claim 1, wherein the human being is female, in step (b1) one or more of the following biomarkers (xi) to (xxviii) are determined in addition to the biomarkers (i) to (x), and the necessary biological samples are provided in step (a):

(xi) cumulative level of cytosine methylation at gene positions ELOVL_2_CpG_22, ELOVL_2_CpG_23, and ELOVL_2_CpG_24 in blood cells;
(xii) cumulative level of cytosine methylation at gene positions FHL_2_CpG_19, and FHL_2_CpG_20 in blood cells;
(xiii) concentration of NGA2FB glycan in serum;
(xiv) concentration of fibrinogen in plasma;
(xv) concentration of urea in plasma;
(xvi) concentration of glucose in serum;
(xvii) length of telomers in blood cells;
(xviii) concentration of triglycerides in serum;
(xix) concentration of immunoglobulin M (IgM) in serum;
(xx) concentration of adiponectin in serum;
(xxi) accumulated concentration of threonine and lactate in urine;
(xxii) concentration of homocysteine in plasma;
(xxiii) concentration of lycopene in plasma;
(xxiv) concentration of cholesterol in serum;
(xxv) concentration of glycosylated hemoglobin ($HbA_{1c}$) in whole blood;
(xxvi) concentration of glycine in urine;
(xxvii) number of T cell receptor excision circles (Trec) in 100,000 T cells from whole blood;
(xxviii) concentration of creatinine in urine.

3. The method according to claim 1, wherein the human being is male, in step (b2) one or more of the following biomarkers (xi) to (xxiii) are determined in addition to the biomarkers (i) to (x), and the necessary biological samples are provided in step (a):

(xi) cumulative level of cytosine methylation at gene positions FHL_2_CpG_19, and FHL_2_CpG_20 in blood cells;
(xii) number of T cell receptor excision circles (Trec) in 100,000 T cells from whole blood;
(xiii) decadic logarithm of the ratio between the concentration of NGA2F glycan in serum and the concentration of NA2F glycan in serum;

(xiv) concentration of prostate specific antigen (PSA) in serum;

(xv) concentration of fibrinogen in plasma;

(xvi) concentration of albumin in plasma;

(xvii) concentration of glucose in serum;

(xviii) concentration of NGA2FB glycan in serum;

(xix) ratio of the concentration of Cu in plasma to the concentration of Zn in plasma;

(xx) ratio of the number of $CD16^+CD56^+$ cells in whole blood to the number of $CD45^+$ cells in whole blood;

(xxi) concentration of IgG antibodies directed against *Clostridium tetani* in plasma;

(xxii) ratio of the number of $CD3^+CD8^+$ cells in whole blood to the number of $CD45^+$ cells in whole blood;

(xxiii) concentration of beta-hydroxyisovalerate in urine.

**4.** The method according to claim 1, wherein the human being is female, the biomarkers (i) to (x) are determined in step (b1), and determining the biological age of the human being based on said biomarkers in step (c) comprises the steps of:

(c1) calculating an apparent biological age B according to formula (3)

$$B = \sum_{i=1}^{10} b_i x_i + c \qquad (3)$$

wherein

$b_1$ is a number between 40.37 and 49.33,
$x_1$ is the determined value of biomarker (i) expressed as a percentage between 0 and 1,
$b_2$ is a number between 42.86 and 52.37,
$x_2$ is the determined value of biomarker (ii) expressed as a percentage between 0 and 1,
$b_3$ is a number between -28.00 and -22.92,
$x_3$ is the determined value of biomarker (iii) expressed as a percentage between 0 and 1,
$b_4$ is a number between 26.52 and 32.40,
$x_4$ is the determined value of biomarker (iv) expressed as a percentage between 0 and 1,
$b_5$ is a number between 11.88 and 14.50,
$x_5$ is the determined value of biomarker (v) expressed as a percentage between 0 and 1,
$b_6$ is a number between -0.97 and -0.80,
$x_6$ is the determined value of biomarker (vi) expressed in $\mu$g per dL,
$b_7$ is a number between 0.023 and 0.027,
$x_7$ is the determined value of biomarker (vii) expressed in ng per mL,
$b_8$ is a number between 9.78 and 11.94
$x_8$ is the determined value of biomarker (viii),
$b_9$ is a number between 0.09 and 0.11
$x_9$ is the determined value of biomarker (ix) expressed in $\mu$mole per L,
$b_{10}$ is a number between 0.028 and 0.034,
$x_{10}$ is the determined value of biomarker (x) expressed in $\mu$mole per L, and
c is a number between -14.47 and -11.85; and

(c2) calculating the corrected biological age (Bioage) according to formula (2)

$$Bioage = \frac{B - mean(a)}{r^2} + mean(a) \qquad (2)$$

wherein

B is the apparent biological age determined in step (c1), mean(a) is a number between 50.27 and 61.43, and $r^2$ is a number between 0.79 and 0.95.

**5.** The method according to claim 4, wherein

| $b_1$ | is 44.85, |
|---|---|
| $b_2$ | is 47.616, |
| $b_3$ | is -25.457, |
| $b_4$ | is 29.463, |
| $b_5$ | is 13.19, |
| $b_6$ | is -0.889, |
| $b_7$ | is 0.025, |
| $b_8$ | is 10.863, |
| $b_9$ | is 0.1, |
| $b_{10}$ | is 0.031, |
| c | is -13.16 |
| mean(a) | is 55.85, and |
| $r^2$ | is 0.87. |

6. The method according to claim 1, wherein the human being is male, the biomarkers (i) to (x) are determined in step (b2), and determining the biological age of the human being based on said biomarkers in step (c) comprises the steps of:

(c1) calculating an apparent biological age B according to formula (3)

$$B = \sum_{i=1}^{10} b_i x_i + c \qquad (3)$$

wherein

$b_1$ is a number between 32.84 and 40.12,
$x_1$ is the determined value of biomarker (i) expressed as a percentage between 0 and 1,
$b_2$ is a number between 33.53 and 40.97,
$x_2$ is the determined value of biomarker (ii) expressed as a percentage between 0 and 1,
$b_3$ is a number between 42.52 and 51.95,
$x_3$ is the determined value of biomarker (iii) expressed as a percentage between 0 and 1,
$b_4$ is a number between 4.06 and 4.95,
$x_4$ is the determined value of biomarker (iv) expressed as a percentage between 0 and 1,
$b_5$ is a number between -0.18 and -0.16,
$x_5$ is the determined value of biomarker (v),
$b_6$ is a number between 1.39 and 1.69,
$x_6$ is the determined value of biomarker (vi),
$b_7$ is a number between -1.14 and -0.94,
$x_7$ is the determined value of biomarker (vii) expressed in g per dL,
$b_8$ is a number between 0.022 and 0.026,
$x_8$ is the determined value of biomarker (viii) expressed in mg per dL,
$b_9$ is a number between -3.44 and -2.82,
$x_9$ is the determined value of biomarker (ix) expressed in $\mu$mole per L,
$b_{10}$ is a number between 0.027 and 0.031,
$x_{10}$ is the determined value of biomarker (x) expressed in $\mu$mole per L, and
c is a number between -4.24 and -3.47; and

(c2) calculating the corrected biological age (Bioage) according to the formula (2)

$$Bioage = \frac{B - mean(a)}{r^2} + mean(a) \qquad (2)$$

wherein

B is the apparent biological age determined in step (c1), mean(a) is a number between 50.27 and 61.43, and $r^2$ is a number between 0.78 and 0.94.

7. The method according to claim 6, wherein

| | |
|---|---|
| $b_1$ | is 36.481, |
| $b_2$ | is 37.25, |
| $b_3$ | is 47.234, |
| $b_4$ | is 4.506, |
| $b_5$ | is -0.172, |
| $b_6$ | is 1.538, |
| $b_7$ | is -1.044, |
| $b_8$ | is 0.024, |
| $b_9$ | is -3.131, |
| $b_{10}$ | is 0.029, |
| c | is -3.858 |
| mean(a) | is 55.85, and |
| $r^2$ | is 0.86. |

8. The method according to any one of claims 1 to 7, wherein the human being has a chronological age of between 30 and 80 years.

9. The use of at least the following biomarkers (i) to (x) for the determination of the biological age of a female human being according to the method of claims 1,2,4,5 and 8:

(i) cumulative level of cytosine methylation at gene positions ELOVL_2_CpG_11, ELOVL_2_CpG_12, ELOVL_2_CpG_13, and ELOVL_2_CpG_14 in blood cells;
(ii) cumulative ievei of cytosine methylation at gene positions ELOVL_2_CpG_15, ELOVL_2_CpG_16, and ELOVL_2_CpG_17 in blood cells;
(iii) cumulative level of cytosine methylation at gene positions FHL_2_CpG_11, and FHL_2_CpG_12 in blood cells;
(iv) cumulative level of cytosine methylation at gene positions FHL_2_CpG_13, FHL_2_CpG_14, and FHL_2_CpG_15 in blood cells;
(v) cumulative level of cytosine methylation at gene positions FHL_2_CpG_16 and FHL_2_CpG_17 in blood cells;
(vi) concentration of dehydroepiandrosterone sulfate in plasma;
(vii) concentration of ferritin in serum;
(viii) decadic logarithm of the ratio between the concentration of NGA2F glycan in serum and the concentration of NA2F glycan in serum;
(ix) concentration of alpha-tocopherol in plasma;
(x) concentration of cysteine in plasma.

10. The use according to claim 9, wherein one or more of the following biomarkers (xi) to (xxviii) are used in addition to the biomarkers (i) to (x):

(xi) cumulative level of cytosine methylation at gene positions ELOVL_2_CpG_22, ELOVL_2_CpG_23, and ELOVL_2_CpG_24 in blood cells;
(xii) cumulative level of cytosine methylation at gene positions FHL_2_CpG_19, and FHL_2_CpG_20 in blood cells;
(xiii) concentration of NGA2FB glycan in serum;
(xiv) concentration of fibrinogen in plasma;
(xv) concentration of urea in plasma;
(xvi) concentration of glucose in serum;

(xvii) length of telomers in blood cells;
(xviii) concentration of triglycerides in serum;
(xix) concentration of immunoglobulin M (IgM) in serum;
(xx) concentration of adiponectin in serum;
(xxi) accumulated concentration of threonine and lactate in urine;
(xxii) concentration of homocysteine in plasma;
(xxiii) concentration of lycopene in plasma;
(xxiv) concentration of cholesterol in serum;
(xxv) concentration of glycosylated hemogiobin ($HbA_{1c}$) in whole blood;
(xxvi) concentration of glycine in urine;
(xxvii) number of T cell receptor excision circles (Trec) in 100,000 T cells from whole blood;
(xxviii) concentration of creatinine in urine.

11. The use of at least the following biomarkers (i) to (x) for the determination of the biological age of a male human being according to method of claims 1,3,6-8:

(i) cumulative level of cytosine methylation at gene positions ELOVL_2_CpG_11, ELOVL_2_CpG_12, ELOVL_2_CpG_13, and ELOVL_2_CpG_14 in blood cells;
(ii) cumulative level of cytosine methylation at gene positions ELOVL_2_CpG_15, ELOVL_2_CpG_16, and ELOVL_2_CpG_17 in blood cells;
(iii) cumulative level of cytosine methylation at gene positions FHL_2_CpG_13, FHL_2_CpG_14, and FHL_2_CpG_15 in blood cells;
(iv) cumulative level of cytosine methylation at gene position FHL_2_CpG_18;
(v) concentration of creatinine in urine;
(vi) ratio of the number of CD3$^+$CD4$^+$ cells in whole blood to the number of CD8$^+$ cells in whole blood;
(vii) concentration of dehydroepiandrosterone sulfate in plasma;
(viii) concentration of alpha-2-macroglubulin in serum;
(ix) concentration of lycopene in plasma;
(x) concentration of cysteine in plasma.

12. The use according to claim 11, wherein one or more of the following biomarkers (xi) to (xxiii) are used in addition to the biomarkers (i) to (x):

(xi) cumulative level of cytosine methylation at gene positions FHL_2_CpG_19, and FHL_2_CpG_20 in blood cells;
(xii) number of T cell receptor excision circles (Trec) in 100,000 T cells from whole blood;
(xiii) decadic logarithm of the ratio between the concentration of NGA2F glycan in serum and the concentration of NA2F glycan in serum;
(xiv) concentration of prostate specific antigen (PSA) in serum;
(xv) concentration of fibrinogen in plasma;
(xvi) concentration of albumin in piasma;
(xvii) concentration of glucose in serum;
(xviii) concentration of NGA2FB glycan in serum;
(xix) ratio of the concentration of Cu in plasma to the concentration of Zn in plasma;
(xx) ratio of the number of CD16$^+$CD56$^+$ cells in whole blood to the number of CD45$^+$ cells in whole blood;
(xxi) concentration of antibodies directed against *Clostridium tetani* in plasma;
(xxii) ratio of the number of CD3$^+$CD8$^+$ cells in whole blood to the number of CD45$^+$ cells in whole blood;
(xxiii) concentration of beta-hydroxyisovalerate in urine.

13. The use according to claim 9 or claim 11, wherein only the biomarkers (i) to (x) are used.

**Patentansprüche**

1. Verfahren zur Bestimmung des biologischen Alters eines Menschen, umfassend die Schritte des:

(a) Bereitstellens biologischer Proben des Menschen, ausgewählt von der Gruppe, bestehend aus Vollblut, Serum, Plasma und Urin, wobei die Bereitstellung der biologischen Proben keine Interaktion mit dem menschlichen Körper beinhaltet,

(b1) falls der Mensch weiblich ist, Bestimmens mindestens der nachstehenden Biomarker (i) bis (x) in den jeweiligen biologischen Proben, wobei die notwendigen biologischen Proben in Schritt (a) bereitgestellt werden:

(i) kumulativer Grad der Cytosin-Methylierung an Genpositionen ELOVL_2_CpG_11, ELOVL_2_CpG_12, ELOVL_2_CpG_13 und ELOVL_2_CpG_14 in Blutzellen;
(ii) kumulativer Grad der Cytosin-Methylierung an Genpositionen ELOVL_2_CpG_15, ELOVL_2_CpG_16 und ELOVL_2_CpG_17 in Blutzellen;
(iii) kumulativer Grad der Cytosin-Methylierung an Genpositionen FHL_2_CpG_11 und FHL_2_CpG_12 in Blutzellen;
(iv) kumulativer Grad der Cytosin-Methylierung an Genpositionen FHL_2_CpG_13, FHL_2_CpG_14 und FHL_2_CpG_15 in Blutzellen;
(v) kumulativer Grad der Cytosin-Methylierung an Genpositionen FHL_2_CpG_16 und FHL_2_CpG_17 in Blutzellen;
(vi) Konzentration von Dehydroepiandrosteronsulfat in Plasma;
(vii) Konzentration von Ferritin in Serum;
(viii) dekadischer Logarithmus des Verhältnisses zwischen der Konzentration von NGA2F-Glykan in Serum und der Konzentration von NA2F-Glykan in Serum;
(ix) Konzentration von alpha-Tocopherol in Plasma;
(x) Konzentration von S_p6_n_Glykan in Serum; oder

(b2) falls der Mensch männlich ist, Bestimmens mindestens der nachstehenden Biomarker (i) bis (x) in den jeweiligen biologischen Proben, wobei die notwendigen biologischen Proben in Schritt (a) bereitgestellt werden:

(i) kumulativer Grad der Cytosin-Methylierung an Genpositionen ELOVL_2_CpG_11, ELOVL_2_CpG_12, ELOVL_2_CpG_13 und ELOVL_2_CpG_14 in Blutzellen;
(ii) kumulativer Grad der Cytosin-Methylierung an Genpositionen ELOVL_2_CpG_15, ELOVL_2_CpG_16 und ELOVL_2_CpG_17 in Blutzellen;
(iii) kumulativer Grad der Cytosin-Methylierung an Genpositionen FHL_2_CpG_13, FHL_2_CpG_14 und FHL_2_CpG_15 in Blutzellen;
(iv) kumulativer Grad der Cytosin-Methylierung an Genposition FHL_2_CpG_18;
(v) Konzentration von Kreatinin in Urin;
(vi) Verhältnis der Anzahl von $CD3^+CD4^+$-Zellen in Vollblut zu der Anzahl von $CD8^+$-Zellen in Vollblut;
(vii) Konzentration von Dehydroepiandrosteronsulfat in Plasma;
(viii) Konzentration von alpha-2-Makroglobulin in Serum;
(ix) Konzentration von Lycopin in Plasma;
(x) Konzentration von S_p6_n_Glykan in Serum; und

(c) Bestimmens des biologischen Alters des Menschen auf Basis der Biomarker, bestimmt in Schritt (b1) oder (b2) durch

(c1) Aufstellen eines mehrfachen-linearen-Regressions-Modells für die bestimmten Biomarker auf Basis der Daten, bereitgestellt in der Studie, die der vorliegenden Erfindung zu Grunde liegt;
(c2) Bestimmen von dem in Schritt (c1) aufgestellten mehrfachen-linearen-Regressions-Modell

(i) den Korrelationskoeffizienten $r^2$,
(ii) die Gewichtungsfaktoren $b_i$ für jeden bestimmten Biomarker, wobei i 1 bis n ist und n die Anzahl der bestimmten Biomarker ist, und
(iii) den Schnittpunkt c;

(c3) Berechnen des scheinbaren biologischen Alters B nach Formel (1)

$$B = \sum_{i=1}^{n} b_i x_i + c \qquad (1)$$

wobei

b$_i$ die Gewichtungsfaktoren, bestimmt in Schritt (c2), sind,

x$_i$ die Werte der jeweilgen Biomarker, wie in Schritt (b1) oder (b2) bestimmt, sind, wobei i 1 bis n ist und

c der Schnittpunkt, bestimmt in Schritt (c2) ist; und

(c4) Berechnen des korrigierten biologischen Alters (Bioage) nach Formel (2)

$$Bioage = \frac{B - mean(a)}{r^2} + mean(a) \qquad (2)$$

wobei

B das scheinbare biologische Alter, bestimmt in Schritt (c3), ist und

mean(a) das mittlere chronologische Alter der statistischen Bevölkerung, verwendet in der Studie, die der vorliegenden Erfindung zu Grunde liegt, ist.

2.  Verfahren nach Anspruch 1, wobei der Mensch weiblich ist, in Schritt (b1) einer oder mehrere der nachstehenden Biomarker (xi) bis (xxviii) zusätzlich zu den Biomarkern (i) bis (x) bestimmt werden und die notwendigen biologischen Proben im Schritt (a) bereitgestellt werden:

(xi) kumulativer Grad der Cytosin-Methylierung an Genpositionen ELOVL_2_CpG_22, ELOVL_2_CpG_23 und ELOVL_2_CpG_24 in Blutzellen;

(xii) kumulativer Grad der Cytosin-Methylierung an Genpositionen FHL_2_CpG_19 und FHL_2_CpG_20 in Blutzellen;

(xiii) Konzentration von NGA2FB-Glykan in Serum;

(xiv) Konzentration von Fibrinogen in Plasma;

(xv) Konzentration von Harnstoff in Plasma;

(xvi) Konzentration von Glucose in Serum;

(xvii) Länge von Telomeren in Blutzellen;

(xviii) Konzentration von Triglyceriden in Serum;

(xix) Konzentration von Immunoglobulin M (IgM) in Serum;

(xx) Konzentration von Adiponektin in Serum;

(xxi) kumulative Konzentration von Threonin und Lactat in Urin;

(xxii) Konzentration von Homocystein in Plasma;

(xxiii) Konzentration von Lycopin in Plasma;

(xxiv) Konzentration von Cholesterin in Serum;

(xxv) Konzentration von glycosyliertem Hämoglobin (HbA$_{1c}$) in Vollblut;

(xxvi) Konzentration von Glycin in Urin;

(xxvii) Anzahl von T-Zell-Rezeptor-Exzisions-Kreisen (Trec) in 100000 T-Zellen aus Vollblut;

(xxviii) Konzentration von Kreatinin in Urin.

3.  Verfahren nach Anspruch 1, wobei der Mensch männlich ist, in Schritt (b2) einer oder mehrere der nachstehenden Biomarker (xi) bis (xxiii) zusätzlich zu den Biomarkern (i) bis (x) bestimmt werden und die notwendigen biologischen Proben im Schritt (a) bereitgestellt werden:

(xi) kumulativer Grad der Cytosin-Methylierung an Genpositionen FHL_2_CpG_19 und FHL_2_CpG_20 in Blutzellen;

(xii) Anzahl von T-Zell-Rezeptor-Exzisions-Kreisen (Trec) in 100000 T-Zellen aus Vollblut;

(xiii) dekadischer Logarithmus des Verhältnisses zwischen der Konzentration von NGA2F-Glykan in Serum und der Konzentration von NA2F-Glykan in Serum;

(xiv) Konzentration von prostataspezifischem Antigen (PSA) in Serum;

(xv) Konzentration von Fibrinogen in Plasma;

(xvi) Konzentration von Albumin in Plasma;

(xvii) Konzentration von Glucose in Serum;

(xviii) Konzentration von NGA2FB-Glykan in Serum;

(xix) Verhältnis der Konzentration von Cu in Plasma zu der Konzentration von Zn in Plasma;

(xx) Verhältnis der Anzahl von CD16$^+$CD56$^+$-Zellen in Vollblut zu der Anzahl von CD45$^+$-Zellen in Vollblut;

(xxi) Konzentration von IgG-Antikörpern, gerichtet gegen *Clostridium tetani,* in Plasma;

(xxii) Verhältnis der Anzahl von CD3$^+$CD8$^+$-Zellen in Vollblut zu der Anzahl von CD45$^+$-Zellen in Vollblut;
(xxiii) Konzentration von beta-Hydroxyisovalerat in Urin.

4. Verfahren nach Anspruch 1, wobei der Mensch weiblich ist, die Biomarker (i) bis (x) in Schritt (b1) bestimmt werden und das Bestimmen des biologischen Alters des Menschen, basierend auf diesen Biomarkern, in Schritt (c) umfasst die Schritte des:

(c1) Berechnens eines scheinbaren biologischen Alters B nach Formel (3)

$$B = \sum_{i=1}^{10} b_i x_i + c \qquad (3)$$

wobei

$b_1$ eine Zahl zwischen 40,37 und 49,33 ist,
$x_1$ der bestimmte Wert des Biomarkers (i), ausgedrückt als ein Prozentwert zwischen 0 und 1, ist,
$b_2$ eine Zahl zwischen 42,86 und 52,37 ist,
$x_2$ der bestimmte Wert des Biomarkers (ii), ausgedrückt als ein Prozentwert zwischen 0 und 1, ist,
$b_3$ eine Zahl zwischen -28,00 und -22,92 ist,
$x_3$ der bestimmte Wert des Biomarkers (iii), ausgedrückt als ein Prozentwert zwischen 0 und 1, ist,
$b_4$ eine Zahl zwischen 26,52 und 32,40 ist,
$x_4$ der bestimmte Wert des Biomarkers (iv), ausgedrückt als ein Prozentwert zwischen 0 und 1, ist,
$b_5$ eine Zahl zwischen 11,88 und 14,50 ist,
$x_5$ der bestimmte Wert des Biomarkers (v), ausgedrückt als ein Prozentwert zwischen 0 und 1, ist,
$b_6$ eine Zahl zwischen -0,97 und -0,80 ist,
$x_6$ der bestimmte Wert des Biomarkers (vi), ausgedrückt in $\mu$g pro dL, ist,
$b_7$ eine Zahl zwischen 0,023 und 0,027 ist,
$x_7$ der bestimmte Wert des Biomarkers (vii), ausgedrückt in ng pro mL, ist,
$b_8$ eine Zahl zwischen 9,78 und 11,94 ist,
$x_8$ der bestimmte Wert des Biomarkers (viii) ist,
$b_9$ eine Zahl zwischen 0,09 und 0,11 ist,
$x_9$ der bestimmte Wert des Biomarkers (ix), ausgedrückt in $\mu$Mol pro L, ist,
$b_{10}$ eine Zahl zwischen 0,028 und 0,034 ist,
$x_{10}$ der bestimmte Wert des Biomarkers (x), ausgedrückt in $\mu$Mol pro L, ist und
c eine Zahl zwischen -14,47 und -11,85 ist; und

(c2) Berechnen des korrigierten biologischen Alters (Bioage) nach Formel (2)

$$Bioage = \frac{B - mean(a)}{r^2} + mean(a) \qquad (2)$$

wobei

B das scheinbare biologische Alter, bestimmt in Schritt (c1), ist,
mean(a) eine Zahl zwischen 50,27 und 61,43 ist und
$r^2$ eine Zahl zwischen 0,79 und 0,95 ist.

5. Verfahren nach Anspruch 4, wobei

| | |
|---|---|
| $b_1$ | 44,85 ist, |
| $b_2$ | 47,616 ist, |
| $b_3$ | -25,457 ist, |
| $b_4$ | 29,463 ist, |
| $b_5$ | 13,19 ist, |

(fortgesetzt)

| | |
|---|---|
| $b_6$ | -0,889 ist, |
| $b_7$ | 0,025 ist, |
| $b_8$ | 10,863 ist, |
| $b_9$ | 0,1 ist, |
| $b_{10}$ | 0,031 ist, |
| c | -13,16 ist, |
| mean(a) | 55,85 ist und |
| $r^2$ | 0,87 ist. |

6. Verfahren nach Anspruch 1, wobei der Mensch männlich ist, die Biomarker (i) bis (x) in Schritt (b2) bestimmt werden und das Bestimmen des biologischen Alters des Menschen, basierend auf diesen Biomarkern, in Schritt (c) umfasst die Schritte des:

(c1) Berechnens eines scheinbaren biologischen Alters B nach Formel (3)

$$B = \sum_{i=1}^{10} b_i x_i + c \qquad (3)$$

wobei

$b_1$ eine Zahl zwischen 32,84 und 40,12 ist,
$x_1$ der bestimmte Wert des Biomarkers (i), ausgedrückt als ein Prozentwert zwischen 0 und 1, ist,
$b_2$ eine Zahl zwischen 33,53 und 40,97 ist,
$x_2$ der bestimmte Wert des Biomarkers (ii), ausgedrückt als ein Prozentwert zwischen 0 und 1, ist,
$b_3$ eine Zahl zwischen 42,52 und 51,95 ist,
$x_3$ der bestimmte Wert des Biomarkers (iii), ausgedrückt als ein Prozentwert zwischen 0 und 1, ist,
$b_4$ eine Zahl zwischen 4,06 und 4,95 ist,
$x_4$ der bestimmte Wert des Biomarkers (iv), ausgedrückt als ein Prozentwert zwischen 0 und 1, ist,
$b_5$ eine Zahl zwischen -0,18 und -0,16 ist,
$x_5$ der bestimmte Wert des Biomarkers (v) ist,
$b_6$ eine Zahl zwischen 1,39 und 1,69 ist,
$x_6$ der bestimmte Wert des Biomarkers (vi) ist,
$b_7$ eine Zahl zwischen -1,14 und -0,94 ist,
$x_7$ der bestimmte Wert des Biomarkers (vii), ausgedrückt in g pro dL, ist,
$b_8$ eine Zahl zwischen 0,022 und 0,026 ist,
$x_8$ der bestimmte Wert des Biomarkers (viii), ausgedrückt in mg pro dL, ist,
$b_9$ eine Zahl zwischen -3,44 und -2,82 ist,
$x_9$ der bestimmte Wert des Biomarkers (ix), ausgedrückt in $\mu$Mol pro L, ist,
$b_{10}$ eine Zahl zwischen 0,027 und 0,031 ist,
$x_{10}$ der bestimmte Wert des Biomarkers (x), ausgedrückt in $\mu$Mol pro L, ist und
c eine Zahl zwischen -4,24 und -3,47 ist; und

(c2) Berechnen des korrigierten biologischen Alters (Bioage) nach Formel (2)

$$Bioage = \frac{B - mean(a)}{r^2} + mean(a) \qquad (2)$$

wobei

B das scheinbare biologische Alter, bestimmt in Schritt (c1), ist,
mean(a) eine Zahl zwischen 50,27 und 61,43 ist und
$r^2$ eine Zahl zwischen 0,78 und 0,94 ist.

**7.** Verfahren nach Anspruch 6, wobei

| | |
|---|---|
| $b_1$ | 36,481 ist, |
| $b_2$ | 37,25 ist, |
| $b_3$ | 47,234 ist, |
| $b_4$ | 4,506 ist, |
| $b_5$ | -0,172 ist, |
| $b_6$ | 1,538 ist, |
| $b_7$ | -1,044 ist, |
| $b_8$ | 0,024 ist, |
| $b_9$ | -3,131 ist |
| $b_{10}$ | 0,029 ist, |
| c | -3,858 ist, |
| mean(a) | 55,85 ist und |
| $r^2$ | 0,86 ist. |

**8.** Verfahren nach einem der Ansprüche 1 bis 7, wobei der Mensch ein chronologisches Alter zwischen 30 und 80 Jahren hat.

**9.** Verwendung von mindestens der nachstehenden Biomarker (i) bis (x) zur Bestimmung des biologischen Alters eines weiblichen Menschen gemäß dem Verfahren der Ansprüche 1, 2, 4, 5 und 8:

(i) kumulativer Grad der Cytosin-Methylierung an Genpositionen ELOVL_2_CpG_11, ELOVL_2_CpG_12, ELOVL_2_CpG_13 und ELOVL_2_CpG_14 in Blutzellen;
(ii) kumulativer Grad der Cytosin-Methylierung an Genpositionen ELOVL_2_CpG_15, ELOVL_2_CpG_16 und ELOVL_2_CpG_17 in Blutzellen;
(iii) kumulativer Grad der Cytosin-Methylierung an Genpositionen FHL_2_CpG_11 und FHL_2_CpG_12 in Blutzellen;
(iv) kumulativer Grad der Cytosin-Methylierung an Genpositionen FHL_2_CpG_13, FHL_2_CpG_14 und FHL_2_CpG_15 in Blutzellen;
(v) kumulativer Grad der Cytosin-Methylierung an Genpositionen FHL_2_CpG_16 und FHL_2_CpG_17 in Blutzellen;
(vi) Konzentration von Dehydroepiandrosteronsulfat in Plasma;
(vii) Konzentration von Ferritin in Serum;
(viii) dekadischer Logarithmus des Verhältnisses zwischen der Konzentration von NGA2F-Glykan in Serum und der Konzentration von NA2F-Glykan in Serum;
(ix) Konzentration von alpha-Tocopherol in Plasma;
(x) Konzentration von Cystein in Plasma.

**10.** Verwendung nach Anspruch 9, wobei einer oder mehrere der nachstehenden Biomarker (xi) bis (xxviii) zusätzlich zu den Biomarkern (i) bis (x) verwendet werden:

(xi) kumulativer Grad der Cytosin-Methylierung an Genpositionen ELOVL_2_CpG_22, ELOVL_2_CpG_23 und ELOVL_2_CpG_24 in Blutzellen;
(xii) kumulativer Grad der Cytosin-Methylierung an Genpositionen FHL_2_CpG_19 und FHL_2_CpG_20 in Blutzellen;
(xiii) Konzentration von NGA2FB-Glykan in Serum;
(xiv) Konzentration von Fibrinogen in Plasma;
(xv) Konzentration von Harnstoff in Plasma;
(xvi) Konzentration von Glucose in Serum;
(xvii) Länge von Telomeren in Blutzellen;
(xviii) Konzentration von Triglyceriden in Serum;
(xix) Konzentration von Immunoglobulin M (IgM) in Serum;
(xx) Konzentration von Adiponektin in Serum;
(xxi) kumulative Konzentration von Threonin und Lactat in Urin;

(xxii) Konzentration von Homocystein in Plasma;

(xxiii) Konzentration von Lycopin in Plasma;

(xxiv) Konzentration von Cholesterin in Serum;

(xxv) Konzentration von glycosyliertem Hämoglobin (HbA$_{1c}$) in Vollblut;

(xxvi) Konzentration von Glycin in Urin;

(xxvii) Anzahl von T-Zell-Rezeptor-Exzisions-Kreisen (Trec) in 100000 T-Zellen aus Vollblut;

(xxviii) Konzentration von Kreatinin in Urin.

**11.** Verwendung von mindestens der nachstehenden Biomarker (i) bis (x) zur Bestimmung des biologischen Alters eines männlichen Menschen gemäß dem Verfahren der Ansprüche 1, 3, 6-8:

(i) kumulativer Grad der Cytosin-Methylierung an Genpositionen ELOVL_2_CpG_11, ELOVL_2_CpG_12, ELOVL_2_CpG_13 und ELOVL_2_CpG_14 in Blutzellen;

(ii) kumulativer Grad der Cytosin-Methylierung an Genpositionen ELOVL_2_CpG_15, ELOVL_2_CpG_16 und ELOVL_2_CpG_17 in Blutzellen;

(iii) kumulativer Grad der Cytosin-Methylierung an Genpositionen FHL_2_CpG_13, FHL_2_CpG_14 und FHL_2_CpG_15 in Blutzellen;

(iv) kumulativer Grad der Cytosin-Methylierung an Genposition FHL_2_CpG_18;

(v) Konzentration von Kreatinin in Urin;

(vi) Verhältnis der Anzahl von CD3$^+$CD4$^+$-Zellen in Vollblut zu der Anzahl von CD8$^+$-Zellen in Vollblut;

(vii) Konzentration von Dehydroepiandrosteronsulfat in Plasma;

(viii) Konzentration von alpha-2-Makroglobulin in Serum;

(ix) Konzentration von Lycopin in Plasma;

(x) Konzentration von Cystein in Plasma.

**12.** Verwendung nach Anspruch 11, wobei einer oder mehrere der nachstehenden Biomarker (xi) bis (xxiii) zusätzlich zu den Biomarkern (i) bis (x) verwendet werden:

(xi) kumulativer Grad der Cytosin-Methylierung an Genpositionen FHL_2_CpG_19 und FHL_2_CpG_20 in Blutzellen;

(xii) Anzahl von T-Zell-Rezeptor-Exzisions-Kreisen (Trec) in 100000 T-Zellen aus Vollblut;

(xiii) dekadischer Logarithmus des Verhältnisses zwischen der Konzentration von NGA2F-Glykan in Serum und der Konzentration von NA2F-Glykan in Serum;

(xiv) Konzentration von prostataspezifischem Antigen (PSA) in Serum;

(xv) Konzentration von Fibrinogen in Plasma;

(xvi) Konzentration von Albumin in Plasma;

(xvii) Konzentration von Glucose in Serum;

(xviii) Konzentration von NGA2FB-Glykan in Serum;

(xix) Verhältnis der Konzentration von Cu in Plasma zu der Konzentration von Zn in Plasma;

(xx) Verhältnis der Anzahl von CD16$^+$CD56$^+$-Zellen in Vollblut zu der Anzahl von CD45$^+$-Zellen in Vollblut;

(xxi) Konzentration von Antikörpern, gerichtet gegen *Clostridium tetani,* in Plasma;

(xxii) Verhältnis der Anzahl von CD3$^+$CD8$^+$-Zellen in Vollblut zu der Anzahl von CD45$^+$-Zellen in Vollblut;

(xxiii) Konzentration von beta-Hydroxyisovalerat in Urin.

**13.** Verwendung nach Anspruch 9 oder Anspruch 11, wobei nur die Biomarker (i) bis (x) verwendet werden.

**Revendications**

**1.** Procédé pour la détermination de l'âge biologique d'un être humain, comprenant les étapes de :

(a) fourniture d'échantillons biologiques, choisis dans le groupe constitué du sang total, du sérum, du plasma, et de l'urine, provenant de l'être humain, dans lequel la fourniture des échantillons biologiques ne comprend aucune interaction avec le corps humain,

(b1) dans le cas où l'être humain est une femme, la détermination d'au moins les biomarqueurs (i) à (x) suivants dans les échantillons biologiques respectifs, dans laquelle les échantillons biologiques nécessaires sont fournis à l'étape (a) :

(i) le taux cumulatif de méthylation des cytosines au niveau des positions de gènes ELOVL_2_CpG_11, ELOVL_2_CpG_12, ELOVL_2_CpG_13, et ELOVL_2_CpG_14 dans les cellules sanguines ;

(ii) le taux cumulatif de méthylation des cytosines au niveau des positions de gènes ELOVL_2_CpG_15, ELOVL_2_CpG_16, et ELOVL_2_CpG_17 dans les cellules sanguines ;

(iii) le taux cumulatif de méthylation des cytosines au niveau des positions de gènes FHL_2_CpG_11, et FHL_2_CpG_12 dans les cellules sanguines ;

(iv) le taux cumulatif de méthylation des cytosines au niveau des positions de gènes FHL_2_CpG_13, FHL_2_CpG_14, et FHL_2_CpG_15 dans les cellules sanguines ;

(v) le taux cumulatif de méthylation des cytosines au niveau des positions de gènes FHL_2_CpG_16 et FHL_2_CpG_17 dans les cellules sanguines ;

(vi) la concentration de sulfate de déhydroépiandrostérone dans le plasma ;

(vii) la concentration de ferritine dans le sérum ;

(viii) le logarithme décimal du rapport entre la concentration du glycane NGA2F dans le sérum et la concentration du glycane NA2F dans le sérum ;

(ix) la concentration d'alpha-tocophérol dans le plasma ;

(x) la concentration de S_p6_n_glycane dans le sérum ; ou

(b2) dans le cas où l'être humain est un homme, la détermination d'au moins les biomarqueurs (i) à (x) suivants dans les échantillons biologiques respectifs, dans lequel les échantillons biologiques nécessaires sont fournis à l'étape (a) :

(i) le taux cumulatif de méthylation des cytosines au niveau des positions de gènes ELOVL_2_CpG_11, ELOVL_2 CpG_12, ELOVL_2_CpG_13, et ELOVL_2_CpG_14 dans les cellules sanguines ;

(ii) le taux cumulatif de méthylation des cytosines au niveau des positions de gènes ELOVL_2_CpG_15, ELOVL_2_CpG_16, et ELOVL_2_CpG_17 dans les cellules sanguines ;

(iii) le taux cumulatif de méthylation des cytosines au niveau des positions de gènes FHL_2_CpG_13, FHL_2_CpG_14, et FHL_2_CpG_15 dans les cellules sanguines ;

(iv) le taux cumulatif de méthylation des cytosines au niveau de la position de gène FHL_2_CpG_18 ;

(v) la concentration de créatinine dans l'urine ;

(vi) le rapport du nombre des cellules $CD3^+CD4^+$ dans le sang total sur le nombre des cellules $CD8^+$ dans le sang total ;

(vii) la concentration de sulfate de déhydroépiandrostérone dans le plasma ;

(viii) la concentration d'alpha-2-macroglobuline dans le sérum ;

(ix) la concentration de lycopène dans le plasma ;

(x) la concentration de S_p6_n_glycane dans le sérum ; et

(c) détermination de l'âge biologique de l'être humain sur la base des biomarqueurs déterminés à l'étape (b1) ou (b2) par

(c1) l'établissement d'un modèle de régression linéaire multiple pour les biomarqueurs déterminés sur la base des données fournies dans l'étude sous-jacente de la présente invention ;

(c2) la détermination à partir du modèle de régression linéaire multiple établi à l'étape (c1) :

(i) du coefficient de corrélation $r^2$,

(ii) des facteurs de pondération $b_i$ pour chaque biomarqueur déterminé, dans lequel i vaut 1 à n, et n est le nombre des biomarqueurs déterminés, et

(iii) de l'intersection c ;

(c3) le calcul d'un âge biologique apparent B selon la formule (1) :

$$B = \sum_{i}^{n} b_i x_i + c \qquad (1)$$

dans laquelle :

$b_i$ représente les facteurs de pondération déterminés à l'étape (c2),

$x_i$ représente les valeurs des biomarqueurs respectifs telles que déterminées à l'étape (b1) ou (b2),

dans laquelle i vaut 1 à n et
c représente l'intersection déterminée à l'étape (c2) ; et

(c4) le calcul de l'âge biologique corrigé (Bioage) selon la formule (2) :

$$Bioage = \frac{B - moyenne(a)}{r^2} + moyenne(a) \qquad (2)$$

dans laquelle :

B représente l'âge biologique apparent déterminé à l'étape (c3), et
moyenne(a) représente l'âge chronologique moyen de la population statistique utilisée dans l'étude sous-jacente de la présente invention.

**2.** Procédé selon la revendication 1, dans lequel l'être humain est une femme, à l'étape (b1) un ou plusieurs des biomarqueurs (xi) à (xxviii) suivants sont déterminés en plus des biomarqueurs (i) à (x), et les échantillons biologiques nécessaires sont fournis à l'étape (a) :

(xi) le taux cumulatif de méthylation des cytosines au niveau des positions de gènes ELOVL_2_CpG_22, ELOVL_2_CpG_23, et ELOVL_2_CpG_24 dans les cellules sanguines ;
(xii) le taux cumulatif de méthylation des cytosines au niveau des positions de gènes FHL_2_CpG_19, et FHL_2_CpG_20 dans les cellules sanguines ;
(xiii) la concentration de glycane NGA2FB dans le sérum ;
(xiv) la concentration de fibrinogène dans le plasma ;
(xv) la concentration d'urée dans le plasma ;
(xvi) la concentration de glucose dans le sérum ;
(xvii) la longueur des télomères dans les cellules sanguines ;
(xviii) la concentration de triglycérides dans le sérum ;
(xix) la concentration d'immunoglobuline M (IgM) dans le sérum ;
(xx) la concentration d'adiponectine dans le sérum ;
(xxi) la concentration accumulée de thréonine et de lactate dans l'urine ;
(xxii) la concentration d'homocystéine dans le plasma ;
(xxiii) la concentration de lycopène dans le plasma ;
(xxiv) la concentration de cholestérol dans le sérum ;
(xxv) la concentration d'hémoglobine glycosylée (HbA1c) dans le sang total ;
(xxvi) la concentration de glycine dans l'urine ;
(xxvii) le nombre des cercles d'excision des récepteurs de lymphocytes T (Trec) dans 100 000 lymphocytes T provenant du sang total ;
(xxviii) la concentration de créatinine dans l'urine.

**3.** Procédé selon la revendication 1, dans lequel l'être humain est un homme, à l'étape (b2) un ou plusieurs des biomarqueurs (xi) à (xxiii) suivants sont déterminés en plus des biomarqueurs (i) à (x), et les échantillons biologiques nécessaires sont fournis à l'étape (a) :

(xi) le taux cumulatif de méthylation des cytosines au niveau des positions de gènes FHL_2_CpG_19, et FHL_2_CpG_20 dans les cellules sanguines ;
(xii) le nombre des cercles d'excision des récepteurs de lymphocytes T (Trec) dans 100 000 lymphocytes T provenant du sang total ;
(xiii) le logarithme décimal du rapport entre la concentration du glycane NGA2F dans le sérum et la concentration du glycane NA2F dans le sérum ;
(xiv) la concentration d'antigène spécifique de la prostate (PSA) dans le sérum ;
(xv) la concentration de fibrinogène dans le plasma ;
(xvi) la concentration d'albumine dans le plasma ;
(xvii) la concentration de glucose dans le sérum ;
(xviii) la concentration de glycane NGA2FB dans le sérum ;
(xix) le rapport de la concentration de Cu dans le plasma sur la concentration de Zn dans le plasma ;
(xx) le rapport du nombre des cellules CD16$^+$CD56$^+$ dans le sang total sur le nombre des cellules CD45$^+$ dans le sang total ;
(xxi) la concentration d'anticorps IgG dirigés contre Clostridium tetani dans le plasma ;
(xxii) le rapport du nombre des cellules CD3+CD8+ dans le sang total sur le nombre de cellules CD45+ dans le sang total ;
(xxiii) la concentration de bêta-hydroxy-isovalérate dans l'urine.

4. Procédé selon la revendication 1, dans lequel l'être humain est une femme, les biomarqueurs (i) à (x) sont déterminés à l'étape (b1), et la détermination de l'âge biologique de l'être humain sur la base desdits biomarqueurs à l'étape (c) comprend les étapes de : ccc

(c1) calcul d'un âge biologique apparent B selon la formule (3)

$$B = \sum_{i=1}^{10} b_i x_i + c \qquad (3)$$

$b_1$ est un nombre entre 40,37 and 49,33,
$x_1$ est la valeur déterminée du biomarqueur (i) exprimée en un pourcentage entre 0 et 1,
$b_2$ est un nombre entre 42,86 et 52,37,
$x_2$ est la valeur déterminée du biomarqueur (ii) exprimée en un pourcentage entre 0 et 1,
$b_3$ est un nombre entre -28,00 et -22,92,
$x_3$ est la valeur déterminée du biomarqueur (iii) exprimée en un pourcentage entre 0 et 1,
$b_4$ est un nombre entre 26,52 et 32,40,
$x_4$ est la valeur déterminée du biomarqueur (iv) exprimée en un pourcentage entre 0 et 1,
$b_5$ est un nombre entre 11,88 et 14,50,
$x_5$ est la valeur déterminée du biomarqueur (v) exprimée en un pourcentage entre 0 et 1,
$b_6$ est un nombre entre -0,97 et -0,80,
$x_6$ est la valeur déterminée du biomarqueur (vi) exprimée en $\mu$g par dl,
$b_7$ est un nombre entre 0,023 et 0,027,
$x_7$ est la valeur déterminée du biomarqueur (vii) exprimée en ng par ml,
$b_8$ est un nombre entre 9,78 et 11,94,
$x_8$ est la valeur déterminée du biomarqueur (viii), $b_9$ est un nombre entre 0,09 et 0,11,
$x_9$ est la valeur déterminée du biomarqueur (ix) exprimée en $\mu$mole par l,
$b_{10}$ est un nombre entre 0,028 et 0,034,
$x_{10}$ est la valeur déterminée du biomarqueur (x) exprimée en $\mu$mole par 1, et

c est un nombre entre -14,47 et -11,85 ; et (c2) calcul de l'âge biologique corrigé (Bioage) selon la formule (2)

$$Bioage = \frac{B - moyenne(a)}{r^2} + moyenne(a) \qquad (2)$$

dans laquelle :

B représente l'âge biologique apparent déterminé à l'étape (c1),
moyenne(a) est un nombre entre 50,27 et 61,43, et
$r^2$ est un nombre entre 0,79 et 0,95.

5. Procédé selon la revendication 4, dans lequel
$b_1$ vaut 44,85,
$b_2$ vaut 47,616,
$b_3$ vaut -25,457,
$b_4$ vaut 29,463,
$b_5$ vaut 13,19,
$b_6$ vaut -0,889,
$b_7$ vaut 0,025,
$b_8$ vaut 10,863,

$b_9$ vaut 0,1,
$b_{10}$ vaut 0,031,
c vaut -13,16,
moyenne(a) vaut 55,85, et
$r^2$ vaut 0,87.

**6.** Procédé selon la revendication 1, dans lequel l'être humain est un homme, les biomarqueurs (ix) à (x) sont déterminés à l'étape (b2), et la détermination de l'âge biologique de l'être humain sur la base desdits biomarqueurs à l'étape (c) comprend les étapes de :

(c1) calcul d'un âge biologique apparent B selon la formule (3)

$$B = \sum_{i=1}^{10} b_i x_i + c \qquad (3)$$

dans laquelle :

$b_1$ est un nombre entre 32,84 et 40,12,
$x_1$ est la valeur déterminée du biomarqueur (i) exprimée en un pourcentage entre 0 et 1,
$b_2$ est un nombre entre 33,53 et 40,97,
$x_2$ est la valeur déterminée du biomarqueur (ii) exprimée en un pourcentage entre 0 et 1,
$b_3$ est un nombre entre 42,52 et 51,95,
$x_3$ est la valeur déterminée du biomarqueur (iii) exprimée en un pourcentage entre 0 et 1,
$b_4$ est un nombre entre 4,06 et 4,95,
$x_4$ est la valeur déterminée du biomarqueur (iv) exprimée en un pourcentage entre 0 et 1,
$b_5$ est un nombre entre -0,18 et -0,16,
$x_5$ est la valeur déterminée du biomarqueur (v),
$b_6$ est un nombre entre 1,39 et 1,69,
$x_6$ est la valeur déterminée du biomarqueur (vi),
$b_7$ est un nombre entre -1,14 et -0,94,
$x_7$ est la valeur déterminée du biomarqueur (vii) exprimée en g par dl,
$b_8$ est un nombre entre 0,022 et 0,026,
$x_8$ est la valeur déterminée du biomarqueur (viii) exprimée en mg par dl,
$b_9$ est un nombre entre -3,44 et -2,82,
$x_9$ est la valeur déterminée du biomarqueur (ix) exprimée en $\mu$mole par 1,
$b_{10}$ est un nombre entre 0,027 et 0,031,
$x_{10}$ est la valeur déterminée du biomarqueur (x) exprimée en $\mu$mole par 1, et
c est un nombre entre -4,24 et -3,47 ; et

(c2) calcul de l'âge biologique corrigé (Bioage) selon la formule (2)

$$Bioage = \frac{B - moyenne(a)}{r^2} + moyenne(a) \qquad (2)$$

dans laquelle :

B représente l'âge biologique apparent déterminé à l'étape (c1),
moyenne(a) est un nombre entre 50,27 et 61,43, et
$r^2$ est un nombre entre 0,78 et 0,94.

**7.** Procédé selon la revendication 6, dans lequel :

$b_1$ vaut 36,481,
$b_2$ vaut 37,25,
$b_3$ vaut 47,234,

$b_4$ vaut 4,506,
$b_5$ vaut -0,172,
$b_6$ vaut 1,538,
$b_7$ vaut -1,044,
$b_8$ vaut 0,024,
$b_9$ vaut -3,131,
$b_{10}$ vaut 0, 029,
c vaut -3,858,
moyenne(a) vaut 55,85, et
$r^2$ vaut 0,86.

8. Procédé selon l'une quelconque des revendications 1 à 7, dans lequel l'être humain a un âge chronologique situé entre 30 et 80 ans.

9. Utilisation d'au moins les biomarqueurs (i) à (x) suivants pour la détermination de l'âge biologique d'un être humain femme selon le procédé des revendications 1, 2, 4, 5 et 8 :

(i) le taux cumulatif de méthylation des cytosines au niveau des positions de gènes ELOVL_2_CpG_11, ELOVL_2_CpG_12, ELOVL_2_CpG_13, et ELOVL_2_CpG_14 dans les cellules sanguines ;
(ii) le taux cumulatif de méthylation des cytosines au niveau des positions de gènes ELOVL_2_CpG_15, ELOVL_2_CpG_16, et ELOVL_2_CpG_17 dans les cellules sanguines ;
(iii) le taux cumulatif de méthylation des cytosines au niveau des positions de gènes FHL_2_CpG_11, et FHL_2_CpG_12 dans les cellules sanguines ;
(iv) le taux cumulatif de méthylation des cytosines au niveau des positions de gènes FHL_2_CpG_13, FHL_2_CpG_14, et FHL_2_CpG_15 dans les cellules sanguines ;
(v) le taux cumulatif de méthylation des cytosines au niveau des positions de gènes FHL_2_CpG_16 et FHL_2_CpG_17 dans les cellules sanguines ;
(vi) la concentration de sulfate de déhydroépiandrostérone dans le plasma ;
(vii) la concentration de ferritine dans le sérum ;
(viii) le logarithme décimal du rapport entre la concentration du glycane NGA2F dans le sérum et la concentration du glycane NA2F dans le sérum ;
(ix) la concentration d'alpha-tocophérol dans le plasma ;
(x) la concentration de cystéine dans le plasma.

10. Utilisation selon la revendication 9, dans lequel un ou plusieurs des biomarqueurs (xi) à (xxviii) suivants sont utilisés en plus des biomarqueurs (i) à (x) :

(xi) le taux cumulatif de méthylation des cytosines au niveau des positions de gènes ELOVL_2_CpG_22, ELOVL_2_CpG_23, et ELOVL_2_CpG_24 dans les cellules sanguines ;
(xii) le taux cumulatif de méthylation des cytosines au niveau des positions de gènes FHL_2_CpG_19, et FHL_2_CpG_20 dans les cellules sanguines ;
(xiii) la concentration de glycane NGA2FB dans le sérum ;
(xiv) la concentration de fibrinogène dans le plasma ;
(xv) la concentration d'urée dans le plasma ;
(xvi) la concentration de glucose dans le sérum ;
(xvii) la longueur des télomères dans les cellules sanguines ;
(xviii) la concentration de triglycérides dans le sérum ;
(xix) la concentration d'immunoglobuline M (IgM) dans le sérum ;
(xx) la concentration d'adiponectine dans le sérum ;
(xxi) la concentration accumulée de thréonine et de lactate dans l'urine ;
(xxii) la concentration d'homocystéine dans le plasma ;
(xxiii) la concentration de lycopène dans le plasma ;
(xxiv) la concentration de cholestérol dans le sérum ;
(xxv) la concentration d'hémoglobine glycosylée ($HbA_{1c}$) dans le sang total ;
(xxvi) la concentration de glycine dans l'urine ;
(xxvii) le nombre des cercles d'excision des récepteurs de lymphocytes T (Trec) dans 100 000 lymphocytes T provenant du sang total ;
(xxviii) la concentration de créatinine dans l'urine.

**11.** Utilisation d'au moins les biomarqueurs (i) à (x) suivants pour la détermination de l'âge biologique d'un être humain homme selon le procédé des revendications 1, 3, 6 à 8 :

(i) le taux cumulatif de méthylation des cytosines au niveau des positions de gènes ELOVL_2_CpG_11, ELOVL_2 CpG_12, ELOVL_2_CpG_13, et ELOVL_2_CpG_14 dans les cellules sanguines ;
(ii) le taux cumulatif de méthylation des cytosines au niveau des positions de gènes ELOVL_2_CpG_15, ELOVL_2_CpG_16, et ELOVL_2_CpG_17 dans les cellules sanguines ;
(iii) le taux cumulatif de méthylation des cytosines au niveau des positions de gènes FHL_2_CpG_13, FHL_2_CpG_14, et FHL_2_CpG_15 dans les cellules sanguines ;
(iv) le taux cumulatif de méthylation des cytosines au niveau de la position de gène FHL_2_CpG_18 ;
(v) la concentration de créatinine dans l'urine ;
(vi) le rapport du nombre des cellules CD3$^+$CD4$^+$ dans le sang total sur le nombre des cellules CD8$^+$ dans le sang total ;
(vii) la concentration de sulfate de déhydroépiandrostérone dans le plasma ;
(viii) la concentration d'alpha-2-macroglobuline dans le sérum ;
(ix) la concentration de lycopène dans le plasma ;
(x) la concentration de cystéine dans le plasma.

**12.** Utilisation selon la revendication 11, dans lequel un ou plusieurs des biomarqueurs (xi) à (xxiii) suivants sont utilisés en plus des biomarqueurs (i) à (x) :

(xi) le taux cumulatif de méthylation des cytosines au niveau des positions de gènes FHL_2_CpG_19, et FHL_2_CpG_20 dans les cellules sanguines ;
(xii) le nombre des cercles d'excision des récepteurs de lymphocytes T (Trec) dans 100 000 lymphocytes T provenant du sang total ;
(xiii) le logarithme décimal du rapport entre la concentration du glycane NGA2F dans le sérum et la concentration du glycane NA2F dans le sérum ;
(xiv) la concentration d'antigène spécifique de la prostate (PSA) dans le sérum ;
(xv) la concentration de fibrinogène dans le plasma ;
(xvi) la concentration d'albumine dans le plasma ;
(xvii) la concentration de glucose dans le sérum ;
(xviii) la concentration de glycane NGA2FB dans le sérum ;
(xix) le rapport de la concentration de Cu dans le plasma sur la concentration de Zn dans le plasma ;
(xx) le rapport du nombre des cellules CD16$^+$CD56$^+$ dans le sang total sur le nombre des cellules CD45$^+$ dans le sang total ;
(xxi) la concentration d'anticorps IgG dirigés contre *Clostridium tetani* dans le plasma ;
(xxii) le rapport du nombre des cellules CD3$^+$CD8$^+$ dans le sang total sur le nombre de cellules CD45$^+$ dans le sang total ;
(xxiii) la concentration de bêta-hydroxy-isovalérate dans l'urine.

**13.** Utilisation selon la revendication 9 ou la revendication 11, dans laquelle seulement les biomarqueurs (i) à (x) sont utilisés.

Fig. 1

Fig. 2

Fig. 3

FHL2_CpG_13.14.15

Fig. 4

Fig. 5

Fig. 6

Fig. 7

Ferritin [ng/ml]

RASIG male = 982
y = 1.09x + 57.47
rp= 0.108 rnp= 0.094

RASIG female = 1093
y = 1.71x + -38.35
rp= 0.372 rnp= 0.462

p=1.0% quantile hidden

p=1.0% quantile hidden

Fig. 8

Fig. 9

Fig. 10

## S_p6_n_glycan

**RASIG male = 1048**
y = -0.07x + 23.06
rp= -0.26  rnp= -0.282

**RASIG female = 1126**
y = -0.19x + 30.13
rp= -0.599  rnp= -0.625

p=1.0% quantile hidden

p=1.0% quantile hidden

AgeDaysAsYear

AgeDaysAsYear

Fig. 11

Fig. 12

55

Fig. 13

Fig. 14

Fig. 15

Fig. 16

Fig. 17

Fig. 18

ELOVL_2_CpG_22.23.24

**RASIG male = 1052**
y = 0x + 0.04
rp= 0.406   rnp= 0.48

**RASIG female = 1113**
y = 0x + 0.03
rp= 0.51   rnp= 0.537

p=1.0% quantile hidden

p=1.0% quantile hidden

AgeDaysAsYear

AgeDaysAsYear

EP 2 976 433 B1

Fig. 19

Fig. 20

Fig. 21

Fig. 22

Fig. 23

Fig. 24

Fig. 25

Fig. 26

ELOVL_2_CpG_6.7

**RASIG male = 1053**
y = 0x + 0.01
rp= 0.268  rnp= 0.352

**RASIG female = 1116**
y = 0x + 0.01
rp= 0.372  rnp= 0.399

p=1.0% quantile hidden

p=1.0% quantile hidden

AgeDaysAsYear

AgeDaysAsYear

EP 2 976 433 B1

Fig. 27

Fig. 28

Fig. 29

## Serum glucose [mmol/l]

**RASIG  male =  986**

y = 0.02x + 4.12

rp= 0.205  rnp= 0.247

**RASIG  female =  1094**

y = 0.02x + 3.83

rp= 0.23  rnp= 0.311

p=1.0% quantile hidden

p=1.0% quantile hidden

AgeDaysAsYear

AgeDaysAsYear

EP 2 976 433 B1

Fig. 30

Threonine/Lactate 10^-6

**RASIG male = 932**
y = 0x + 1.16
rp= -0.041 rnp= -0.101

**RASIG female = 945**
y = -0.01x + 1.45
rp= -0.22 rnp= -0.299

p=1.0% quantile hidden

p=1.0% quantile hidden

AgeDaysAsYear

AgeDaysAsYear

Fig. 31

Fig. 32

Fig. 33

Fig. 34

Fig. 35

Immunglobulin M [g/l]

Fig. 36

EP 2 976 433 B1

79

Fig. 37

Fig. 38

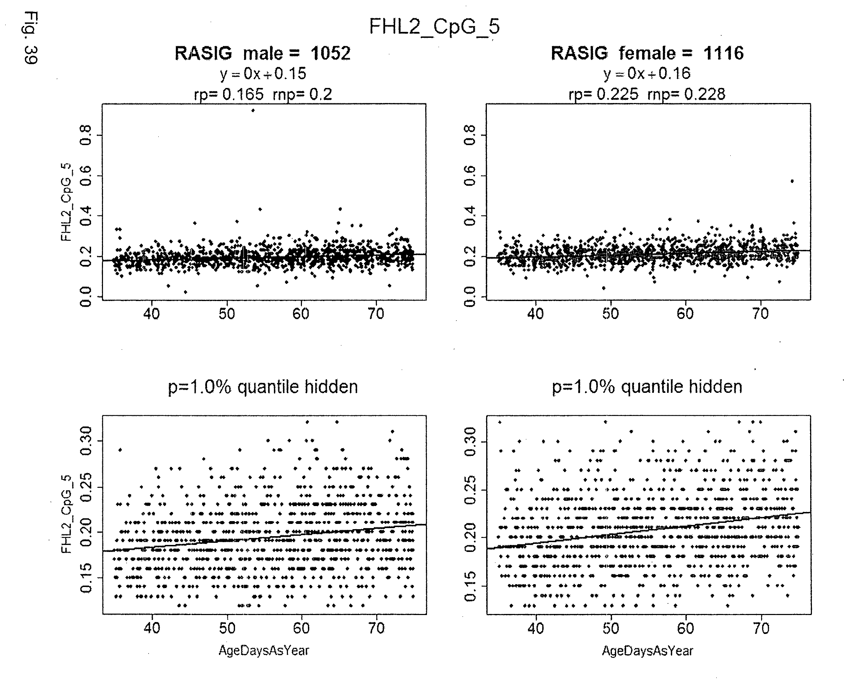

Fig. 39

# FHL2_CpG_5

EP 2 976 433 B1

**RASIG  male =  1052**
y = 0x + 0.15
rp= 0.165  rnp= 0.2

**RASIG  female =  1116**
y = 0x + 0.16
rp= 0.225  rnp= 0.228

p=1.0% quantile hidden

p=1.0% quantile hidden

AgeDaysAsYear

AgeDaysAsYear

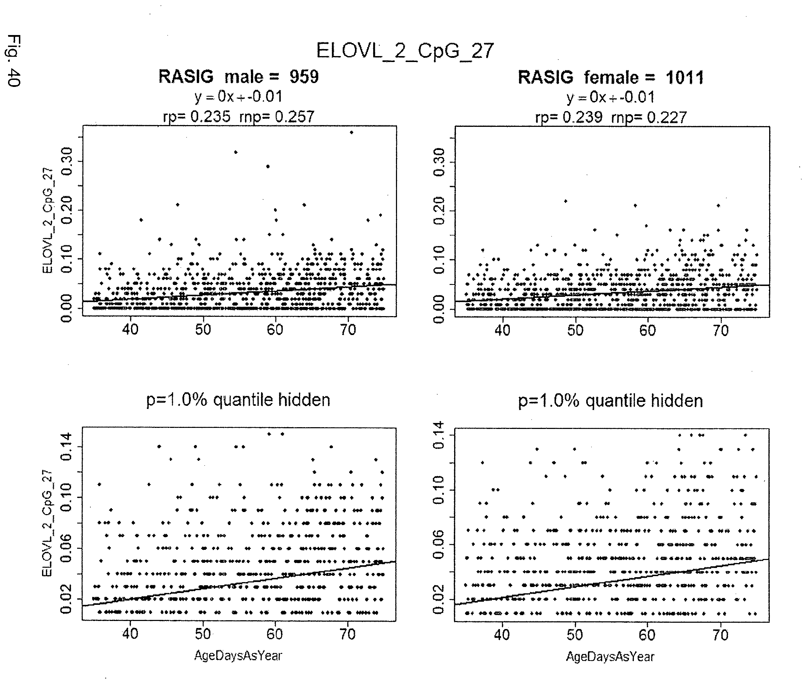

Fig. 40

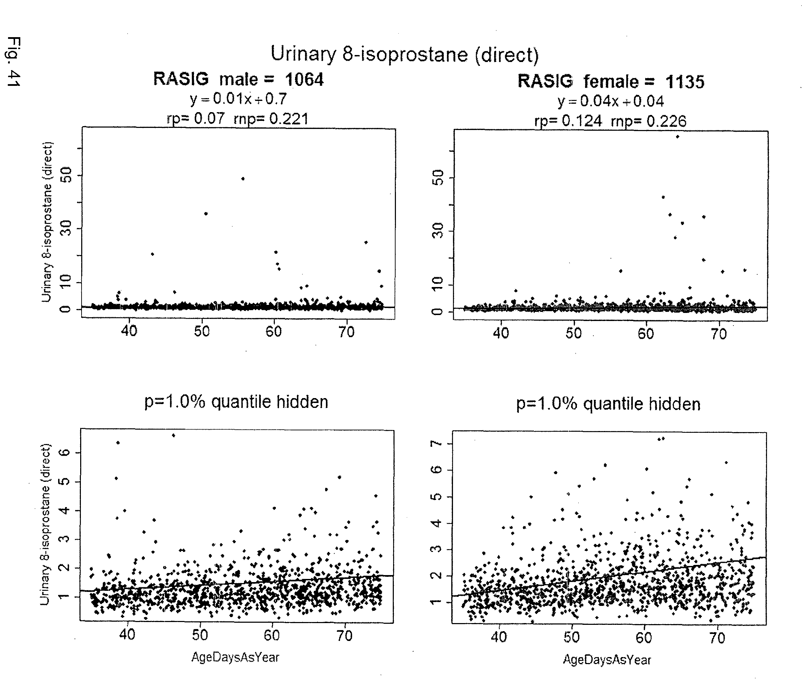

Fig. 41

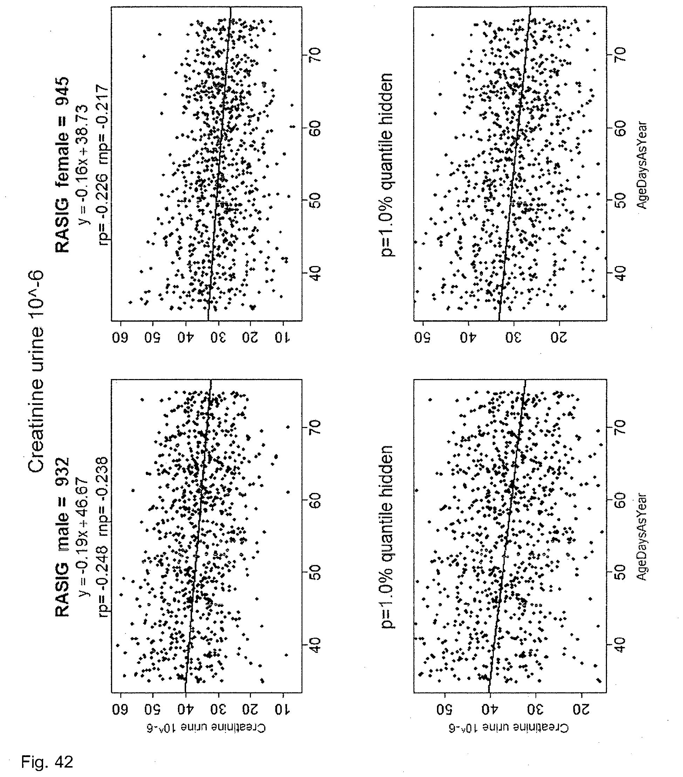

Fig. 42

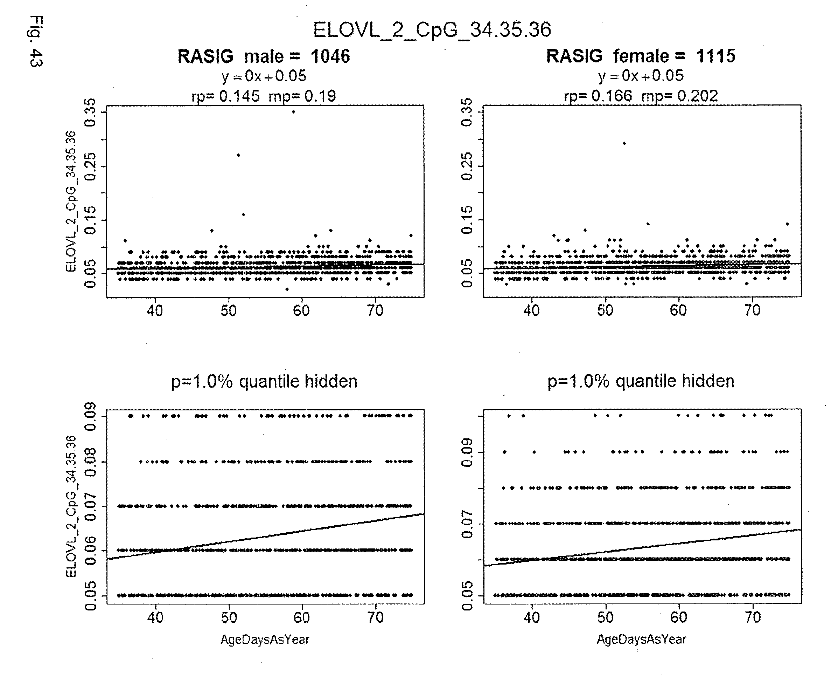

Fig. 43

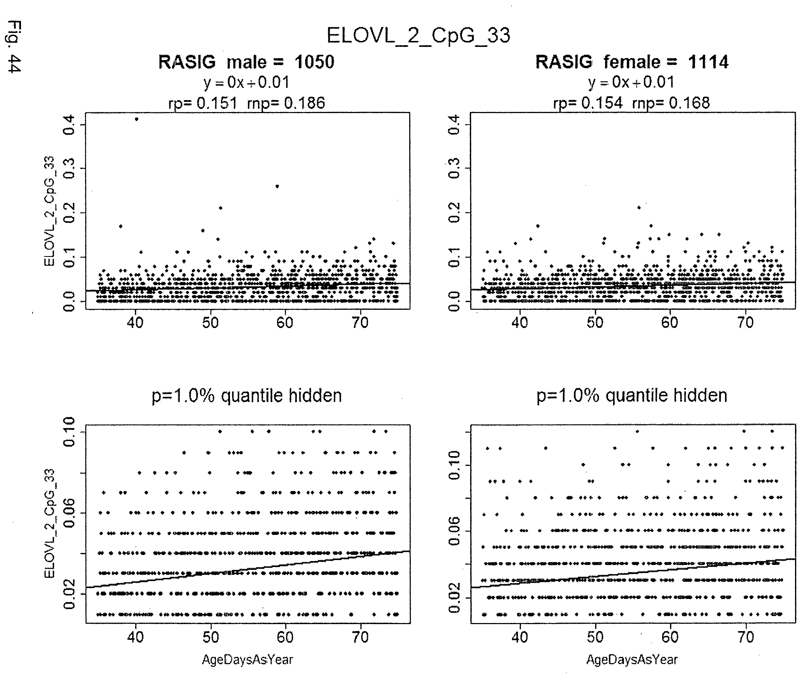

Fig. 44

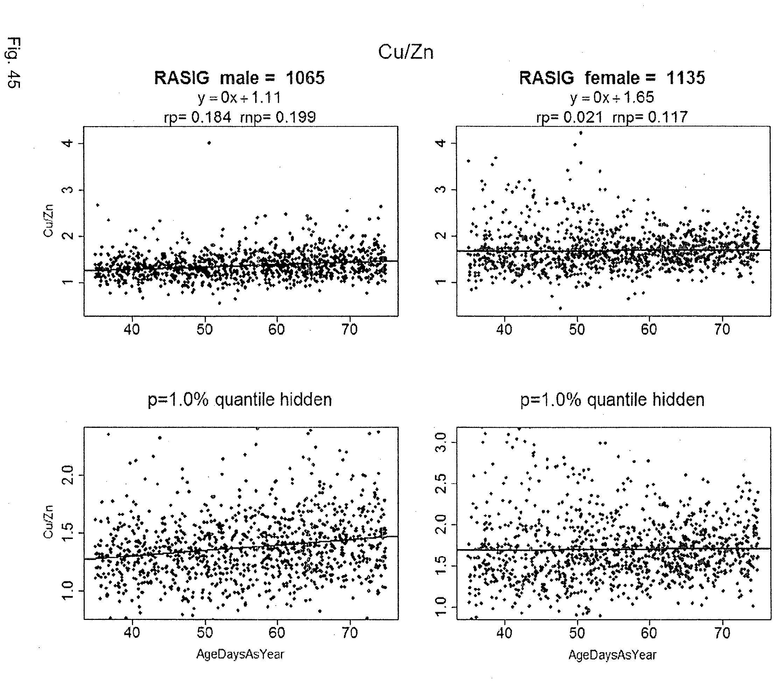

Fig. 45

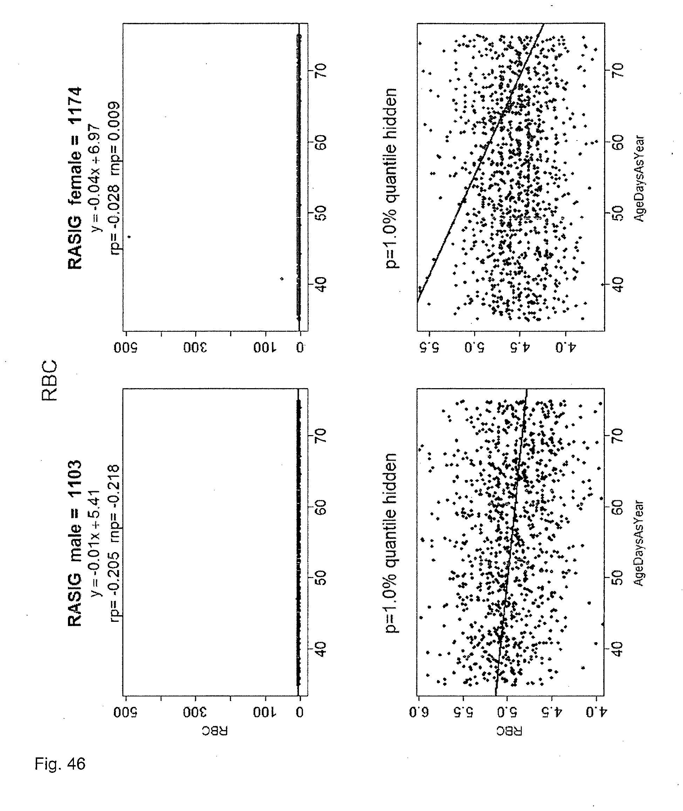

Fig. 46

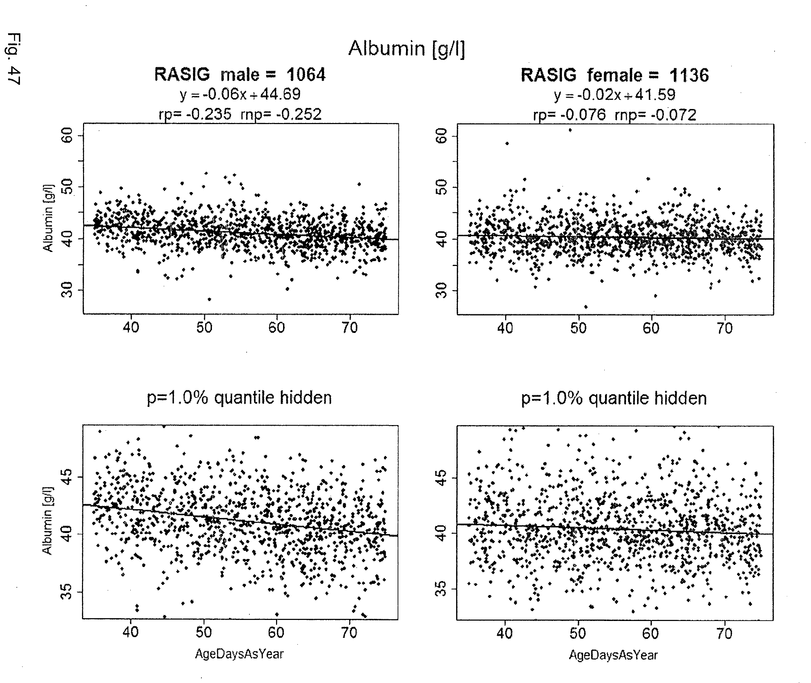

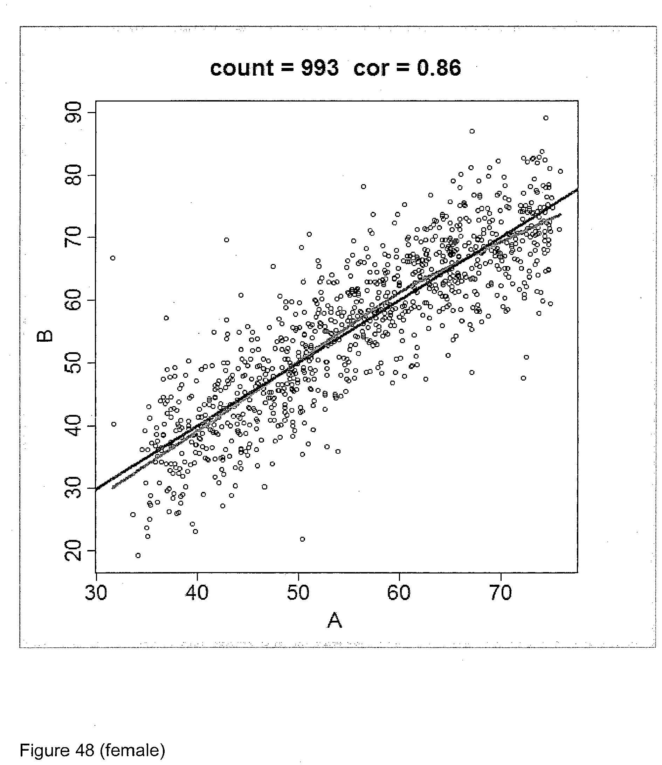

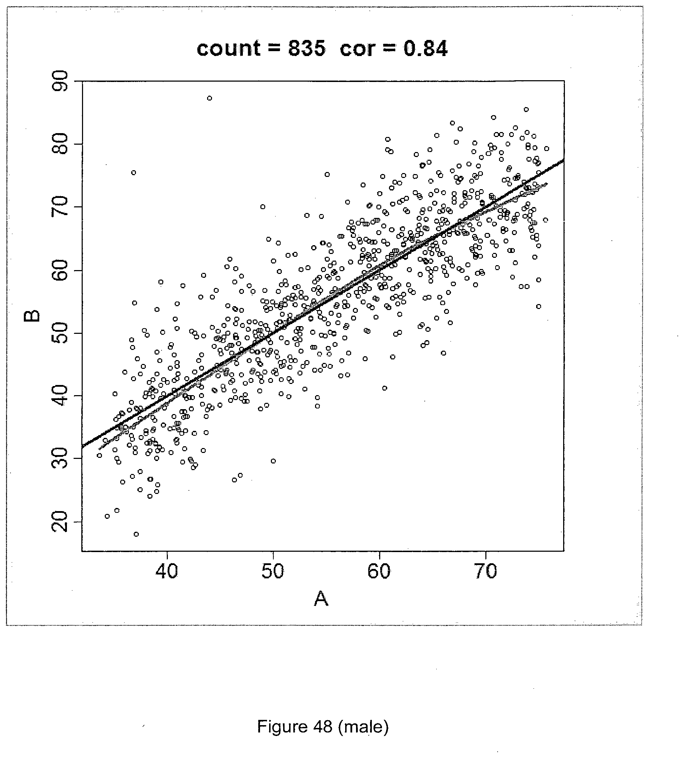

Fig. 47

Figure 48 (female)

Figure 48 (male)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0210445 A2

- WO 2012162139 A1


**Non-patent literature cited in the description**

- **HERNANDEZ et al.** *Hum. Mol. Genetics,* 2011, vol. 20, 1164-1172
- **GARAGNANI et al.** *Aging Cell,* 2012, vol. 11, 1132-1134
- **MADRIGANO et al.** *Epigenetics,* 2012, vol. 7, 63-70

- **BOCKLANDT et al.** *Plos One,* 2011, vol. 6, e14821
- **KOCH et al.** *Aging,* 2001, vol. 3, 1018-1027
- **MAGALHAES et al.** *Bioinformatics,* 2009, vol. 25, 875-881
- **VANHOOREN et al.** *Biogerontology,* 2008, vol. 9, 351-356